# EUROPEAN PATENT SPECIFICATION

(11) **EP 3 723 746 B1**
(45) Date of publication and mention of the grant of the patent: **23.02.2022**
(21) Application number: 18816078.2
(22) Date of filing: 14.12.2018
(51) Int. Cl.: C07C 13/04, C07C 13/06, C07C 15/02, A61K 31/192, A61K 31/216, A61P 21/00, A61P 21/04

(54) **PHENOXY ACIDS FOR THE TREATMENT OF NEUROMUSCULAR DISORDERS**
PHENOXY-SAÜRE ZUR BEHANDLUNG NEUROMUSKULÄRER ERKRANKUNGEN
PHÉNOXY ACIDES POUR LE TRAITEMENT DE TROUBLES NEUROMUSCULAIRES

(30) Priority: 14.12.2017 US 201762598959 P; 15.01.2018 EP 18151651
(43) Date of publication of application: 21.10.2020
(73) Proprietor: NMD Pharma A/S, 8200 Aarhus N (DK)
(72) Inventor: J.S. KNUTSEN, Lars, Frinton-on-Sea Essex CO13 9AY (GB); KELLY, Nicholas, 2880 Bagsvaerd (DK); HOLM PEDERSEN, Thomas, 8240 Risskov (DK); ELSBORG OLESEN, Claus, 8230 Abyhoj (DK); LABELLE, Marc, Deceased (DK); LITTLE, Paul, Brian, 4270 Hong (DK); E COOPER, Martin, Nottingham NG4 1AQ (GB); SARASWAT, Neerja, Winnipeg Manitoba, R3X 1R4 (CA); DUDEKULA, Dastagiri, Winnipeg Manitoba R3N 1R4 (CA); A. TAJ, Rafiq, Winnipeg Manitoba, R3T 3A1 (CA)
(74) Representative: Høiberg P/S
(86) International application number: PCT/EP2018/084988
(87) International publication number: WO 2019/115780

(56) References cited:
- WO-A1-2016/202341
- FERORELLI, S.; LOIODICE, F.; TORTORELLA, V.; CONTE-CAMERINO, D.; DE LUCA, A.M.: "Carboxylic acids and skeletal muscle chloride channel conductance: effects on the biological activity induced by the introduction of methyl groups on the aromatic ring of chiral a-(4-chloro-phenoxy)alkanoic acids", FARMACO, vol. 56, 2001, pages 239-246, XP002781719, cited in the application
- E.C. AROMATARIS: "Pharmacology of the CIC-1 Chloride channel", PhD Thesis , 2009, page 1-3, 89-121, XP002781720, Retrieved from the Internet: URL:https://digital.library.adelaide.edu.a u/dspace/bitstream/2440/58973/8/02whole.pd f [retrieved on 2018-06-06] cited in the application

## Description

### Technical field

The present invention relates to compounds and their use in treating, ameliorating and/or preventing neuromuscular disorders, including the reversal of drug-induced neuromuscular blockade. The compounds as defined herein preferably inhibit the CIC-1 ion channel. The invention further relates to methods of treating, preventing and/or ameliorating neuromuscular disorders, by administering said composition to a person in need thereof.

### Background

Walking, breathing, and eye movement are examples of essential everyday physiological activities that are powered by the contractile activity of skeletal muscle. Skeletal muscles are inherently in a resting state and contractile activity occurs exclusively in response to commands from the central nervous system (CNS). Such neuronal commands take the form of action potentials that travel from the brain to the muscle fibres in several steps. The neuromuscular junction (NMJ) is a highly specialized membrane area on muscle fibres where motor neurons come into close contact with the muscle fibres, and it is at the NMJ where neuronal action potentials are transmitted to muscular action potentials in a one-to-one fashion *via* synaptic transmission.

Neuromuscular transmission refers to the sequence of cellular events at the NMJ whereby an action potential in the lower motor neuron is transmitted to a corresponding action potential in a muscle fibre (Wood SJ, Slater CR. Safety factor at the neuromuscular junction. Prog. Neurobiol. 2001, 64, 393-429). When a neuronal action potential arrives at the pre-synaptic terminal it triggers influx of Ca²⁺ through voltage gated P/Q-type Ca²⁺ channels in the nerve terminal membrane. This influx causes a rise in cytosolic Ca²⁺ in the nerve terminal that triggers exocytosis of acetylcholine (ACh). Released ACh next diffuses across the synaptic cleft to activate nicotinic ACh receptors in the post-synaptic, muscle fibre membrane. Upon activation, ACh receptors convey an excitatory current flow of Na⁺ into the muscle fibre, which results in a local depolarization of the muscle fibre at the NMJ that is known as the endplate potential (EPP). If the EPP is sufficiently large, voltage gated Na⁺ channels in the muscle fibre will activate and an action potential in the muscle fibre will ensue. This action potential then propagates from the NMJ throughout the muscle fibre and triggers release of Ca²⁺ release from the sarcoplasmic reticulum. The released Ca²⁺ activates the contractile proteins within the muscle fibres, thus resulting in contraction of the fibre.

Failure of neuromuscular transmission can arise from both pre-synaptic dysfunction [Lambert Eaton syndrome (Titulaer MJ, Lang B, Verschuuren JJ. Lambert-Eaton myasthenic syndrome: from clinical characteristics to therapeutic strategies. Lancet Neurol. 2011, 10, 1098-107), amyotrophic lateral sclerosis (Killian JM, Wilfong AA, Burnett L, Appel SH, Boland D. Decremental motor responses to repetitive nerve stimulation in ALS. Muscle Nerve, 1994, 17, 747-754), spinal muscular atrophy (Wadman RI, Vrancken AF, van den Berg LH, van der Pol WL. Dysfunction of the neuromuscular junction in spinal muscular atrophy types 2 and 3. Neurology, 2012, 79, 2050-2055) and as a result of post-synaptic dysfunction as occurs in myasthenia gravis (Le Panse R, Berrih-Aknin S. Autoimmune myasthenia gravis: autoantibody mechanisms and new developments on immune regulation. Curr Opin Neurol., 2013, 26, 569-576)]. Failure to excite and/or propagate action potentials in muscle can also arise from reduced muscle excitability such as in critical illness myopathy (CIM) (Latronico, N., Bolton, C.F. Critical illness polyneuropathy and myopathy: a major cause of muscle weakness and paralysis. Lancet Neurol. 2011, 10, 931-941). In Lambert Eaton syndrome, an autoimmune attack against the pre-synaptic P/Q-type Ca²⁺ channels results in markedly reduced Ca²⁺ influx into the nerve terminal during the pre-synaptic action potential and consequently a reduced release of ACh into the synaptic cleft. In myasthenia gravis, the most common finding is an autoimmune attack on the post-synaptic membrane either against the nicotinic ACh receptors or the muskreceptor in the muscle fibre membrane³. Congenital forms of myasthenia are also known⁵. Common to disorders with neuromuscular transmission failure (Lambert Eaton syndrome, amyotrophic lateral sclerosis, spinal muscular atrophy and myasthenia gravis) is that the current flow generated by ACh receptor activation is markedly reduced, and EPPs therefore become insufficient to trigger muscle fibre action potentials.

Neuromuscular blocking agents also reduce EPP by antagonizing ACh receptors. In CIM with reduced muscle excitability, the EPP may be of normal amplitude but they are still insufficient to trigger muscle fibre action potentials because the membrane potential threshold for action potential excitation has become more depolarized because of loss of function of voltage gated Na⁺ channels in the muscle fibres.

While ACh release (Lambert Eaton, amyotrophic lateral sclerosis, spinal muscular atrophy), ACh receptor function (myasthenia gravis, neuromuscular blockade) and function of voltage gated Na⁺ channels (CIM) are essential components in the synaptic transmission at NMJ, the magnitude of the EPP is also affected by inhibitory currents flowing in the NMJ region of muscle fibres. These currents tend to outbalance excitatory current through ACh receptors and, expectedly, they thereby tend to reduce EPP amplitude. The most important ion channel for carrying such inhibitory membrane currents in muscle fibres is the muscle-specific CIC-1 Cl⁻ ion channel (Kwieciński H, Lehmann-Horn F, Rüdel R. Membrane currents in human intercostal muscle at varied extracellular potassium. Muscle Nerve. 1984, 7, 465-469; Kwieciński H, Lehmann-Horn F, Rüdel R. Drug-induced myotonia in human intercostal muscle. Muscle Nerve. 1988, 11, 576-581; Pedersen, T.H., F. de Paoli, and O.B. Nielsen. Increased excitability of acidified skeletal muscle: role of chloride conductance. J. Gen. Physiol., 2005, 125, 237-246).

ACh esterase (AChE) inhibitors are traditionally used in the treatment of myasthenia gravis. This treatment leads to improvement in most patients but it is associated with side effects, some of which are serious (Mehndiratta MM, Pandey S, Kuntzer T. Acetylcholinesterase inhibitor treatment for myasthenia gravis. Cochrane Database Syst Rev. 2014, Oct 13;10). Because ACh is an import neurotransmitter in the autonomic nervous system, delaying its breakdown can lead to gastric discomfort, diarrhea, salivation and muscle cramping. Overdosing is a serious concern as it can lead to muscle paralysis and respiratory failure, a situation commonly referred to as cholinergic crisis. Despite the serious side effects of AChE inhibitors, these drugs are today the treatment of choice for a number of disorders involving neuromuscular impairment. In patients where pyridostigmine (a parasympathomimetic and a reversible ACHE inhibitor) is insufficient, corticosteroid treatment (prednisone) and immunosuppressive treatment (azathioprine) is used. Plasma exchange can be used to obtain a fast but transient improvement.

Unfortunately, all of the currently-employed drug regimens for treatment of myasthenia gravis are associated with deleterious long-term consequences (Howard, J.F. Jr. Adverse drug effects on neuromuscular transmission. Semin Neurol. 1990, 10, 89 - 102) despite research to identify new treatments (Gilhus, N.E. New England Journal of Medicine, 2016, 375, 2570-2581).

The CIC-1 ion channel (Pedersen, T.H., Riisager, A., Vincenzo de Paoli, F., Chen, T-Y, Nielsen, O.B. Role of physiological CIC-1 Cl- ion channel regulation for the excitability and function of working skeletal muscle. J. Gen. Physiol. 2016, 147, 291 - 308) is emerging as a target for potential drugs, although its potential has been largely unrealized.

There have been publications of various ligands at the CIC-1 ion channels, see for example: Liantonio, A., Accardi, A., Carbonara, G., Fracchiolla, G., Loiodice, F., Tortorella P, Traverso S, Guida P, Pierno S, De Luca A, Camerino DC, Pusch M. Molecular requisites for drug binding to muscle CLC-1 and renal CLC-K channel revealed by the use of phenoxy-alkyl derivatives of 2-(p-chlorophenoxy)propionic acid. Mol. Pharmacol., 2002, 62, 265 - 271 and Liantonio, A. et al., Structural requisites of 2-(p-chlorophenoxy)propionic acid analogues for activity on native rat skeletal muscle chloride conductance and on heterologously expressed CLC-1. Br. J. Phamacol., 2003, 129, 1255 - 1264.

In the article Liantonio, A., Pusch, M., Picollo, A., Guida, P., De Luca, A., Pierno, S., Fracchiolla, G., Loiodice, F., Tortorella, P., Conte-Camerino, D. Investigations of pharmacologic properties of the renal CLC-K1 chloride channel co-expressed with barttin by the use of 2-(p-chlorophenoxy)propionic acid derivatives and other structurally unrelated chloride hannels blockers. Journal of the American Society of Nephrology, 2004, 15, 13 - 20, ligands for CLC-K1 chloride channels were disclosed.

In Bettoni, G., Ferorelli, S., Loiodice, F., Tangari, N., Tortorella, V., Gasparrini, F., Misiti, D., Villani, C. Chiral α-substituted α-aryloxyacetic acids: synthesis, absolute configuration, chemical resolution, and direct separation by HPLC. Chirality, 1992, 4, 193 - 203, some further substituted phenoxyacetic acids were investigated.

In the publication Pusch, M., Liantonio, A., Bertorello, L., Accardi, A., De Luca, A., Pierno, S., Tortorella, V., Conte-Camerino, D. Pharmacological characterization of chloride channels belonging to the CIC family by the use of chiral clofibric acid derivatives. Molecular Pharmacology, 2000, 58, 498 - 507, the authors disclosed effects of enantiomers of 2-(p-chlorophenoxy)propionic acid on CLC-1 and CLC-2 ion channels.

In the article Ferorelli, S., Loiodice, F., Tortorella, V., Conte-Camerino, D., De Luca, A.M. Carboxylic acids and skeletal muscle chloride channel conductance: effects on the biological activity induced by the introduction of methyl groups on the aromatic ring of chiral α-(4-chloro-phenoxy)alkanoic acids, Farmaco, 2001, 56, 239 - 246, derivatives of (4-chloro-phenoxy)alkanoic acids were tested for skeletal muscle chloride conductance.

In the publication: Conte-Camerino, D., Mambrini, M., DeLuca, A., Tricarico, D., Bryant, S.H., Tortorella, V., Bettoni, G. Enantiomers of clofibric acid analogs have opposite actions on rat skeletal muscle chloride channels, Pfluegers Archiv., 1988, 413, 105 - 107, enantiomers of clofibric acid were investigated, as well as Feller, D.R., Kamanna, V.S., Newman, H.A.I., Romstedt, K.J., Witiak, D.T., Bettoni, G., Bryant, S.H., Conte-Camerino, D., Loiodice, F., Tortorella, V. Dissociation of hypolipidemic and antiplatelet actions from adverse myotonic effects of clofibric acid related enantiomers. Journal of Medicinal Chemistry, 1987, 30, 1265 - 1267.

Edoardo Aromataris investigated 4-chlorophenoxyisobutyric acid derivatives in his PhD thesis "Pharmacology of the CIC-1 Chloride Channel"; see:
https://digital.library.adelaide.edu.au/dspace/bitstream/2440/58973/8/02whole.pdf

In WO 2005/105727, Sandham et al., reported a range of substituted phenoxyacetic acids, including 2-(4-chloro-2-cycloheptylphenoxy)acetic acid, as CRTh2 receptor antagonists for treating inflammatory or allergic conditions.

In WO 2006/037982, Bonnert et al. described a set of substituted phenoxyacetic acids for the treatment of respiratory disorders.

In US 2006/0211765, Pairaudeau et al. reported a further series of substituted biphenyloxy acetic acids for treatment of respiratory disorders

In WO 2016/202341, Pedersen et al. reported a series of of phenoxypropionic acids and related compounds that appear to block the CIC-1 ion channel for use in treating, ameliorating and/or preventing neuromuscular disorders. However, they possess alternative structural features to those in the current invention.

### Summary

The present invention comprises a new series of compounds that alleviate disorders of the neuromuscular junction through inhibition of CIC-1 channels.

It has been found that a set of novel compounds that inhibit CIC-1 ion channels are capable of restoring neuromuscular transmission, as evidenced by the data generated by investigation of the compound set in biological models described herein. These compounds thus constitute a new group of potential drugs that can be used to treat or ameliorate muscle weakness and muscle fatigue in neuromuscular junction disorders caused by disease or by neuromuscular blocking agents.

The present invention thus concerns the discovery of new CIC-1 ion channel inhibitors with application in the treatment of a range of conditions, such as reversal of block, ALS and myasthenic conditions, in which muscle activation by the nervous system is compromised and symptoms of weakness and fatigue are prominent.

In one aspect, the invention concerns a compound of Formula (I): 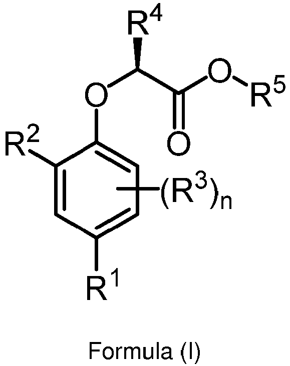 wherein:
- R¹ is selected from the group consisting of F, Cl, Br, I, -CN, -CF3, -CCl₃, - CHF₂, -CHCl₂, -CH₂F, -CH₂Cl, -OCF₃ and -OCCl₃;
- R² is selected from the group consisting of C₂₋₆ alkyl, C₂₋₆ alkenyl, C₂₋₆ alkynyl, C₃₋₆ cycloalkyl, C₅₋₆ cycloalkenyl, -C(=O)-C₁₋₅ alkyl, -C(=O)-C₂₋₅ alkenyl, -C(=O)-C₂₋₅ alkynyl, -C(=O)-C₃₋₅ cycloalkyl and -C(=O)-C₅ cycloalkenyl, each of which may be optionally substituted with one or more, identical or different, substituents R⁶;
- R³ is selected from the group consisting of hydrogen, deuterium, tritium, F, Cl, Br, I, -CN, -CF3, -CCl₃, -CHF2, -CHCl₂, -CH2F, -CH2CI, -OCF3, -OCCl₃ and isocyanide;
- R⁴ is selected from the group consisting of C₁₋₅ alkyl, C₂₋₅ alkenyl, C₂₋₅ alkynyl, C₃₋₅ cycloalkyl, C₅ cycloalkenyl, each of which may be optionally substituted with one or more, identical or different, substituents R⁷;
- R⁵ is selected from the group consisting of H, C₁₋₅ alkyl optionally substituted with one or more, identical or different, substituents R¹¹, C₂₋₅ alkenyl, C₂₋₅ alkynyl, C₃₋₆ cycloalkyl optionally substituted with one or more, identical or different, substituents R¹¹, phenyl optionally substituted with one or more, identical or different, substituents R¹² and benzyl optionally substituted with one or more, identical or different, substituents R¹²;
- R⁶ is independently selected from the group consisting of hydrogen, deuterium, tritium, F, Cl, Br, I, -CN, isocyanide, -O-C₁₋₃ alkyl, -S-C₁₋₃ alkyl, -CH2-OH, -CH₂-O-C₁₋₃ alkyl, -O-CH₂-Ph, -CH2-SH and -CH₂-S-C₁₋₃ alkyl;
- R⁷ is independently selected from the group consisting of deuterium, tritium, F, Cl, Br, I, -CN, isocyanide, -O-C₁₋₃ alkyl, -S-C₁₋₃ alkyl, -CH₂-O-C₁₋₃ alkyl and -CH₂-S-C₁₋₃ alkyl;
- R¹¹ is independently selected from the group consisting of deuterium and F;
- R¹² is independently selected from the group consisting of deuterium, methoxy, nitro, cyano, Cl, Br, I and F; and
- n is an integer 0, 1, 2 or 3;
or a pharmaceutically acceptable salt, hydrate, polymorph, tautomer, or solvate thereof, for use in treating, ameliorating and/or preventing a neuromuscular disorder, and/or for use in reversing and/or ameliorating a neuromuscular blockade.

In another aspect, the invention concerns a compound of Formula (I): 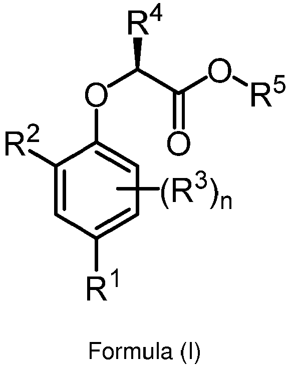 wherein:
- R¹ is selected from the group consisting of F, Cl, Br and I;
- R² is selected from the group consisting of C₂₋₆ alkyl, C₂₋₆ alkenyl, C₂₋₆ alkynyl, C₃₋₅ cycloalkyl, C₅ cycloalkenyl, -C(=O)-C₁₋₅ alkyl, -C(=O)-C₂₋₅ alkenyl, - C(=O)-C₂₋₅ alkynyl, -C(=O)-C₃₋₅ cycloalkyl and -C(=O)-C₅ cycloalkenyl, each of which may be optionally substituted with one or more, identical or different, substituents R⁶;
- R³ is selected from the group consisting of hydrogen, deuterium, tritium, F, Cl, Br, I, -CN, -CF3, -CCl₃, -CHF2, -CHCl₂, -CH2F, -CH2CI, -OCF₃, -OCCl₃ and isocyanide;
- R⁴ is selected from the group consisting of C₁₋₅ alkyl, C₂₋₅ alkenyl, C₂₋₅ alkynyl, C₃₋₅ cycloalkyl, C₅ cycloalkenyl, each of which may be optionally substituted with one or more, identical or different, substituents R⁷;
- R⁵ is selected from the group consisting of H, C₁₋₅ alkyl optionally substituted with one or more, identical or different, substituents R¹¹, C₂₋₅ alkenyl, C₂₋₅ alkynyl, C₃₋₆ cycloalkyl optionally substituted with one or more, identical or different, substituents R¹¹, phenyl optionally substituted with one or more, identical or different, substituents R¹² and benzyl optionally substituted with one or more, identical or different, substituents R¹²;
- R⁶ is independently selected from the group consisting of hydrogen, deuterium, tritium, F, Cl, Br, I, -CN, isocyanide, -O-C₁₋₃ alkyl, -S-C₁₋₃ alkyl, -CH₂-OH, -CH₂-O-C₁₋₃ alkyl, -O-CH₂-Ph, -CH₂-SH and -CH₂-S-C₁₋₃ alkyl;
- R⁷ is independently selected from the group consisting of deuterium, tritium, F, Cl, Br, I, -CN, isocyanide, -O-C₁₋₃ alkyl, -S-C₁₋₃ alkyl, -CH₂-O-C₁₋₃ alkyl and -CH₂-S-C₁₋₃ alkyl;
- R¹¹ is independently selected from the group consisting of deuterium and F;
- R¹² is independently selected from the group consisting of deuterium, methoxy, nitro, cyano, Cl, Br, I and F; and
- n is an integer 0, 1, 2 or 3;
or a pharmaceutically acceptable salt, hydrate, polymorph, tautomer, or solvate thereof, with the proviso that when R² is C(=O)-CH₃, R¹ is Br and R⁵ is H or Me then R⁴ is not Me or CH2CHMe2; and with the proviso that when R² is CHMe2, R¹ is Br and R⁵ is H or Me then R⁴ is not Me.

In yet another aspect, the invention concerns a composition comprising a compound as defined herein.

### Description of Drawings

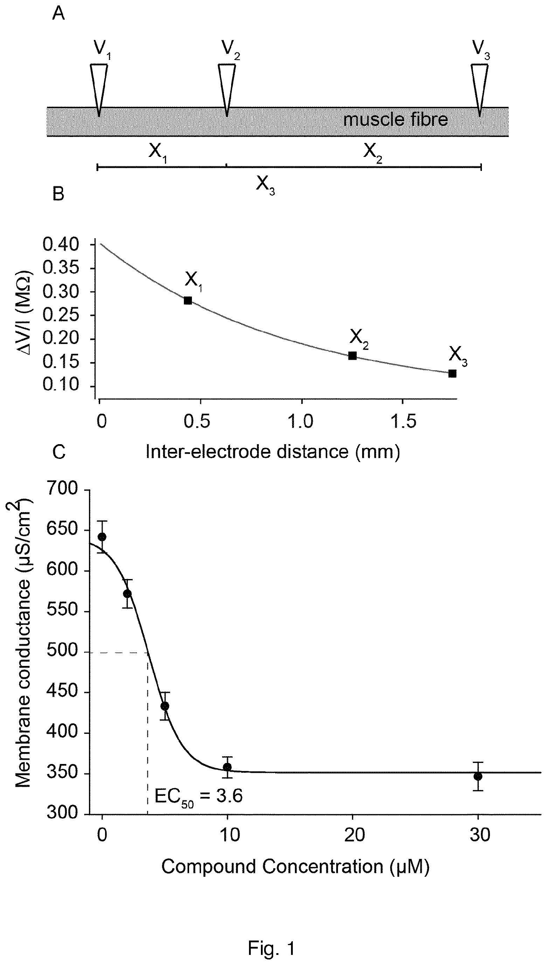

**Figure 1****:** Panel A shows a schematic representation of the positioning of the three microelectrodes (V₁, V₂ and V₃) when inserted in a single skeletal muscle fibre for Gₘ determination. Please note that the drawing illustrates only the impaled fibre although it is part of an intact muscle that contains many such fibres. All electrodes recorded the membrane potential of the fibre and the two peripheral electrodes were used to inject current (-30 nA, 50 ms). The electrodes were inserted with known inter-electrode distances (X₁, X₂ and X₃). After insertion, current was passed first via the V₁ electrode and then via the V3 electrode. The resulting deflections in the membrane voltage were measured by the other electrodes. The steady state deflections in membrane potential were measured and divided by the magnitude of the injected current (-30 nA) to obtain transfer resistances. These were next plotted against inter-electrode distances, and fitted to an exponential function (Panel B), from which Gₘ could be calculated using linear cable theory. The approach described in panel A and B, was repeated for several muscle fibres in the muscle during exposure at increasing concentrations of compound A-19, with approx. 10 fibres at each concentration. Average Gₘ at each concentration was plotted as a function of compound concentration in panel C, and fitted to a 4-parameter sigmoidal function from which the EC₅₀ value for the compound was obtained (dashed line)

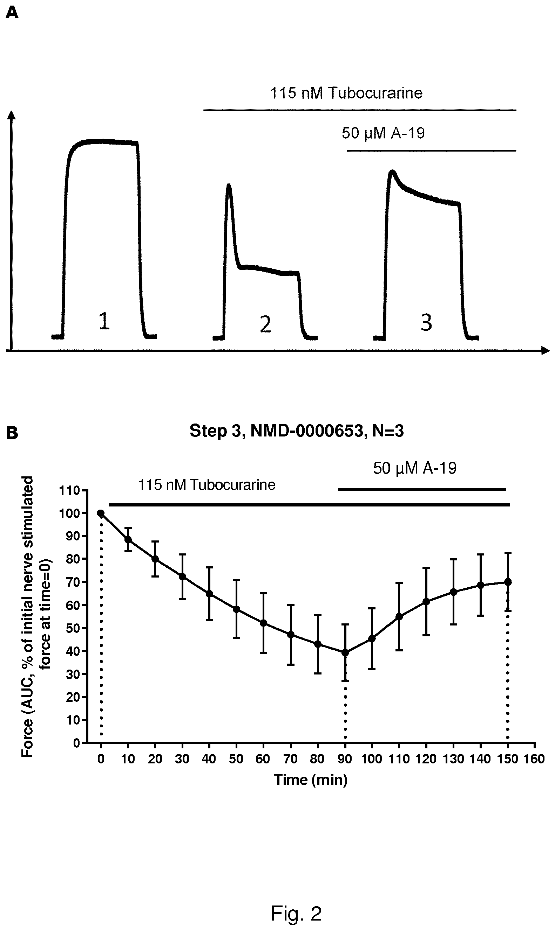

**Figure 2****:** Panel A shows representative force traces before and after exposure to compound A-19. Force traces from a representative muscle stimulated to contract in 1) control condition before addition of neuromuscular blocking agent, 2) the force response to stimulation after 90 minutes incubation with Tubocurarine. Here the muscle displays severe neuromuscular transmission impediment, and 3) The muscle force response after addition of 50 µM compound A-19. Panel B shows average force (AUC) from 3 muscles relative to their initial force. The traces presented in panel A (1, 2, 3), correspond to the dotted lines in panel B, respectively. Thus, force is lost due to 90 min incubation in tubocurarine and is subsequently recovered when compound A-19 is added.

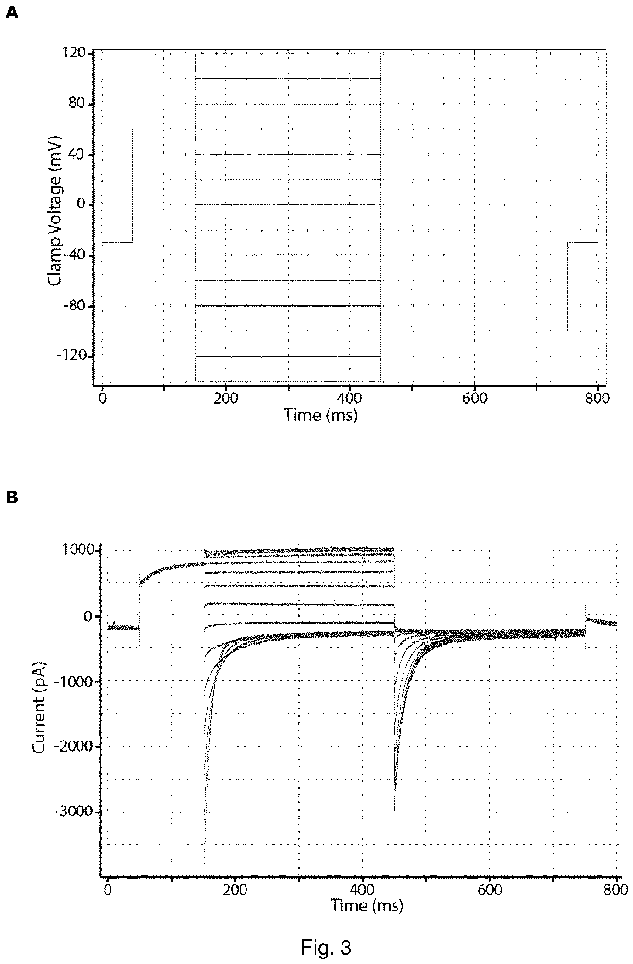

**Figure 3****:** Panel A illustrates the voltage protocol used to evoke currents in whole cell patches of CHO cells expressing human CIC-1 channels. Panel B shows representative whole cell current traces from a patched CHO cell expressing human CIC-1 channels. Currents were evoked by applying the voltage protocol shown in Panel A.

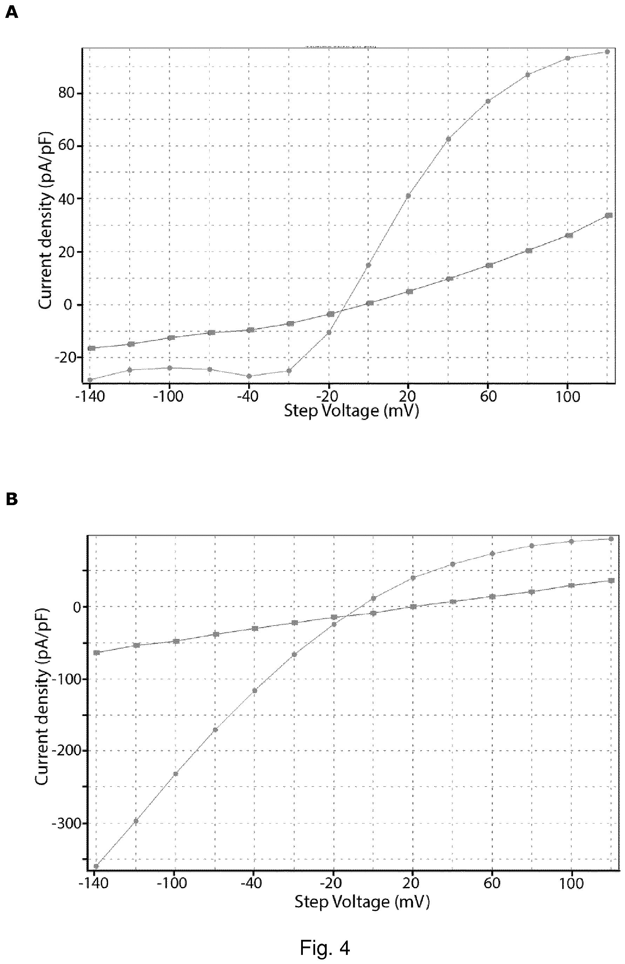

**Figure 4****:** Panel A shows a representative I/V plot of constant current density in a CIC-1 expressing CHO cell before (circles) and after (squares) application of 100 µM of the CIC-1 inhibitor, 9-anthracenecarboxylic acid (9-AC, Sigma A89405). Panel B shows a I/V plot of instant tail current density from the same CIC-1 expressing CHO cell as illustrated in Panel A, before (circles) and after (squares) application of 100 µM 9-AC.

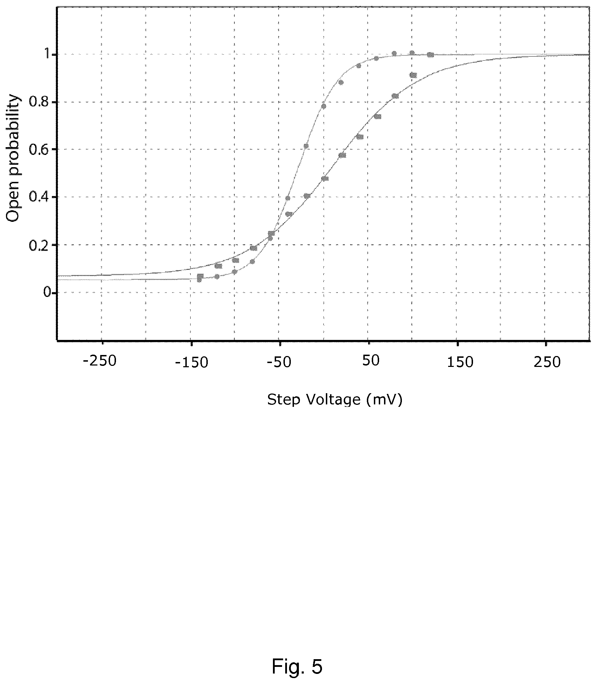

**Figure 5:** Figure 5 shows representative plots of normalized instant tail currents from a CIC-1 expressing CHO cell patch before (circles) and after (squares) application of 100 µM 9-AC. The instant tail currents at each voltage step were normalized to the maximal tail current obtained following the (+)120 mV voltage step and fitted to a Boltzmann function to determine the half activation potential, V_{1/2}.

### Detailed description

### Definitions

The terms "C₁₋₃ alkyl", "C₁₋₅-alkyl" and "C₂₋₆-alkyl" refers to a branched or unbranched alkyl group having from one to three, one to five or two to six carbon atoms respectively, including but not limited to methyl, ethyl, prop-1-yl, prop-2-yl, 2-methyl-prop-1-yl, 2-methyl-prop-2-yl, 2,2-dimethyl-prop-1-yl, but-1-yl, but-2-yl, 3-methyl-but-1-yl, 3-methyl-but-2-yl, pent-1-yl, pent-2-yl, pent-3-yl, hex-1-yl, hex-2-yl and hex-3-yl.

The term "C₁₋₅-alkenyl" and "C₂₋₆-alkenyl" refers to a branched or unbranched alkenyl group having from one to five or two to six carbon atoms respectively, two of which are connected by a double bond, including but not limited to ethenyl, propenyl, isopropenyl, butenyl, isobutenyl, pentenyl, isopentenyl and hexenyl.

The term "C₁₋₅-alkynyl" and "C₂₋₆-alkynyl" refers to a branched or unbranched alkynyl group having from one to five or two to six carbon atoms respectively, two of which are connected by a triple bond, including but not limited to ethynyl, propynyl, butynyl, pentynyl and hexynyl.

The term "-C(=O)-" refers to a carbonyl group and is used herein followed by a specification of the group connected thereto, such as for example the term "-C(=O)-C₁₋₅ alkyl" which refers to a carbonyl group connected to a branched or unbranched alkyl group having from one to five carbon atoms, including but not limited to a carbonyl group connected to methyl, ethyl, prop-1-yl, prop-2-yl, 2-methyl-prop-1-yl, 2-methyl-prop-2-yl, 2,2-dimethyl-prop-1-yl, but-1-yl, but-2-yl, 3-methyl-but-1-yl, 3-methyl-but-2-yl, pent-1-yl, pent-2-yl or pent-3-yl.

The term "C₃₋₅-cycloalkyl" and "C₃₋₆ cycloalkyl" refers to a group having three to five or three to six carbon atoms respectively including a monocyclic or bicyclic carbocycle, including but not limited to cyclopropyl, cyclobutyl, cyclopentyl, cyclopropylmethyl, cyclopropylethyl, cyclobutylmethyl, cyclobutylethyl and cyclohexyl.

The term "C₅₋₆-cycloalkenyl" and "C₅ cycloalkenyl" refers to a group having five to six or five carbon atoms respectively including a monocyclic or bicyclic carbocycle wherein two carbon atoms in the ring are connected by a double bond, including but not limited to cyclobutenylmethyl, cyclobutenylethyl, cyclopentenyl, cyclopentenylmethyl and cyclohexenyl.

The term "half-life" as used herein is the time it takes for the compound to lose one-half of its pharmacologic activity. The term "plasma half-life" is the time that it takes the compound to lose one-half of its pharmacologic activity in the blood plasma.

The term "treatment" refers to the combating of a disease or disorder. "Treatment" or "treating," as used herein, includes any desirable effect on the symptoms or pathology of a disease or condition as described herein, and may include even minimal changes or improvements in one or more measurable markers of the disease or condition being treated. "Treatment" or "treating" does not necessarily indicate complete eradication or cure of the disease or condition, or associated symptoms thereof. In some embodiments, the term "treatment" encompasses amelioration and prevention.

The term "amelioration" refers to moderation in the severity of the symptoms of a disease or condition. Improvement in a patient's condition, or the activity of making an effort to correct, or at least make more acceptable, conditions that are difficult to endure related to patient's conditions is considered "ameliorative" treatment.

The term "prevent" or "preventing" refers to precluding, averting, obviating, forestalling, stopping, or hindering something from happening, especially by advance action.

The term "reversal" or "reversing" refers to the ability of a compound to restore nerve-stimulated force in skeletal muscle exposed either ex vivo or in vivo to a non-depolarizing neuromuscular blocking agent or another pharmaceutical that is able to depress neuromuscular transmission

The term "ester hydrolysing reagent" refers to a chemical reagent which is capable of converting an ester functional group to a carboxylic acid with elimination of the alcohol moiety of the original ester, including but not limited to acid, base, a fluoride source, PBr3, PCl₃ and lipase enzymes.

The term "non-depolarizing blockers" refers to pharmaceutical agents that antagonize the activation of acetylcholine receptors at the post-synaptic muscle fibre membrane by blocking the acetylcholine binding site on the receptor. These agents are used to block neuromuscular transmission and induce muscle paralysis in connection with surgery.

The term "recovery of force in muscle with neuromuscular dysfunction" refers to the ability of a compound to recover contractile force in nerve-stimulated healthy rat muscle after exposure to submaximal concentration of (115 nM) tubocurarine for 90 mins. Recovery of force is quantified as the percentage of the force prior to tubocurarine that is recovered by the compound.

The term "total membrane conductance (Gm)" is the electrophysiological measure of the ability of ions to cross the muscle fibre surface membrane. It reflects the function of ion channels that are active in resting muscle fibres of which CIC-1 is known to contribute around 80 % in most animal species.

### Compounds

The goal of the invention is to provide a compound for use in treating, ameliorating and/or preventing neuromuscular disorders characterized in that the neuromuscular function is reduced. As disclosed herein, inhibition of CIC-1 improves or restores neuromuscular function. The compounds of the present invention comprise compounds capable of inhibiting the CIC-1 channel thereby improving or restoring neuromuscular function.

In one aspect, the invention relates to a compound of Formula (I): 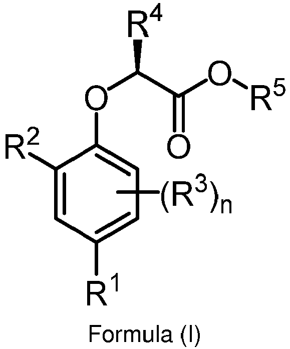 wherein:
- R¹ is selected from the group consisting of F, Cl, Br and I;
- R² is selected from the group consisting of C₂₋₆ alkyl, C₂₋₆ alkenyl, C₂₋₆ alkynyl, C₃₋₅ cycloalkyl, C₅ cycloalkenyl, -C(=O)-C₁₋₅ alkyl, -C(=O)-C₂₋₅ alkenyl, - C(=O)-C₂₋₅ alkynyl, -C(=O)-C₃₋₅ cycloalkyl and -C(=O)-C₅ cycloalkenyl, each of which may be optionally substituted with one or more, identical or different, substituents R⁶;
- R³ is selected from the group consisting of hydrogen, deuterium, tritium, F, Cl, Br, I, -CN, -CF₃, -CCl₃, -CHF₂, -CHCl₂, -CH₂F, -CH₂Cl, -OCF₃, -OCCl₃ and isocyanide;
- R⁴ is selected from the group consisting of C₁₋₅ alkyl, C₂₋₅ alkenyl, C₂₋₅ alkynyl, C₃₋₅ cycloalkyl, C₅ cycloalkenyl, each of which may be optionally substituted with one or more, identical or different, substituents R⁷;
- R⁵ is selected from the group consisting of H, C₁₋₅ alkyl optionally substituted with one or more, identical or different, substituents R¹¹, C₂₋₅ alkenyl, C₂₋₅ alkynyl, C₃₋₆ cycloalkyl optionally substituted with one or more, identical or different, substituents R¹¹, phenyl optionally substituted with one or more, identical or different, substituents R¹² and benzyl optionally substituted with one or more, identical or different, substituents R¹²;
- R⁶ is independently selected from the group consisting of hydrogen, deuterium, tritium, F, Cl, Br, I, -CN, isocyanide, -O-C₁₋₃ alkyl, -S-C₁₋₃ alkyl,
   - CH₂-OH, -CH₂-O-C₁₋₃ alkyl, -O-CH₂-Ph, -CH₂-SH and -CH₂-S-C₁₋₃ alkyl;
- R⁷ is independently selected from the group consisting of deuterium, tritium, F, Cl, Br, I, -CN, isocyanide, -O-C₁₋₃ alkyl, -S-C₁₋₃ alkyl, -CH₂-O-C₁₋₃ alkyl and -CH₂-S-C₁₋₃ alkyl;
- R¹¹ is independently selected from the group consisting of deuterium and F;
- R¹² is independently selected from the group consisting of deuterium, methoxy, nitro, cyano, Cl, Br, I and F; and
- n is an integer 0, 1, 2 or 3;
or a pharmaceutically acceptable salt, hydrate, polymorph, tautomer, or solvate thereof, with the proviso that when R² is -C(=O)-CH₃ and R¹ is Br and R⁵ is H or Me then R⁴ is not -CH3 or -CH2CH(CH3)2 and with the proviso that when R² is -CH(CH3)2 and R¹ is Br and R⁵ is H or Me then R⁴ is not -CH₃.

In one aspect, the invention relates to a compound of Formula (I), wherein:
- R¹ is selected from the group consisting of F, Cl, Br and I;
- R² is selected from the group consisting of ethyl, vinyl, ethynyl, cyclopropyl, cyclobutyl, -C(=O)-methyl and -C(=O)-ethyl, each of which may be optionally substituted with one or more, identical or different, substituents R⁶;
- R³ is selected from the group consisting of deuterium, tritium, F, Cl, Br and I;
- R⁴ is selected from the group consisting of C₁₋₅ alkyl, C₂₋₅ alkenyl, C₂₋₅ alkynyl, C₃₋₅ cycloalkyl, C₅ cycloalkenyl, each of which may be optionally substituted with one or more, identical or different, substituents R⁷;
- R⁵ is selected from the group consisting of H, C₁₋₅ alkyl optionally substituted with one or more, identical or different, substituents R¹¹, C₂₋₅ alkenyl, C₂₋₅ alkynyl, C₃₋₆ cycloalkyl optionally substituted with one or more, identical or different, substituents R¹¹, phenyl optionally substituted with one or more, identical or different, substituents R¹² and benzyl optionally substituted with one or more, identical or different, substituents R¹²;
- R⁶ is independently selected from the group consisting of deuterium, tritium, F, Cl, Br, I, -CN, isocyanide, -O-C₁₋₃ alkyl, -S-C₁₋₃ alkyl, -CH2-OH, -CH2-O-C₁₋₃ alkyl, -O-CH₂-Ph, -CH₂-SH and -CH₂-S-C₁₋₃ alkyl;
- R⁷ is independently selected from the group consisting of deuterium, tritium, F, Cl, Br, I and -O-C₁₋₃ alkyl;
- R¹¹ is independently selected from the group consisting of deuterium and F;
- R¹² is independently selected from the group consisting of deuterium, methoxy, nitro, cyano, Cl, Br, I and F; and
- n is an integer 0 or 1;
or a pharmaceutically acceptable salt, hydrate, polymorph, tautomer, or solvate thereof.

In an embodiment, R⁵ is H.

In an embodiment, R⁴ is selected from the group consisting of C₁₋₅ alkyl and C₃₋₅ cycloalkyl, each of which may be optionally substituted with one or more, identical or different, substituents R⁷.

In an embodiment, R⁶ is selected from the group consisting of deuterium, F and -O-C₁₋₃ alkyl.

In an embodiment, R⁷ is selected from the group consisting of deuterium, F and -O-C₁₋₃ alkyl.

In an embodiment, R⁵ is H and R⁴ is selected from the group consisting of C₁₋₅ alkyl and C₃₋₅ cycloalkyl, each of which may be optionally substituted with one or more, identical or different, substituents R⁷.

In an embodiment, R⁵ is H and R⁴ is selected from the group consisting of C₁₋₅ alkyl and C₃₋₅ cycloalkyl, each of which may be optionally substituted with one or more, identical or different, substituents R⁷, wherein R⁷ is selected from the group consisting of deuterium, F and -O-C₁₋₃ alkyl.

In an embodiment, R⁵ is H, R⁶ is selected from the group consisting of deuterium, F and -O-C₁₋₃ alkyl and R⁴ is selected from the group consisting of C₁₋₅ alkyl and C₃₋₅ cycloalkyl, each of which may be optionally substituted with one or more, identical or different, substituents R⁷, wherein R⁷ is selected from the group consisting of deuterium, F and -O-C₁₋₃ alkyl.

In one embodiment, the invention relates to a compound of Formula (II): 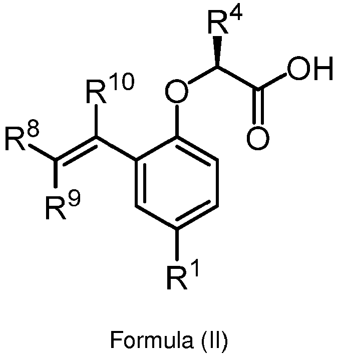 wherein:
R¹ is selected from the group consisting of F, Cl, Br and I;
R⁴ is methyl, ethyl, n-propyl, isopropyl or -CH2F; and
R⁸ and R⁹ are independently selected from the group consisting of hydrogen, deuterium, tritium, F, Cl, Br, I, -CN, isocyanide, -O-C₁₋₃ alkyl, -S-C₁₋₃ alkyl, -CH2-OH, -CH₂-O-C₁₋₃ alkyl, -CH2-SH, -CH₂-S-C₁₋₃ alkyl, C₁₋₄ alkyl and C₂₋₄ alkenyl and wherein the C₁₋₄ alkyl and C₂₋₄ alkenyl group may be optionally substituted with one or more, identical or different, substituents R⁶; and
R⁶ is independently selected from the group consisting of hydrogen, deuterium, tritium, F, Cl, Br, I, -CN, isocyanide, -O-C₁₋₃ alkyl, -S-C₁₋₃ alkyl, -CH2-OH, -CH₂-O-C₁₋₃ alkyl, -O-CH₂-Ph, -CH2-SH and -CH₂-S-C₁₋₃ alkyl;
or a pharmaceutically acceptable salt, hydrate, polymorph, tautomer, or solvate thereof.

In one embodiment, the invention relates to a compound of Formula (III): 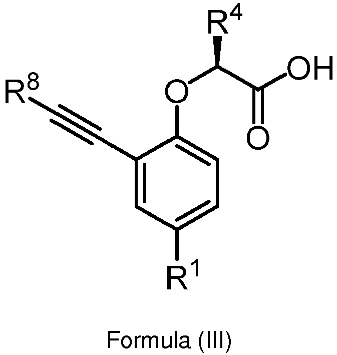 wherein:
R¹ is selected from the group consisting of F, Cl, Br and I;
R⁴ is methyl, ethyl, n-propyl, isopropyl or -CH2F;
R⁸ is independently selected from the group consisting of hydrogen, deuterium, tritium, F, Cl, Br, I, -CN, isocyanide, -O-C₁₋₃ alkyl, -S-C₁₋₃ alkyl, -CH2-OH, -CH₂-O-C₁₋₃ alkyl, -CH2-SH, -CH₂-S-C₁₋₃ alkyl, C₁₋₄ alkyl and C₂₋₄ alkenyl and wherein the C₁₋₄ alkyl and C₂₋₄ alkenyl group may be optionally substituted with one or more, identical or different, substituents R⁶; and
R⁶ is independently selected from the group consisting of hydrogen, deuterium, tritium, F, Cl, Br, I, -CN, isocyanide, -O-C₁₋₃ alkyl, -S-C₁₋₃ alkyl, -CH2-OH, -CH₂-O-C₁₋₃ alkyl, -O-CH₂-Ph, -CH2-SH and -CH₂-S-C₁₋₃ alkyl;
or a pharmaceutically acceptable salt, hydrate, polymorph, tautomer, or solvate thereof.

In one embodiment, the invention relates to a compound of Formula (IV): 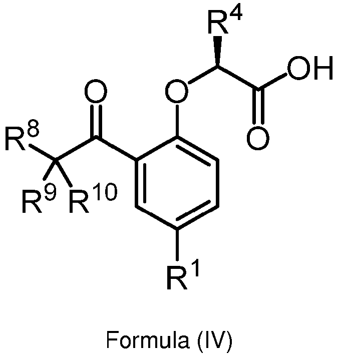 wherein:
R¹ is selected from the group consisting of F, Cl, Br and I;
R⁴ is methyl, ethyl, n-propyl, isopropyl or -CH2F;
R⁶ is independently selected from the group consisting of hydrogen, deuterium, tritium, F, Cl, Br, I, -CN, isocyanide, -O-C₁₋₃ alkyl, -S-C₁₋₃ alkyl, -CH2-OH, -CH₂-O-C₁₋₃ alkyl, -O-CH₂-Ph, -CH2-SH and -CH₂-S-C₁₋₃ alkyl; and
R⁸, R⁹ and R¹⁰ are independently selected from the group consisting of hydrogen, deuterium, tritium, F, Cl, Br, I, -CN, isocyanide, -O-C₁₋₃ alkyl, - S-C₁₋₃ alkyl, -CH2-OH, -CH₂-O-C₁₋₃ alkyl, -CH2-SH, -CH₂-S-C₁₋₃ alkyl, C₁₋₄ alkyl and C₂₋₄ alkenyl and wherein the C₁₋₄ alkyl and C₁₋₄ alkenyl group may be optionally substituted with one or more, identical or different, substituents R⁶;
or a pharmaceutically acceptable salt, hydrate, polymorph, tautomer, or solvate thereof.

In one embodiment, the invention relates to a compound of Formula (V): 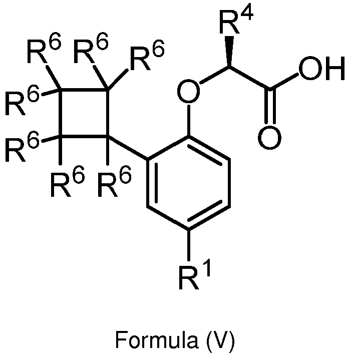 wherein:
R¹ is selected from the group consisting of F, Cl, Br and I;
R⁴ is methyl, ethyl, n-propyl, isopropyl or -CH2F; and
R⁶ is independently selected from the group consisting of hydrogen, deuterium, tritium, F, Cl, Br, I, -CN, isocyanide, -O-C₁₋₃ alkyl, -S-C₁₋₃ alkyl, -CH2-OH, -CH₂-O-C₁₋₃ alkyl, -O-CH₂-Ph, -CH2-SH and -CH₂-S-C₁₋₃ alkyl;
or a pharmaceutically acceptable salt, hydrate, polymorph, tautomer, or solvate thereof.

In one embodiment, the invention relates to a compound of Formula (VI): 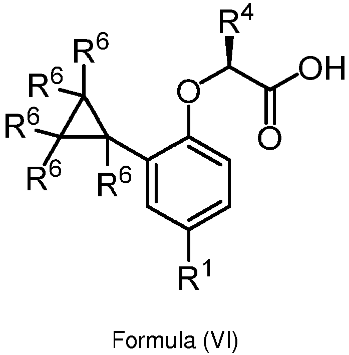 wherein:
R¹ is selected from the group consisting of F, Cl, Br and I;
R⁴ is methyl, ethyl, n-propyl, isopropyl or -CH2F; and
R⁶ is independently selected from the group consisting of hydrogen, deuterium, tritium, F, Cl, Br, I, -CN, isocyanide, -O-C₁₋₃ alkyl, -S-C₁₋₃ alkyl, -CH2-OH, -CH₂-O-C₁₋₃ alkyl, -O-CH₂-Ph, -CH2-SH and -CH₂-S-C₁₋₃ alkyl;
or a pharmaceutically acceptable salt, hydrate, polymorph, tautomer, or solvate thereof.

In one embodiment, the invention relates to a compound of Formula (VII): 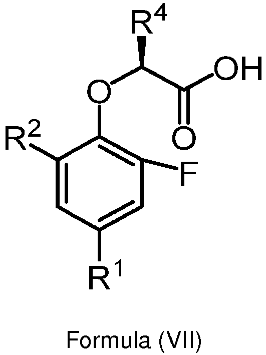 wherein:
R¹ is selected from the group consisting of F, Cl, Br and I;
R² is selected from the group consisting of C₂₋₆ alkyl, C₂₋₆ alkenyl, C₂₋₆ alkynyl, C₃₋₅ cycloalkyl, C₅ cycloalkenyl, -C(=O)-C₁₋₅ alkyl, -C(=O)-C₂₋₅ alkenyl, - C(=O)-C₂₋₅ alkynyl, -C(=O)-C₃₋₅ cycloalkyl and -C(=O)-C₅ cycloalkenyl, each of which may be optionally substituted with one or more, identical or different, substituents R⁶;
R⁴ is selected from the group consisting of C₁₋₅ alkyl, C₂₋₅ alkenyl, C₂₋₅ alkynyl, C₃₋₅ cycloalkyl, C₅ cycloalkenyl, each of which may be optionally substituted with one or more, identical or different, substituents R⁷;
R⁶ is independently selected from the group consisting of hydrogen, deuterium, tritium, F, Cl, Br, I, -CN, isocyanide, -O-C₁₋₃ alkyl, -S-C₁₋₃ alkyl, -CH2-OH, -CH₂-O-C₁₋₃ alkyl, -O-CH₂-Ph, -CH2-SH and -CH₂-S-C₁₋₃ alkyl; and
R⁷ is independently selected from the group consisting of deuterium, tritium, F, Cl, Br, I, -CN, isocyanide, -O-C₁₋₃ alkyl, -S-C₁₋₃ alkyl, -CH₂-O-C₁₋₃ alkyl and -CH₂-S-C₁₋₃ alkyl;
or a pharmaceutically acceptable salt, hydrate, polymorph, tautomer, or solvate thereof.

In one embodiment, the invention relates to a compound of Formula (VIII): 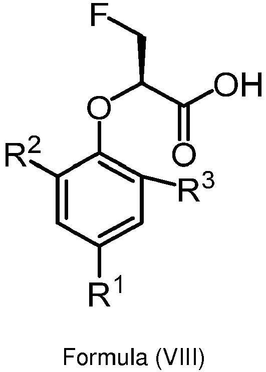 wherein:
R¹ is selected from the group consisting of F, Cl, Br and I;
R² is selected from the group consisting of C₂₋₆ alkyl, C₂₋₆ alkenyl, C₂₋₆ alkynyl, C₃₋₅ cycloalkyl, C₅ cycloalkenyl, -C(=O)-C₁₋₅ alkyl, -C(=O)-C₂₋₅ alkenyl, - C(=O)-C₂₋₅ alkynyl, -C(=O)-C₃₋₅ cycloalkyl and -C(=O)-C₅ cycloalkenyl, each of which may be optionally substituted with one or more, identical or different, substituents R⁶;
R³ is selected from the group consisting of hydrogen, deuterium, tritium, F, Cl, Br, I, -CN, -CF₃, -CCl₃, -CHF₂, -CHCl₂, -CH₂F, -CH₂Cl, -OCF₃, -OCCl₃ and isocyanide; and
R⁶ is independently selected from the group consisting of hydrogen, deuterium, tritium, F, Cl, Br, I, -CN, isocyanide, -O-C₁₋₃ alkyl, -S-C₁₋₃ alkyl, -CH2-OH, -CH₂-O-C₁₋₃ alkyl, -O-CH₂-Ph, -CH2-SH and -CH₂-S-C₁₋₃ alkyl;
or a pharmaceutically acceptable salt, hydrate, polymorph, tautomer, or solvate thereof.

In one aspect, the invention relates to a compound of Formula (I): 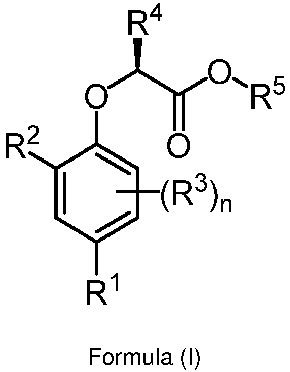 wherein:
- R¹ is selected from the group consisting of F, Cl, Br, I, -CN, -CF3, -CCl₃, - CHF₂, -CHCl₂, -CH₂F, -CH₂Cl, -OCF₃ and -OCCl₃;
- R² is selected from the group consisting of C₂₋₆ alkyl, C₂₋₆ alkenyl, C₂₋₆ alkynyl, C₃₋₆ cycloalkyl, C₅₋₆ cycloalkenyl, -C(=O)-C₁₋₅ alkyl, -C(=O)-C₂₋₅ alkenyl, -C(=O)-C₂₋₅ alkynyl, -C(=O)-C₃₋₅ cycloalkyl and -C(=O)-C₅ cycloalkenyl, each of which may be optionally substituted with one or more, identical or different, substituents R⁶;
- R³ is selected from the group consisting of hydrogen, deuterium, tritium, F, Cl, Br, I, -CN, -CF₃, -CCl₃, -CHF₂, -CHCl₂, -CH₂F, -CH₂Cl, -OCF₃, -OCCl₃ and isocyanide;
- R⁴ is H;
- R⁵ is selected from the group consisting of H, C₁₋₅ alkyl optionally substituted with one or more, identical or different, substituents R¹¹, C₂₋₅ alkenyl, C₂₋₅ alkynyl, C₃₋₆ cycloalkyl optionally substituted with one or more, identical or different, substituents R¹¹, phenyl optionally substituted with one or more, identical or different, substituents R¹² and benzyl optionally substituted with one or more, identical or different, substituents R¹²;
- R⁶ is independently selected from the group consisting of hydrogen, deuterium, tritium, F, Cl, Br, I, -CN, isocyanide, -O-C₁₋₃ alkyl, -S-C₁₋₃ alkyl, -CH2-OH, -CH₂-O-C₁₋₃ alkyl, -O-CH₂-Ph, -CH2-SH and -CH₂-S-C₁₋₃ alkyl;
- R¹¹ is independently selected from the group consisting of deuterium and F;
- R¹² is independently selected from the group consisting of deuterium, methoxy, nitro, cyano, Cl, Br, I and F; and
- n is an integer 0, 1, 2 or 3;
or a pharmaceutically acceptable salt, hydrate, polymorph, tautomer, or solvate thereof.

In a specific embodiment, the compound is selected from the group consisting of:

| | |
|---|---|
| 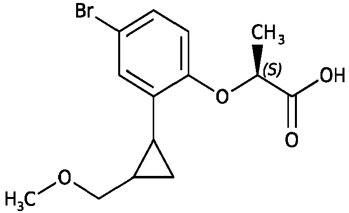 | 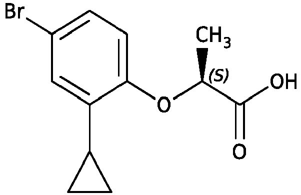 |
| Compound A-1 | Compound A-2 |
| 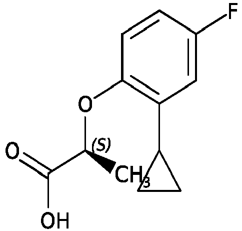 | 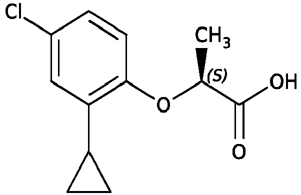 |
| Compound A-3 | Compound A-4 |
| 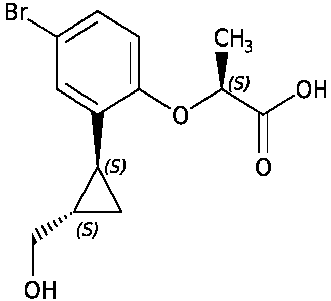 | 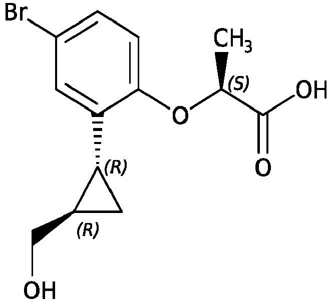 |
| Compound A-5 | Compound A-6 |
| 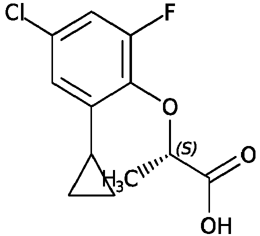 | 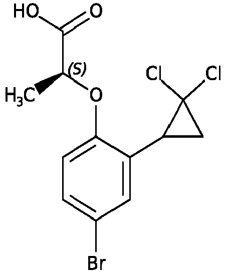 |
| Compound A-7 | Compound A-8 |
| 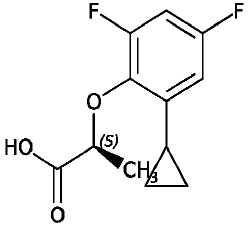 | 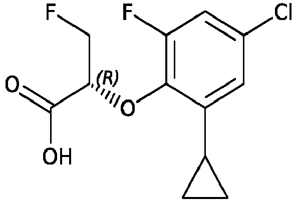 |
| Compound A-9 | Compound A-10 |
| 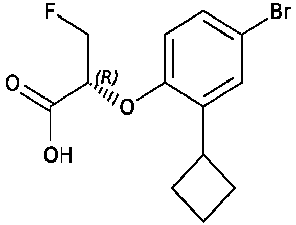 | 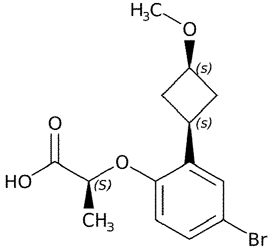 |
| Compound A-11 | Compound A-12 |
| 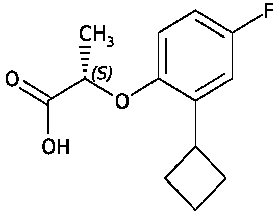 | 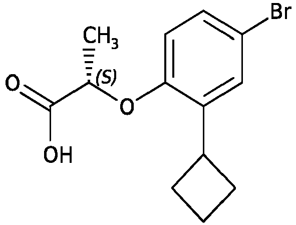 |
| Compound A-13 | Compound A-14 |
| 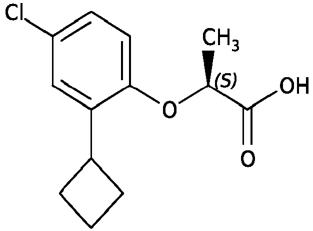 | 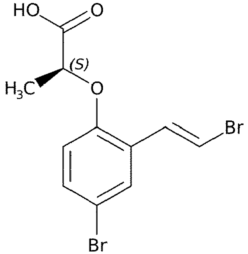 |
| Compound A-15 | Compound A-16 |
| 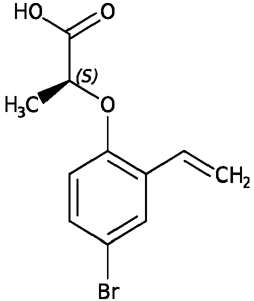 | 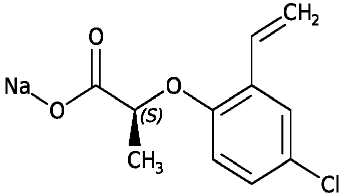 |
| Compound A-17 | Compound A-18 |
| 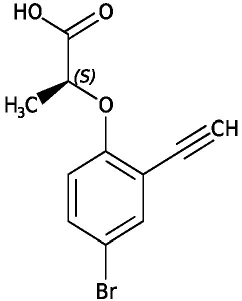 | 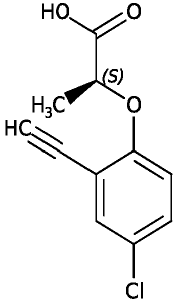 |
| Compound A-19 | Compound A-20 |
| 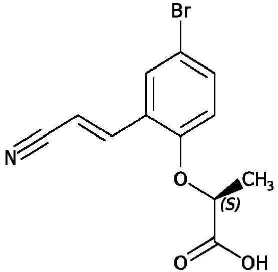 | 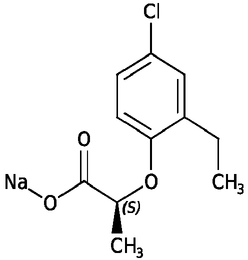 |
| Compound A-21 | Compound A-22 |
| 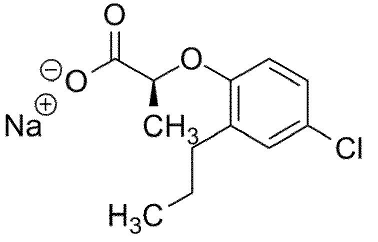 | 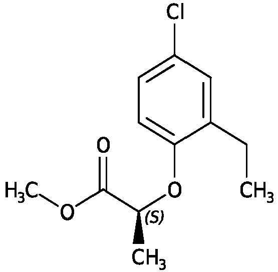 |
| Compound A-23 | Compound A-24 |
| 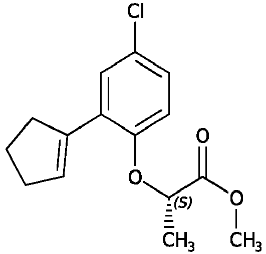 | 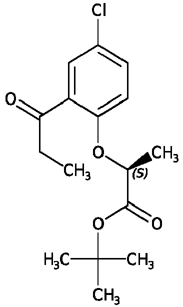 |
| Compound A-25 | Compound A-26 |
| 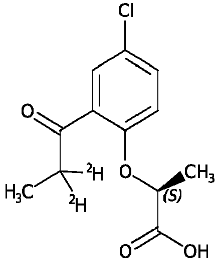 | 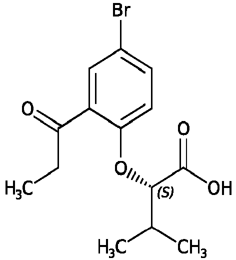 |
| Compound A-27 | Compound A-28 |
| 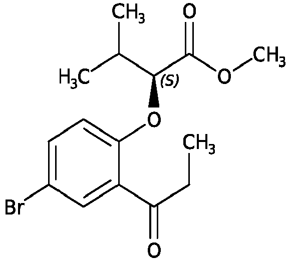 | 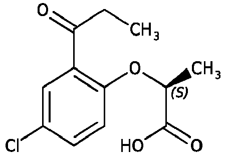 |
| Compound A-29 | Compound A-30 |
| 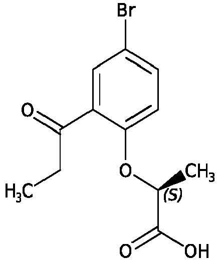 | 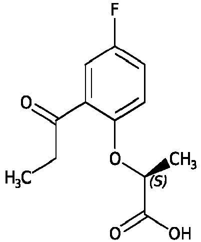 |
| Compound A-31 | Compound A-32 |
| 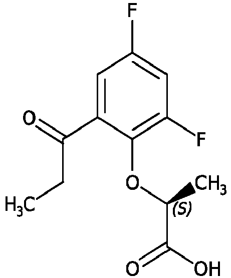 | 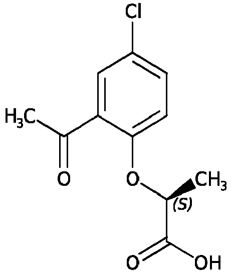 |
| Compound A-33 | Compound A-34 |
| 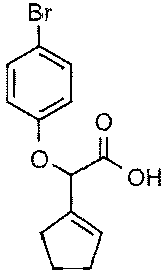 | 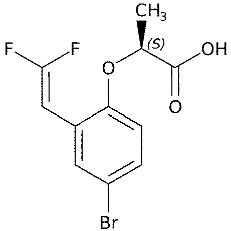 |
| Compound A-35 | Compound A-36 |
| 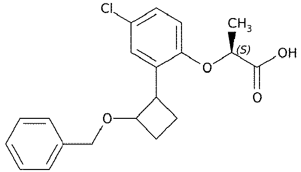 | 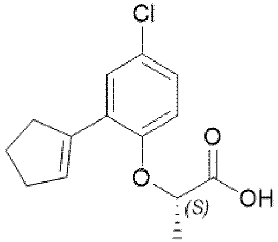 |
| Compound A-37 | Compound A-38 |

In a specific embodiment, the compound is selected from the group consisting of:
(2S)-2-{4-bromo-2-[2-(methoxymethyl)cyclopropyl]phenoxy}propanoic acid;
(2S)-2-[4-bromo-2-(2,2-dichlorocyclopropyl)phenoxy]propanoic acid;
(2S)-2-{4-bromo-2-[(1s,3s)-3-methoxycyclobutyl]phenoxy}propanoic acid;
(2S)-2-{4-bromo-2-[(E)-2-bromoethenyl]phenoxy}propanoic acid;
(2*R*)-2-(4-bromo-2-cyclobutylphenoxy)-3-fluoropropanoic acid;
(2*S*)-2-{4-bromo-2-[(1*S*,2*S*)-2-(hydroxymethyl)cyclopropyl]phenoxy}propanoic acid;
(2*S*)-2-{4-bromo-2-[(1*R*,2*R*)-2-(hydroxymethyl)cyclopropyl]phenoxy}propanoic acid;
(2S)-2-(4-bromo-2-ethynylphenoxy)propanoic acid;
(2*S*)-2-{4-bromo-2-[(1 *E*)-2-cyanoeth-1-en-1 -yl]phenoxy}propanoic acid;
(2S)-2-(4-bromo-2-cyclopropylphenoxy)propanoic acid;
(2S)-2-(4-bromo-2-ethenylphenoxy)propanoic acid;
(2S)-2-(2-cyclopropyl-4-fluorophenoxy)propanoic acid;
(2S)-2-(2-cyclobutyl-4-fluorophenoxy)propanoic acid;
(2S)-2-(4-bromo-2-cyclobutylphenoxy)propanoic acid;
(2S)-2-(4-chloro-2-cyclobutylphenoxy)propanoic acid;
tert-butyl (2S)-2-(4-chloro-2-propanoylphenoxy)propanoate;
(2*S*)-2-{4-chloro-2-[(2,2-²H₂)propanoyl]phenoxy}propanoic acid;
(2S)-2-(4-bromo-2-propanoylphenoxy)-3-methylbutanoic acid;
methyl (2*S*)-2-[4-chloro-2-(cyclopent-1-en-1-yl)phenoxy]propanoate;
methyl (2S)-2-(4-bromo-2-propanoylphenoxy)-3-methylbutanoate;
(2S)-2-(4-chloro-2-ethynylphenoxy)propanoic acid;
(2S)-2-(4-chloro-2-propanoylphenoxy)propanoic acid;
sodium (2S)-2-(4-chloro-2-ethenylphenoxy)propanoate;
(2S)-2-(4-chloro-2-cyclopropylphenoxy)propanoic acid;
sodium (2S)-2-(4-chloro-2-propylphenoxy)propanoic acid;
sodium (2S)-2-(4-chloro-2-ethylphenoxy)propanoate;
methyl (2S)-2-(4-chloro-2-ethylphenoxy)propanoate;
(2*R*)-2-(4-chloro-2-cyclopropyl-6-fluorophenoxy)-3-fluoropropanoic acid;
(2*S*)-2-(2-cyclopropyl-4,6-difluorophenoxy)propanoic acid;
(2S)-2-(4-bromo-2-propanoylphenoxy)propanoic acid;
(2*S*)-2-(4-chloro-2-cyclopropyl-6-fluorophenoxy)propanoic acid;
(2S)-2-(2,4-difluoro-6-propanoylphenoxy)propanoic acid;
(2S)-2-(2-acetyl-4-chlorophenoxy)propanoic acid;
(2S)-2-(4-fluoro-2-propanoylphenoxy)propanoic acid;
(2*S*)-2-[4-bromo-2-(cyclopent-1-en-1-yl)phenoxy]propanoic acid;
(2S)-2-[4-bromo-2-(2,2-difluoroethenyl)phenoxy]propanoic acid;
(2*S*)-2-{2-[2-(benzyloxy)cyclobutyl]-4-chlorophenoxy}propanoic acid;
(2*S*)-2-[4-bromo-2-(cyclopent-1-en-1-yl)phenoxy]propanoic acid;
(2S)-2-[4-bromo-2-(2-methoxyethyl)phenoxy]propanoic acid;
(2S)-2-[2,4-dibromo-6-(2-methoxyethyl)phenoxy]propanoic acid;
(2S)-2-[4-bromo-2-(cyclopropylidenemethyl)phenoxy]propanoic acid;
(2S)-2-(4-bromo-2-ethenyl-5-fluorophenoxy)propanoic acid;
(2S)-2-(2-acetyl-4-bromo-5-fluorophenoxy)propanoic acid;
(2S)-2-(4-bromo-2-cyclopropyl-5-fluorophenoxy)propanoic acid;
(2S)-2-[4-bromo-2-(2,2-difluoroethenyl)-5-fluorophenoxy]propanoic acid;
(2S)-2-(4-bromo-2-ethynylphenoxy)-2-cyclobutylacetic acid;
(2S)-2-(4-bromo-2-ethynyl-5-fluorophenoxy)propanoic acid;
(2S)-2-(4-chloro-2-ethynyl-5-fluorophenoxy)propanoic acid;
(2S)-2-(4-bromo-2-cyclopropylphenoxy)-4-fluorobutanoic acid;
(2S)-2-(4-bromo-2-ethynylphenoxy)-4-fluorobutanoic acid;
(2S)-2-(4-bromo-2-ethenylphenoxy)-4-fluorobutanoic acid;
(2*S*)-2-{4-bromo-2-[(1 *E*)-2-fluoroethenyl]phenoxy}propanoic acid;
(2*S*)-2-{4-chloro-2-[(1 *E*)-2-fluoroethenyl]phenoxylpropanoic acid;
(2S)-2-(4-bromo-2-ethynylphenoxy)butanoic acid;
2-(4-bromo-2-cyclopropylphenoxy)acetic acid;
2-(4-bromo-2-ethynylphenoxy)acetic acid, and
(2S)-2-[4-bromo-2-(2,2-difluorocyclobutyl)phenoxy]propanoic acid.

### Methods of Treatment

In one aspect, the invention relates to the use of compounds of Formula (I) in treating, ameliorating and/or preventing a neuromuscular disorder. In one aspect, the invention relates to the use of compounds of Formula (I) in reversing and/or ameliorating a neuromuscular blockade. Thus in one aspect, the invention relates to a compound of Formula (I): 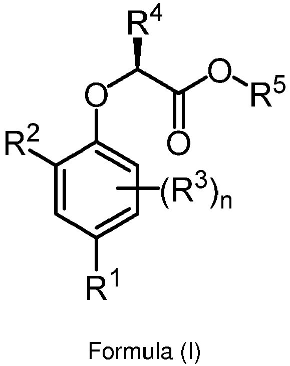 wherein:
- R¹ is selected from the group consisting of F, Cl, Br, I, -CN, -CF3, -CCl₃, - CHF₂, -CHCl₂, -CH₂F, -CH₂Cl, -OCF₃ and -OCCl₃;
- R² is selected from the group consisting of C₂₋₆ alkyl, C₂₋₆ alkenyl, C₂₋₆ alkynyl, C₃₋₆ cycloalkyl, C₅₋₆ cycloalkenyl, -C(=O)-C₁₋₅ alkyl, -C(=O)-C₁₋₅ alkenyl, -C(=O)-C₁₋₅ alkynyl, -C(=O)-C₃₋₅ cycloalkyl and -C(=O)-C₅ cycloalkenyl, each of which may be optionally substituted with one or more, identical or different, substituents R⁶;
- R³ is selected from the group consisting of hydrogen, deuterium, tritium, F, Cl, Br, I, -CN, -CF3, -CCl₃, -CHF₂, -CHCl₂, -CH₂F, -CH₂Cl, -OCF₃, -OCCl₃ and isocyanide;
- R⁴ is selected from the group consisting of C₁₋₅ alkyl, C₁₋₅ alkenyl, C₁₋₅ alkynyl, C₃₋₅ cycloalkyl, C₅ cycloalkenyl, each of which may be optionally substituted with one or more, identical or different, substituents R⁷;
- R⁵ is selected from the group consisting of H, C₁₋₅ alkyl optionally substituted with one or more, identical or different, substituents R¹¹, C₂₋₅ alkenyl, C₂₋₅ alkynyl, C₃₋₆ cycloalkyl optionally substituted with one or more, identical or different, substituents R¹¹, phenyl optionally substituted with one or more, identical or different, substituents R¹² and benzyl optionally substituted with one or more, identical or different, substituents R¹²;
- R⁶ is independently selected from the group consisting of hydrogen, deuterium, tritium, F, Cl, Br, I, -CN, isocyanide, -O-C₁₋₃ alkyl, -S-C₁₋₃ alkyl, -CH2-OH, -CH₂-O-C₁₋₃ alkyl, -O-CH₂-Ph, -CH2-SH and -CH₂-S-C₁₋₃ alkyl;
- R⁷ is independently selected from the group consisting of deuterium, tritium, F, Cl, Br, I, -CN, isocyanide, -O-C₁₋₃ alkyl, -S-C₁₋₃ alkyl, -CH₂-O-C₁₋₃ alkyl and -CH₂-S-C₁₋₃ alkyl;
- R¹¹ is independently selected from the group consisting of deuterium and F;
- R¹² is independently selected from the group consisting of deuterium, methoxy, nitro, cyano, Cl, Br, I and F; and
- n is an integer 0, 1, 2 or 3;
or a pharmaceutically acceptable salt, hydrate, polymorph, tautomer, or solvate thereof, for use in treating, ameliorating and/or preventing a neuromuscular disorder, and/or for use in reversing and/or ameliorating a neuromuscular blockade.

In one aspect, the invention relates to a compound of Formula (I), wherein:
- R¹ is selected from the group consisting of F, Cl, Br and I;
- R² is selected from the group consisting of ethyl, vinyl, ethynyl, cyclopropyl, cyclobutyl, -C(=O)-methyl and -C(=O)-ethyl, each of which may be optionally substituted with one or more, identical or different, substituents R⁶;
- R³ is selected from the group consisting of deuterium, tritium, F, Cl, Br and I;
- R⁴ is selected from the group consisting of C₁₋₅ alkyl, C₂₋₅ alkenyl, C₂₋₅ alkynyl, C₃₋₅ cycloalkyl, C₅ cycloalkenyl, each of which may be optionally substituted with one or more, identical or different, substituents R⁷;
- R⁵ is selected from the group consisting of H, C₁₋₅ alkyl optionally substituted with one or more, identical or different, substituents R¹¹, C₂₋₅ alkenyl, C₂₋₅ alkynyl, C₃₋₆ cycloalkyl optionally substituted with one or more, identical or different, substituents R¹¹, phenyl optionally substituted with one or more, identical or different, substituents R¹² and benzyl optionally substituted with one or more, identical or different, substituents R¹²;
- R⁶ is independently selected from the group consisting of deuterium, tritium, F, Cl, Br, I, -CN, isocyanide, -O-C₁₋₃ alkyl, -S-C₁₋₃ alkyl, -CH₂-OH, -CH₂-O-C₁₋₃ alkyl, -O-CH₂-Ph, -CH₂-SH and -CH₂-S-C₁₋₃ alkyl;
- R⁷ is independently selected from the group consisting of deuterium, tritium, F, Cl, Br and I;
- R¹¹ is independently selected from the group consisting of deuterium and F;
- R¹² is independently selected from the group consisting of deuterium, methoxy, nitro, cyano, Cl, Br, I and F; and
- n is an integer 0 or 1,
or a pharmaceutically acceptable salt, hydrate, polymorph, tautomer, or solvate thereof, for use in treating, ameliorating and/or preventing a neuromuscular disorder, and/or for use in reversing and/or ameliorating a neuromuscular blockade.

In an embodiment, R² is selected from the group consisting of C₂₋₆ alkyl, C₂₋₆ alkenyl, C₂₋₆ alkynyl, C₃₋₅ cycloalkyl, C₅ cycloalkenyl, -C(=O)-C₁₋₅ alkyl, -C(=O)-C₁₋₅ alkenyl, - C(=O)-C₁₋₅ alkynyl, -C(=O)-C₃₋₅ cycloalkyl and -C(=O)-C₅ cycloalkenyl, each of which may be optionally substituted with one or more, identical or different, substituents R⁶.

In an embodiment, R⁵ is H.

In an embodiment, R⁴ is selected from the group consisting of C₁₋₅ alkyl and C₃₋₅ cycloalkyl, each of which may be optionally substituted with one or more, identical or different, substituents R⁷.

In an embodiment, R⁶ is selected from the group consisting of deuterium, F and -O-C₁₋₃ alkyl.

In an embodiment, R⁷ is selected from the group consisting of deuterium, F and -O-C₁₋₃ alkyl.

In an embodiment, R⁵ is H and R⁴ is selected from the group consisting of C₁₋₅ alkyl and C₃₋₅ cycloalkyl, each of which may be optionally substituted with one or more, identical or different, substituents R⁷.

In an embodiment, R⁵ is H and R⁴ is selected from the group consisting of C₁₋₅ alkyl and C₃₋₅ cycloalkyl, each of which may be optionally substituted with one or more, identical or different, substituents R⁷, wherein R⁷ is selected from the group consisting of deuterium, F and -O-C₁₋₃ alkyl.

In an embodiment, R⁵ is H, R⁶ is selected from the group consisting of deuterium, F and -O-C₁₋₃ alkyl and R⁴ is selected from the group consisting of C₁₋₅ alkyl and C₃₋₅ cycloalkyl, each of which may be optionally substituted with one or more, identical or different, substituents R⁷, wherein R⁷ is selected from the group consisting of deuterium, F and -O-C₁₋₃ alkyl.

In one embodiment, the invention is related to a compound of Formula (II): 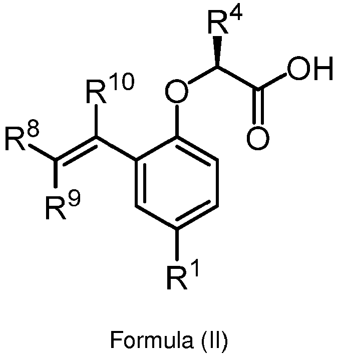 wherein:
R¹ is selected from the group consisting of F, Cl, Br, I, -CN, -CF₃, -CCl₃, - CHF₂, -CHCl₂, -CH₂F, -CH₂Cl, -OCF₃ and -OCCl₃;
R⁴ is methyl, ethyl, n-propyl, isopropyl or -CH2F;
R⁸ and R⁹ are independently selected from the group consisting of hydrogen, deuterium, tritium, F, Cl, Br, I, -CN, isocyanide, -O-C₁₋₃ alkyl, -S-C₁₋₃ alkyl, -CH₂-OH, -CH₂-O-C₁₋₃ alkyl, -CH₂-SH, -CH₂-S-C₁₋₃ alkyl, C₁₋₄ alkyl and C₂₋₄ alkenyl and wherein the C₁₋₄ alkyl and C₂₋₄ alkenyl group may be optionally substituted with one or more, identical or different, substituents R⁶; and
R⁶ is independently selected from the group consisting of hydrogen, deuterium, tritium, F, Cl, Br, I, -CN, isocyanide, -O-C₁₋₃ alkyl, -S-C₁₋₃ alkyl, -CH2-OH, -CH₂-O-C₁₋₃ alkyl, -O-CH₂-Ph, -CH₂-SH and -CH₂-S-C₁₋₃ alkyl;
or a pharmaceutically acceptable salt, hydrate, polymorph, tautomer, or solvate thereof, for use in treating, ameliorating and/or preventing a neuromuscular disorder, and/or for use in reversing and/or ameliorating a neuromuscular blockade.

In one embodiment, the invention is related to a compound of Formula (III): 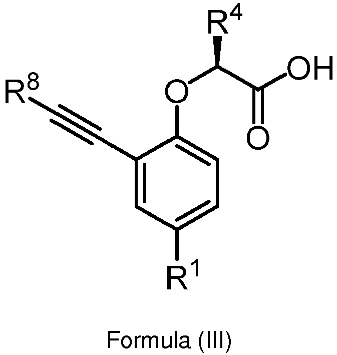 wherein:
R¹ is selected from the group consisting of F, Cl, Br, I, -CN, -CF₃, -CCl₃, - CHF₂, -CHCl₂, -CH₂F, -CH₂Cl, -OCF₃ and -OCCl₃;
R⁴ is methyl, ethyl, n-propyl, isopropyl or -CH2F;
R⁸ is independently selected from the group consisting of hydrogen, deuterium, tritium, F, Cl, Br, I, -CN, isocyanide, -O-C₁₋₃ alkyl, -S-C₁₋₃ alkyl, -CH2-OH, -CH₂-O-C₁₋₃ alkyl, -CH₂-SH, -CH₂-S-C₁₋₃ alkyl, C₁₋₄ alkyl and C₂₋₄ alkenyl and wherein the C₁₋₄ alkyl and C₂₋₄ alkenyl group may be optionally substituted with one or more, identical or different, substituents R⁶; and
R⁶ is independently selected from the group consisting of hydrogen, deuterium, tritium, F, Cl, Br, I, -CN, isocyanide, -O-C₁₋₃ alkyl, -S-C₁₋₃ alkyl, -CH₂-OH, -CH₂-O-C₁₋₃ alkyl, -O-CH₂-Ph, -CH₂-SH and -CH₂-S-C₁₋₃ alkyl,
or a pharmaceutically acceptable salt, hydrate, polymorph, tautomer, or solvate thereof, for use in treating, ameliorating and/or preventing a neuromuscular disorder, and/or for use in reversing and/or ameliorating a neuromuscular blockade.

In one embodiment, the invention is related to a compound of Formula (IV): 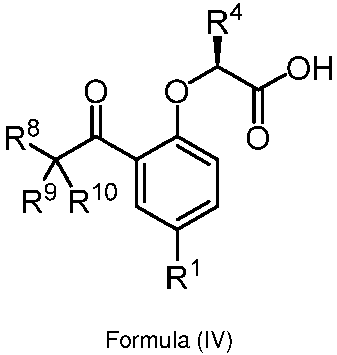 wherein:
R¹ is selected from the group consisting of F, Cl, Br, I, -CN, -CF₃, -CCl₃, - CHF₂, -CHCl₂, -CH₂F, -CH₂Cl, -OCF₃ and -OCCl₃;
R⁴ is methyl, ethyl, n-propyl, isopropyl or -CH2F;
R⁶ is independently selected from the group consisting of hydrogen, deuterium, tritium, F, Cl, Br, I, -CN, isocyanide, -O-C₁₋₃ alkyl, -S-C₁₋₃ alkyl, -CH₂-OH, -CH₂-O-C₁₋₃ alkyl, -O-CH₂-Ph, -CH₂-SH and -CH₂-S-C₁₋₃ alkyl; and
R⁸, R⁹ and R¹⁰ are independently selected from the group consisting of hydrogen, deuterium, tritium, F, Cl, Br, I, -CN, isocyanide, -O-C₁₋₃ alkyl, - S-C₁₋₃ alkyl, -CH₂-OH, -CH₂-O-C₁₋₃ alkyl, -CH₂-SH, -CH₂-S-C₁₋₃ alkyl, C₁₋₄ alkyl and C₂₋₄ alkenyl and wherein the C₁₋₄ alkyl and C₂₋₄ alkenyl group may be optionally substituted with one or more, identical or different, substituents R⁶;
or a pharmaceutically acceptable salt, hydrate, polymorph, tautomer, or solvate thereof, for use in treating, ameliorating and/or preventing a neuromuscular disorder, and/or for use in reversing and/or ameliorating a neuromuscular blockade.

In one embodiment, the invention is related to a compound of Formula (V): 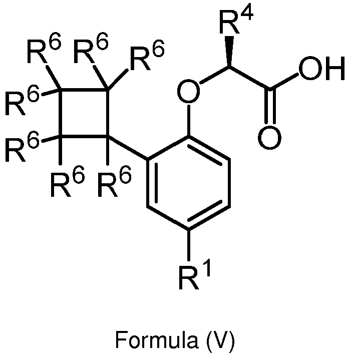 wherein:
R¹ is selected from the group consisting of F, Cl, Br, I, -CN, -CF₃, -CCl₃, - CHF₂, -CHCl₂, -CH₂F, -CH₂Cl, -OCF₃ and -OCCl₃;
R⁴ is methyl, ethyl, n-propyl, isopropyl or -CH2F; and
R⁶ is independently selected from the group consisting of hydrogen, deuterium, tritium, F, Cl, Br, I, -CN, isocyanide, -O-C₁₋₃ alkyl, -S-C₁₋₃ alkyl, -CH₂-OH, -CH₂-O-C₁₋₃ alkyl, -O-CH₂-Ph, -CH₂-SH and -CH₂-S-C₁₋₃ alkyl,
or a pharmaceutically acceptable salt, hydrate, polymorph, tautomer, or solvate thereof, for use in treating, ameliorating and/or preventing a neuromuscular disorder, and/or for use in reversing and/or ameliorating a neuromuscular blockade.

In one embodiment, the invention is related to a compound of Formula (VI): 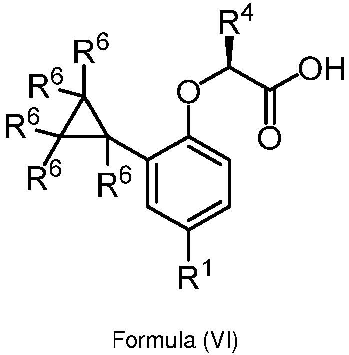 wherein:
R¹ is selected from the group consisting of F, Cl, Br, I, -CN, -CF3, -CCl₃, - CHF₂, -CHCl₂, -CH₂F, -CH₂Cl, -OCF₃ and -OCCl₃;
R⁴ is methyl, ethyl, n-propyl, isopropyl or -CH2F; and
R⁶ is independently selected from the group consisting of hydrogen, deuterium, tritium, F, Cl, Br, I, -CN, isocyanide, -O-C₁₋₃ alkyl, -S-C₁₋₃ alkyl, -CH₂-OH, -CH₂-O-C₁₋₃ alkyl, -O-CH₂-Ph, -CH₂-SH and -CH₂-S-C₁₋₃ alkyl,
or a pharmaceutically acceptable salt, hydrate, polymorph, tautomer, or solvate thereof, for use in treating, ameliorating and/or preventing a neuromuscular disorder, and/or for use in reversing and/or ameliorating a neuromuscular blockade.

In one embodiment, the invention is related to a compound of Formula (VII): 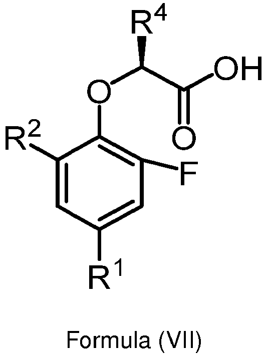 wherein:
R¹ is selected from the group consisting of F, Cl, Br, I, -CN, -CF₃, -CCl₃, - CHF₂, -CHCl₂, -CH₂F, -CH₂Cl, -OCF₃ and -OCCl₃;
   - R² is selected from the group consisting of C₂₋₆ alkyl, C₂₋₆ alkenyl, C₂₋₆ alkynyl, C₃₋₅ cycloalkyl, C₅ cycloalkenyl, -C(=O)-C₁₋₅ alkyl, -C(=O)-C₂₋₅ alkenyl, - C(=O)-C₂₋₅ alkynyl, -C(=O)-C₃₋₅ cycloalkyl and -C(=O)-C₅ cycloalkenyl, each of which may be optionally substituted with one or more, identical or different, substituents R⁶;
   - R⁴ is selected from the group consisting of C₁₋₅ alkyl, C₂₋₅ alkenyl, C₂₋₅ alkynyl, C₃₋₅ cycloalkyl, C₅ cycloalkenyl, each of which may be optionally substituted with one or more, identical or different, substituents R⁷;
R⁶ is independently selected from the group consisting of hydrogen, deuterium, tritium, F, Cl, Br, I, -CN, isocyanide, -O-C₁₋₃ alkyl, -S-C₁₋₃ alkyl, -CH₂-OH, -CH₂-O-C₁₋₃ alkyl, -O-CH₂-Ph, -CH₂-SH and -CH₂-S-C₁₋₃ alkyl; and
R⁷ is independently selected from the group consisting of deuterium, tritium, F, Cl, Br, I, -CN, isocyanide, -O-C₁₋₃ alkyl, -S-C₁₋₃ alkyl, -CH₂-O-C₁₋₃ alkyl and -CH₂-S-C₁₋₃ alkyl,
or a pharmaceutically acceptable salt, hydrate, polymorph, tautomer, or solvate thereof, for use in treating, ameliorating and/or preventing a neuromuscular disorder, and/or for use in reversing and/or ameliorating a neuromuscular blockade.

In one embodiment, the invention is related to a compound of Formula (VIII): 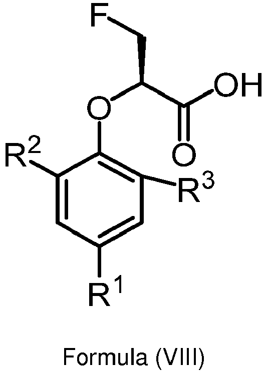 wherein:
R¹ is selected from the group consisting of F, Cl, Br, I, -CN, -CF3, -CCl₃, - CHF₂, -CHCl₂, -CH₂F, -CH₂Cl, -OCF₃ and -OCCl₃;
   - R² is selected from the group consisting of C₂₋₆ alkyl, C₂₋₆ alkenyl, C₂₋₆ alkynyl, C₃₋₅ cycloalkyl, C₅ cycloalkenyl, -C(=O)-C₁₋₅ alkyl, -C(=O)-C₂₋₅ alkenyl, - C(=O)-C₂₋₅ alkynyl, -C(=O)-C₃₋₅ cycloalkyl and -C(=O)-C₅ cycloalkenyl, each of which may be optionally substituted with one or more, identical or different, substituents R⁶;
R³ is selected from the group consisting of hydrogen, deuterium, tritium, F, Cl, Br, I, -CN, -CF₃, -CCl₃, -CHF₂, -CHCl₂, -CH₂F, -CH₂Cl, -OCF₃, -OCCl₃ and isocyanide; and
R⁶ is independently selected from the group consisting of hydrogen, deuterium, tritium, F, Cl, Br, I, -CN, isocyanide, -O-C₁₋₃ alkyl, -S-C₁₋₃ alkyl, -CH₂-OH, -CH₂-O-C₁₋₃ alkyl, -O-CH₂-Ph, -CH₂-SH and -CH₂-S-C₁₋₃ alkyl,
or a pharmaceutically acceptable salt, hydrate, polymorph, tautomer, or solvate thereof, for use in treating, ameliorating and/or preventing a neuromuscular disorder, and/or for use in reversing and/or ameliorating a neuromuscular blockade.

In one aspect, the invention relates to a compound of Formula (I): 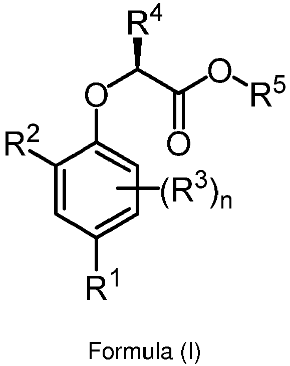 wherein:
- R¹ is selected from the group consisting of F, Cl, Br, I, -CN, -CF₃, -CCl₃, - CHF₂, -CHCl₂, -CH₂F, -CH₂Cl, -OCF₃ and -OCCl₃;
- R² is selected from the group consisting of C₂₋₆ alkyl, C₂₋₆ alkenyl, C₂₋₆ alkynyl, C₃₋₆ cycloalkyl, C₅₋₆ cycloalkenyl, -C(=O)-C₁₋₅ alkyl, -C(=O)-C₂₋₅ alkenyl, -C(=O)-C₂₋₅ alkynyl, -C(=O)-C₃₋₅ cycloalkyl and -C(=O)-C₅ cycloalkenyl, each of which may be optionally substituted with one or more, identical or different, substituents R⁶;
- R³ is selected from the group consisting of hydrogen, deuterium, tritium, F, Cl, Br, I, -CN, -CF₃, -CCl₃, -CHF₂, -CHCl₂, -CH₂F, -CH₂Cl, -OCF₃, -OCCl₃ and isocyanide;
- R⁴ is H;
- R⁵ is selected from the group consisting of H, C₁₋₅ alkyl optionally substituted with one or more, identical or different, substituents R¹¹, C₂₋₅ alkenyl, C₂₋₅ alkynyl, C₃₋₆ cycloalkyl optionally substituted with one or more, identical or different, substituents R¹¹, phenyl optionally substituted with one or more, identical or different, substituents R¹² and benzyl optionally substituted with one or more, identical or different, substituents R¹²;
- R⁶ is independently selected from the group consisting of hydrogen, deuterium, tritium, F, Cl, Br, I, -CN, isocyanide, -O-C₁₋₃ alkyl, -S-C₁₋₃ alkyl, -CH₂-OH, -CH₂-O-C₁₋₃ alkyl, -O-CH₂-Ph, -CH2-SH and -CH₂-S-C₁₋₃ alkyl;
- R¹¹ is independently selected from the group consisting of deuterium and F;
- R¹² is independently selected from the group consisting of deuterium, methoxy, nitro, cyano, Cl, Br, I and F; and
- n is an integer 0, 1, 2 or 3;
or a pharmaceutically acceptable salt, hydrate, polymorph, tautomer, or solvate thereof for use in treating, ameliorating and/or preventing a neuromuscular disorder, and/or for use in reversing and/or ameliorating a neuromuscular blockade.

In one embodiment, the compound for use in treating, ameliorating and/or preventing a neuromuscular disorder, and/or for use in reversing and/or ameliorating a neuromuscular blockade is selected from the group consisting of Compound A-1, Compound A-2, Compound A-3, Compound A-4, Compound A-5, Compound A-6, Compound A-7, Compound A-8, Compound A-9, Compound A-10, Compound A-11, Compound A-12, Compound A-13, Compound A-14, Compound A-15, Compound A-16, Compound A-17, Compound A-18, Compound A-19, Compound A-20, Compound A-21, Compound A-22, Compound A-23, Compound A-24, Compound A-25, Compound A-26, Compound A-27, Compound A-28, Compound A-29, Compound A-30, Compound A-31, Compound A-32, Compound A-33, Compound A-34, Compound A-35, Compound A-36, Compound A-37 and Compound A-38.

In a specific embodiment, the compound for use in treating, ameliorating and/or preventing a neuromuscular disorder, and/or for use in reversing and/or ameliorating a neuromuscular blockade is selected from the group consisting of:
(2*S*)-2-{4-bromo-2-[2-(methoxymethyl)cyclopropyl]phenoxy}propanoic acid;
(2*S*)-2-[4-bromo-2-(2,2-dichlorocyclopropyl)phenoxy]propanoic acid;
(2*S*)-2-{4-bromo-2-[(1s,3s)-3-methoxycyclobutyl]phenoxy}propanoic acid;
(2*S*)-2-{4-bromo-2-[(E)-2-bromoethenyl]phenoxy}propanoic acid;
(2*R*)-2-(4-bromo-2-cyclobutylphenoxy)-3-fluoropropanoic acid;
(2*S*)-2-{4-bromo-2-[(1*S*,2*S*)-2-(hydroxymethyl)cyclopropyl]phenoxy}propanoic acid;
(2*S*)-2-{4-bromo-2-[(1*R*,2*R*)-2-(hydroxymethyl)cyclopropyl]phenoxy}propanoic acid;
(2*S*)-2-(4-bromo-2-ethynylphenoxy)propanoic acid;
(2*S*)-2-{4-bromo-2-[(1*E*)-2-cyanoeth-1-en-1-yl]phenoxy}propanoic acid;
(2*S*)-2-(4-bromo-2-cyclopropylphenoxy)propanoic acid;
(2*S*)-2-(4-bromo-2-ethenylphenoxy)propanoic acid;
(2*S*)-2-(2-cyclopropyl-4-fluorophenoxy)propanoic acid;
(2*S*)-2-(2-cyclobutyl-4-fluorophenoxy)propanoic acid;
(2*S*)-2-(4-bromo-2-cyclobutylphenoxy)propanoic acid;
(2*S*)-2-(4-chloro-2-cyclobutylphenoxy)propanoic acid;
tert-butyl (2*S*)-2-(4-chloro-2-propanoylphenoxy)propanoate;
(2*S*)-2-{4-chloro-2-[(2,2-²H₂)propanoyl]phenoxy}propanoic acid;
(2*S*)-2-(4-bromo-2-propanoylphenoxy)-3-methylbutanoic acid;
methyl (2*S*)-2-[4-chloro-2-(cyclopent-1-en-1-yl)phenoxy]propanoate;
methyl (2*S*)-2-(4-bromo-2-propanoylphenoxy)-3-methylbutanoate;
(2*S*)-2-(4-chloro-2-ethynylphenoxy)propanoic acid;
(2*S*)-2-(4-chloro-2-propanoylphenoxy)propanoic acid;
sodium (2*S*)-2-(4-chloro-2-ethenylphenoxy)propanoate;
(2*S*)-2-(4-chloro-2-cyclopropylphenoxy)propanoic acid;
sodium (2*S*)-2-(4-chloro-2-propylphenoxy)propanoic acid;
sodium (2*S*)-2-(4-chloro-2-ethylphenoxy)propanoate;
methyl (2*S*)-2-(4-chloro-2-ethylphenoxy)propanoate;
(2*R*)-2-(4-chloro-2-cyclopropyl-6-fluorophenoxy)-3-fluoropropanoic acid;
(2*S*)-2-(2-cyclopropyl-4,6-difluorophenoxy)propanoic acid;
(2*S*)-2-(4-bromo-2-propanoylphenoxy)propanoic acid;
(2*S*)-2-(4-chloro-2-cyclopropyl-6-fluorophenoxy)propanoic acid;
(2*S*)-2-(2,4-difluoro-6-propanoylphenoxy)propanoic acid;
(2*S*)-2-(2-acetyl-4-chlorophenoxy)propanoic acid;
(2*S*)-2-(4-fluoro-2-propanoylphenoxy)propanoic acid;
(2*S*)-2-[4-bromo-2-(cyclopent-1-en-1-yl)phenoxy]propanoic acid;
(2*S*)-2-[4-bromo-2-(2,2-difluoroethenyl)phenoxy]propanoic acid;
(2*S*)-2-{2-[2-(benzyloxy)cyclobutyl]-4-chlorophenoxy}propanoic acid;
(2*S*)-2-[4-bromo-2-(cyclopent-1-en-1-yl)phenoxy]propanoic acid;
(2*S*)-2-[4-bromo-2-(2-methoxyethyl)phenoxy]propanoic acid;
(2*S*)-2-[2,4-dibromo-6-(2-methoxyethyl)phenoxy]propanoic acid;
(2*S*)-2-[4-bromo-2-(cyclopropylidenemethyl)phenoxy]propanoic acid;
(2*S*)-2-(4-bromo-2-ethenyl-5-fluorophenoxy)propanoic acid;
(2*S*)-2-(2-acetyl-4-bromo-5-fluorophenoxy)propanoic acid;
(2*S*)-2-(4-bromo-2-cyclopropyl-5-fluorophenoxy)propanoic acid;
(2*S*)-2-[4-bromo-2-(2,2-difluoroethenyl)-5-fluorophenoxy]propanoic acid;
(2*S*)-2-(4-bromo-2-ethynylphenoxy)-2-cyclobutylacetic acid;
(2*S*)-2-(4-bromo-2-ethynyl-5-fluorophenoxy)propanoic acid;
(2*S*)-2-(4-chloro-2-ethynyl-5-fluorophenoxy)propanoic acid;
(2*S*)-2-(4-bromo-2-cyclopropylphenoxy)-4-fluorobutanoic acid;
(2*S*)-2-(4-bromo-2-ethynylphenoxy)-4-fluorobutanoic acid;
(2*S*)-2-(4-bromo-2-ethenylphenoxy)-4-fluorobutanoic acid;
(2*S*)-2-{4-bromo-2-[(1*E*)-2-fluoroethenyl]phenoxy}propanoic acid;
(2*S*)-2-{4-chloro-2-[(1*E*)-2-fluoroethenyl]phenoxylpropanoic acid;
(2*S*)-2-(4-bromo-2-ethynylphenoxy)butanoic acid;
2-(4-bromo-2-cyclopropylphenoxy)acetic acid;
2-(4-bromo-2-ethynylphenoxy)acetic acid; and
(2*S*)-2-[4-bromo-2-(2,2-difluorocyclobutyl)phenoxy]propanoic acid.

### Neuromuscular disorders

The compound or compound for use of the present invention is used for treating, ameliorating and/or preventing a neuromuscular disorder, or reversing neuromuscular blockade caused by non-depolarizing neuromuscular blocker or antibiotic agent.

The inventors of the present invention have shown that inhibition of CIC-1 channels strengthens neuromuscular transmission. CIC-1 function may therefore contribute to muscle weakness in conditions of compromised neuromuscular transmission.

Thus, in one embodiment of the present invention, the compound or the compound for use as described herein inhibits CIC-1 channels. Thus, it is appreciated that compounds and/or compounds for use of Formula (I) inhibit CIC-1 channels.

The neuromuscular disorder may also include neuromuscular dysfunctions.

Neuromuscular disorders include for example disorders with symptoms of muscle weakness and fatigue. Such disorders may include conditions with reduced neuromuscular transmission safety factor. In one embodiment the neuromuscular disorders are motor neuron disorders. Motor neuron disorders are disorders with reduced safety in the neuromuscular transmission. In one embodiment motor neuron disorders are selected from the group consisting of amyotrophic lateral sclerosis (ALS) (Killian JM, Wilfong AA, Burnett L, Appel SH, Boland D. Decremental motor responses to repetitive nerve stimulation in ALS. Muscle Nerve, 1994, 17, 747-754), spinal muscular atrophy (SMA) (Wadman RI, Vrancken AF, van den Berg LH, van der Pol WL. Dysfunction of the neuromuscular junction in spinal muscular atrophy types 2 and 3. Neurology, 2012, 79, 2050-2055), Charcot-Marie Tooth disease (Bansagi B, Griffin H, Whittaker RG, Antoniadi T, Evangelista T, Miller J, Greenslade M, Forester N, Duff J, Bradshaw A, Kleinle S, Boczonadi V, Steele H, Ramesh V, Franko E, Pyle A, Lochmüller H, Chinnery PF, Horvath R. Genetic heterogeneity of motor neuropathies. Neurology, 2017, 28;88(13):1226-1234), X-linked spinal and bulbar muscular atrophy (Yamada, M., Inaba, A., Shiojiri, T. X-linked spinal and bulbar muscular atrophy with myasthenic symptoms. Journal of the Neurological Sciences, 1997, 146, 183-185), Kennedy's disorder (Stevic, Z., Peric, S., Pavlovic, S., Basta, I., Lavrnic, D., Myasthenic symptoms in a patient with Kennedy's disorder. Acta Neurologica Belgica, 2014, 114, 71-73), multifocal motor neuropathy (Roberts, M., Willison, H.J., Vincent, A., Newsom-Davis, J. Multifocal motor neuropathy human sera block distal motor nerve conduction in mice. Ann Neurol. 1995, 38, 111-118), Guillain-Barré syndrome (Ansar, V., Valadi, N. Guillain-Barré Syndrome Prim. Care, 2015, 42, 189-193; poliomyelitis (Trojan, D.A., Gendron, D., Cashman, N.R. Electrophysiology and electrodiagnosis of the post-polio motor unit. Orthopedics, 1991, 14, 1353-1361, and Birk T.J. Poliomyelitis and the post-polio syndrome: exercise capacities and adaptation - current research, future directions, and widespread applicability. Med. Sci. Sports Exerc., 1993, 25, 466-472), post-polio syndrome (Garcia, C.C., Potian, J.G., Hognason, K., Thyagarajan, B., Sultatos, L.G., Souayah, N., Routh, V.H., McArdle, J.J. Acetylcholinesterase deficiency contributes to neuromuscular junction dysfunction in type 1 diabetic neuropathy. Am. J. Physiol. Endocrinol. Metab., 2012, 15, E551 - 561) and sarcopenia (Gilmore K.J., Morat T., Doherty T.J., Rice C.L., Motor unit number estimation and neuromuscular fidelity in 3 stages of sarcopenia. 2017 55(5):676-684).

Thus, in one preferred embodiment of the present invention the neuromuscular disorder is amyotrophic lateral sclerosis (ALS). In another preferred embodiment the neuromuscular disorder is spinal muscular atrophy (SMA). In another preferred embodiment the neuromuscular disorder is Charcot-Marie tooth disease (CMT). In another preferred embodiment the neuromuscular disorder is sarcopenia. In yet another preferred embodiment, the neuromuscular disorder is critical illness myopathy (CIM).

As stated above the neuromuscular disorders include for example disorders with symptoms of muscle weakness and fatigue. Such disorder may for example include diabetes (Burton, A. Take your pyridostigmine: that's an (ethical?) order! Lancet Neurol., 2003, 2, 268).

In one embodiment the compound or the compound for use of the present invention is used to prevent neuromuscular disorder. The compound or the compound for use may for example be used prophylactically against nerve gas that is known to cause symptoms of muscle weakness and fatigue (Kawamura, Y., Kihara, M., Nishimoto, K., Taki, M. Efficacy of a half dose of oral pyridostigmine in the treatment of chronic fatigue syndrome: three case reports. Pathophysiology, 2003, 9, 189-194.

In another embodiment the neuromuscular disorders is chronic fatigue syndrome. Chronic fatigue syndrome (CFS) (Fletcher, S.N., Kennedy, D.D., Ghosh, I.R., Misra, V.P., Kiff, K., Coakley, J.H., Hinds, C.J. Persistent neuromuscular and neurophysiologic abnormalities in long-term survivors of prolonged critical illness. Crit. Care Med. 2003, 31, 1012 - 1016) is the common name for a medical condition characterized by debilitating symptoms, including fatigue that lasts for a minimum of six months in adults. CFS may also be referred to as systemic exertion intolerance disorder (SEID), myalgic encephalomyelitis (ME), post-viral fatigue syndrome (PVFS), chronic fatigue immune dysfunction syndrome (CFIDS), or by several other terms. Symptoms of CFS include malaise after exertion; unrefreshing sleep, widespread muscle and joint pain, physical exhaustion, and muscle weakness.

In a further embodiment the neuromuscular disorder is a critical illness polyneuropathy (Angelini C. Spectrum of metabolic myopathies. Biochim. Biophys. Acta., 2015, 1852, 615 - 621) or CIM (Latronico, N., Bolton, C.F. Critical illness polyneuropathy and myopathy: a major cause of muscle weakness and paralysis. Lancet Neurol. 2011, 10, 931-941). Critical illness polyneuropathy and CIM are overlapping syndromes of widespread muscle weakness and neurological dysfunction developing in critically ill patients.

The neuromuscular disorder may also include metabolic myopathy (Milone, M., Wong, L.J. Diagnosis of mitochondrial myopathies. Mol. Genet. Metab., 2013, 110, 35 - 41) and mitochondrial myopathy (Srivastava, A., Hunter, J.M. Reversal of neuromuscular block. Br. J. Anaesth. 2009, 103, 115 - 129). Metabolic myopathies result from defects in biochemical metabolism that primarily affects muscle. These may include glycogen storage disorders, lipid storage disorder and 3-phosphocreatine stores disorder. Mitochondrial myopathy is a type of myopathy associated with mitochondrial disorder. Symptoms of mitochondrial myopathies include muscular and neurological problems such as muscle weakness, exercise intolerance, hearing loss and trouble with balance and coordination.

In another embodiment the neuromuscular disorder is periodic paralysis, in particular hypokalemic periodic paralysis which is a disorder of skeletal muscle excitability that presents with recurrent episodes of weakness, often triggered by exercise, stress, or carbohydrate-rich meals (Wu, F., Mi, W., Cannon, S.C., Neurology, 2013, 80, 1110-1116 and Suetterlin, K. et at, Current Opinion Neurology, 2014, 27, 583-590) or hyperkalemic periodic paralysis which is an inherited autosomal dominant disorder that affects sodium channels in muscle cells and the ability to regulate potassium levels in the blood (Ammat, T. et at, Journal of General Physiology, 2015, 146, 509-525).

In a preferred embodiment the neuromuscular disorder is a myasthenic condition. Myasthenic conditions are characterized by muscle weakness and neuromuscular transmission failure. Congenital myasthenia gravis (Finlayson, S., Beeson, D., Palace, J. Congenital myasthenic syndromes: an update. Pract. Neurol., 2013, 13, 80 - 91) is an inherited neuromuscular disorder caused by defects of several types at the neuromuscular junction.

Myasthenia gravis and Lambert-Eaton syndrome (Titulaer MJ, Lang B, Verschuuren JJ. Lambert-Eaton myasthenic syndrome: from clinical characteristics to therapeutic strategies. Lancet Neurol. 2011, 10, 1098-107) are examples of myasthenic conditions. Myasthenia gravis is either an autoimmune or congenital neuromuscular disorder that leads to fluctuating muscle weakness and fatigue. In the most common cases, muscle weakness is caused by circulating antibodies that block ACh receptors at the postsynaptic neuromuscular junction, inhibiting the excitatory effects of the neurotransmitter ACh on nicotinic ACh-receptors at neuromuscular junctions (Gilhus, N.E., Owe, J.F., Hoff, J.M., Romi, F., Skeie, G.O., Aarli, J.A. Myasthenia Gravis: A Review of Available Treatment Approaches, Autoimmune Diseases, 2011, Article ID 84739). Lambert-Eaton myasthenic syndrome (also known as LEMS, Lambert-Eaton syndrome, or Eaton-Lambert syndrome) is a rare autoimmune disorder that is characterized by muscle weakness of the limbs. It is the result of an autoimmune reaction in which antibodies are formed against presynaptic voltage-gated calcium channels, and likely other nerve terminal proteins, in the neuromuscular junction.

Thus, in one embodiment of the present invention the neuromuscular disorder is myasthenia gravis. In another preferred embodiment the neuromuscular disorder is Lambert-Eaton syndrome.

Neuromuscular blockade is used in connection with surgery under general anesthesia. Reversing agents are used for more rapid and safer recovery of muscle function after such blockade. Complications with excessive muscle weakness after blockade during surgery can result in delayed weaning from mechanical ventilation and respiratory complications after the surgery. Since such complications have pronounced effects on outcome of the surgery and future quality of life of patients, there is a need for improved reversing agents (Murphy GS, Brull SJ. Residual neuromuscular block: lessons unlearned. Part I: definitions, incidence, and adverse physiologic effects of residual neuromuscular block. Anesth Analg. 2010 111(1):120-8). Thus, in one embodiment, the neuromuscular disorder has been induced by a neuromuscular blocking agent. In one particular embodiment the neuromuscular disorder is muscle weakness caused by neuromuscular blockade after surgery. In another preferred embodiment of the present invention the compound or the compound for use is used for reversing and/or ameliorating neuromuscular blockade after surgery. In one embodiment, the neuromuscular blockade is drug induced. In one embodiment the neuromuscular blockade is induced by an antibiotic. In one embodiment the neuromuscular blockade is induced by a non-depolarizing neuromuscular blocker.

### Pharmaceutical formulations

In one embodiment, a composition comprising the compound or the compound for use, according to the present invention, is provided. The composition according to the present invention is used for treating, ameliorating and/or preventing a neuromuscular disorder, and/or for use in reversing and/or ameliorating a neuromuscular blockade. Thus, it is preferred that the compositions and compounds described herein are pharmaceutically acceptable. In one embodiment the composition as described herein is in the form of a pharmaceutical formulation. In one embodiment, the composition as described herein further comprises a pharmaceutically acceptable carrier.

### Combination therapy

The composition of the present invention may comprise further active ingredients/agents or other components to increase the efficiency of the composition. Thus, in one embodiment the composition further comprises at least one further active agent. It is appreciated that the active agent is suitable for treating, preventing or ameliorating said neuromuscular disorder.

The active agent is in a preferred embodiment an acetylcholine esterase inhibitor. Said acetylcholine esterase inhibitor may for example be selected from the group consisting of delta-9-tetrahydrocannabinol, carbamates, physostigmine, neostigmine, pyridostigmine, ambenonium, demecarium, rivastigmine, phenanthrene derivatives, galantamine, piperidines, donepezil, tacrine, edrophonium, huperzine, ladostigil, ungeremine and lactucopicrin.

Preferably the acetylcholine esterase inhibitor is selected from the group consisting of neostigmine, physostigmine and pyridostigmine. It is preferred that the acetylcholine esterase inhibitor is neostigmine or pyridostigmine.

The active agent may also be an immunosuppressive drug. Immunosuppressive drugs are drugs that suppress or reduce the strength of the body's immune system. They are also known as anti-rejection drugs. Immunosuppressive drugs include but are not limited to glucocorticoids, corticosteroids, cytostatics, antibodies and drugs acting on immunophilins. In one embodiment the active agent is prednisone.

The active agent may also be an agent that is used in anti-myotonic treatment. Such agents include for example blockers of voltage gated Na⁺ channels, and aminoglycosides.

The active agent may also be an agent for reversing a neuromuscular blockade after surgery. Such agents include for example neostigmine or sugammadex (Org 25969, tradename Bridion). The active agent may also be an agent for increasing the Ca²⁺ sensitivity of the contractile filaments in muscle. Such agents include tirasemtiv and CK-2127107 (Hwee, D.T., Kennedy, A.R., Hartman, J.J., Ryans, J., Durham, N., Malik, F.I., Jasper, J.R. The small-molecule fast skeletal troponin activator, CK-2127107, improves exercise tolerance in a rat model of heart failure. Journal of Pharmacology and Experimental Therapeutics, 2015, 353, 159 - 168).

The active agent may also be an agent for increasing ACh release by blocking voltage-gated K⁺ channels in the pre-synaptic terminal. Such agent includes 3,4-aminopyridine.

### Methods

The compounds are used in a method of treating, preventing and/or ameliorating a neuromuscular disorder, said method comprising administering a therapeutically effective amount of the compound or the compound for use as defined herein to a person in need thereof.

The compounds are also used in a method of reversing and/or ameliorating a neuromuscular blockade, said method comprising administering a therapeutically effective amount of the compound or the compound for use as defined herein to a person in need thereof.

The compounds are also used in a method for recovery of neuromuscular transmission, said method comprising administering a therapeutically effective amount of the compound or the compound for use as defined herein to a person in need thereof.

The person in need thereof may be a person having a neuromuscular disorder or a person at risk of developing a neuromuscular disorder or a person having symptoms of muscle weakness and/or fatigue. In another embodiment the person in need thereof is a person with reduced neuromuscular transmission safety with prolonged recovery after neuromuscular blockade. Types of neuromuscular disorders are defined herein above. In a preferred embodiment the person has, amyotrophic lateral sclerosis, spinal muscular atrophy, myasthenia gravis or Lambert-Eaton syndrome.

A therapeutically effective amount is an amount that produces a therapeutic response or desired effect in the person taking it. Administration routes, formulations and dosages can be optimized by persons of skill in the art.

The compounds may be combined with other compounds that are known to treat, prevent and/or ameliorate neuromuscular disorders. They may for example be combined with any of the agents mentioned herein above. In one embodiment they are combined with an acetylcholine esterase inhibitor such as for example neostigmine or pyridostigmine.

### Method of manufacturing

The methods of manufacturing are not part of the invention. In one aspect, the present invention relates to methods of manufacturing compounds or compounds for use according to formula (I).

One method for manufacturing the compounds or compounds for use according to the present invention comprises the steps of
a. reacting a compound having formula (IX) 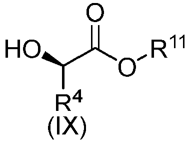 wherein R⁴ is as defined herein and R¹¹ is a protecting group, such as selected from the group consisting of alkyl, alkenyl, akynyl, cycloalkyl, cycloalkenyl, aromatic ring, heteroaromatic ring and -alkylene-Si-alkyl, with first a reagent capable of converting the alcohol (OH) into a leaving group and secondly with a compound having formula (X) 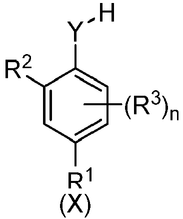 wherein R¹, R², R³ and n are as defined herein and Y is O to generate a compound having formula (XI) 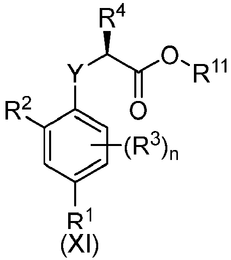 ; and
b. reacting the product compound of a) with an ester hydrolysing reagent thus generating a compound as defined herein.

A second method for manufacturing the compounds or compounds for use according to the present invention comprises the steps of
a. reacting a compound having formula (XII) 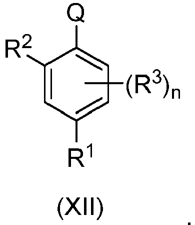 wherein R¹, R², R³ and n are as defined herein and Q is a leaving group, such as selected from the group consisting of fluorine and iodine, with a compound having formula (IX) 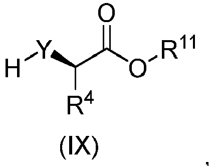 wherein R⁴ is as defined herein, and R¹¹ a protecting group, such as selected from the group consisting of alkyl, alkenyl, akynyl, cycloalkyl, cycloalkenyl, aromatic ring, heteroaromatic ring and -alkylene-Si-alkyl and Y is O to generate a compound having formula (XI) 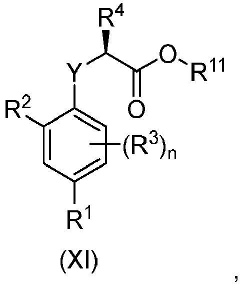 wherein Y is O; and
b. reacting the product compound of a) with an ester hydrolysing reagent thus generating a compound as defined herein.

Yet a third method for manufacturing the compounds or compounds for use according to the present invention comprises the steps of
a. reacting a compound having formula (XIII) 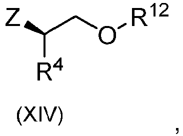 wherein R⁴ is as defined herein, Z is OH and R¹² is a protecting group, such as an -Si-alkyl, with first a reagent capable of converting the alcohol (Z) into a leaving group and secondly with a compound having formula (X) 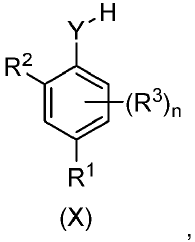 wherein R¹, R², R³ and n are as defined herein, and Y is O to generate a compound having formula (XIV) 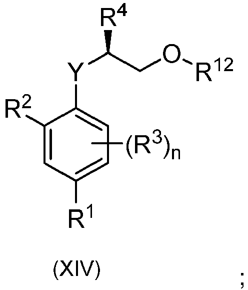
b. reacting the product compound of a) with an ether cleaving reagent to generate a compound having formula (XV) 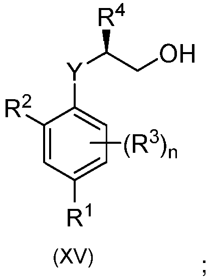 and
c. reacting the product compound of b) with an oxidising agent thus generating a compound as defined herein.

### Examples

### Materials and methods

### Chemicals

Compounds for testing were obtained from different suppliers including Enamine, Vitas, and CanAm Bioresearch. For synthesis of particular compounds please see below.

### General synthetic strategies

Compounds of formula (I) may be synthesized by the following synthetic strategies, general methods A-C:

### NMR Spectra

¹H-NMR spectra were recorded on a Bruker AM-300 spectrometer and were calibrated using residual nondeuterated solvent as internal reference. Spectra were processed using Spinworks version 4.0 (developed by Dr. Kirk Marat, Department of Chemistry, University of Manitoba).

### HPLC method 1

The product was analysed by Waters 2695 HPLC consisting of a Waters 996 photodiode array detector, Kromasil Eternity C18, 5 µm, 4.6 × 150 mm column. Flow rate: 1 mL/minute, run time 20 minutes. Solvent A: methanol; solvent B: 0.1% formic acid in water. Gradient 0-100 % Solvent B over 15 minutes with monitoring at 280 nm.

### HPLC method 2

Waters Acquity UPLC, X-Select; column: Waters X-Select UPLC C18, 1.7 µm, 2.1 × 30mm. Solvent A: 0.1% formic acid in water; solvent B: 0.1% formic acid in MeCN. Gradient 5-95 % Solvent B over 10 minutes; detector: diode array.

### HPLC method 3

Waters Acquity UPLC, X-Select; column: Waters X-Select UPLC C18, 1.7 µm, 2.1 × 30mm. Solvent A: 0.1% formic acid in water; solvent B: 0.1% formic acid in MeCN. Gradient 5-95 % Solvent B over 3 minutes; detector: diode array.

### HPLC method 4

Waters Acquity UPLC, X-Select; column: Waters X-Select UPLC C18, 1.7 µm, 2.1 × 30mm. Solvent A: 0.1% formic acid in water; solvent B: 0.1% formic acid in MeCN. Gradient 5-95 % Solvent B over 4 minutes; detector: diode array.

### General Method A

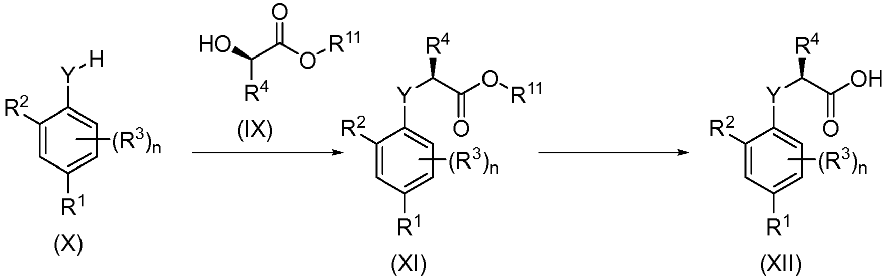

Method A involves the synthesis of compounds of Formula (XII) (which is the same as Formula (I) in which R⁵ is H), which is an ether structure wherein Y = oxygen, and -R₁, - R₂, -R₃ and -R₄, are as defined in Formula (I) above. Compound (X), in the case where Y = O is a phenol, is available either commercially or synthetically (see below), and can be converted into an ether (XI) by methods which include Mitsunobu reaction conditions. This ether contains an ester functionality -CO₂R¹¹, which can be hydrolysed under a range of standard conditions, involving treatment with acid or base, to provide the carboxylic acid structure (XII), Y = O. Standard conditions for hydrolysis of the ester can also for example involve an enzymatic hydrolysis, employing for example an esterase or lipase. Furthermore, if an ester molecule (XI) comprises for example a (CH₃)₃SiCH₂CH₂O- group as -OR¹¹, then a fluoride ion source such as tetra-n-butylammonium fluoride can be employed to convert (XI) into the corresponding carboxylic acid (XII).

Substituted phenols of general formula (X), Y = O, can be prepared by a variety of standard methods, for example by an ester rearrangement in the Fries rearrangement, by a rearrangement of *N*-phenylhydroxylamines in the Bamberger rearrangement, by hydrolysis of phenolic esters or ethers, by reduction of quinones, by replacement of an aromatic amine or by a hydroxyl group with water and sodium bisulfide in the Bucherer reaction. Other methods include hydrolysis of diazonium salts, by rearrangement reaction of dienones in the dienone phenol rearrangement, by the oxidation of aryl silanes or by the Hock process.

### General Method B

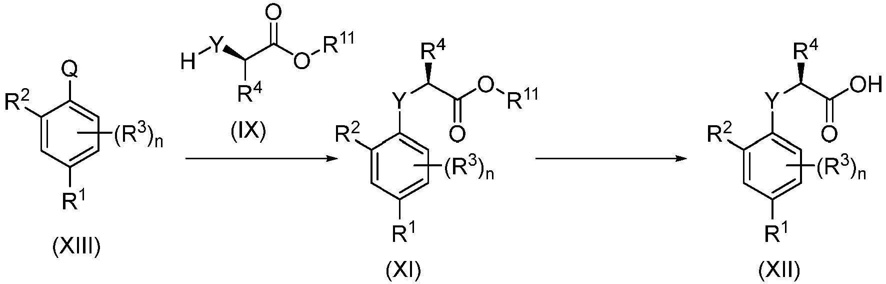

Carboxylic acids of Formula (XII) (which is the same as Formula (I) in which R⁵ is H) can also be prepared by the procedure illustrated as General Method B. A phenolic ether of formula (XI) can be prepared by displacement of a suitable leaving group Q in (XIII) with the nucleophilic YH in (IX) (wherein Y=O). Q can for example be a halogen such as fluorine or iodine, and the ether product of formula (XI) can be converted into the carboxylic acid derivative (XII) by one of a range of methods outlined in General Method A, involving hydrolysis of the ester functionality.

### General Method C

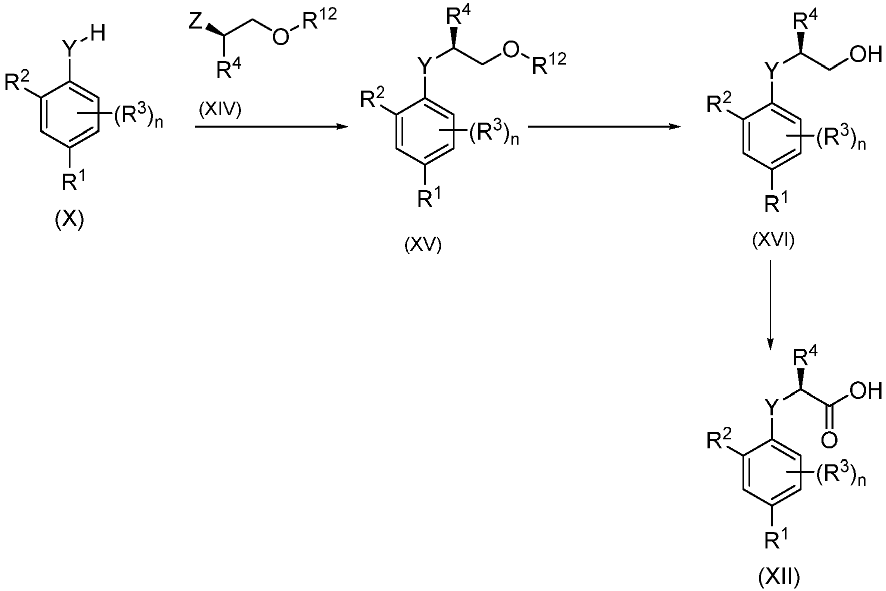

Carboxylic acids of Formula (XII) (which is the same as Formula (I) in which R⁵ is H) can be prepared by the procedure illustrated as General Method C. A phenolic ether of formula (XV) can be prepared by utilising e.g. Mitsunobu conditions when (X) is a phenol structure, i.e. Y = O, and (XIV) is a suitable secondary alcohol, i.e. Z = OH, and -R¹² is a suitable protecting group, such as a silyl-containing moiety. On removal of the protecting group -R¹², the primary alcohol in (XVI) can be oxidised to a carboxylic acid under standard conditions involving potassium permanganate, Jones oxidation conditions, the Heyns oxidation, ruthenium tetroxide or TEMPO, generating (XII).

Table 1 below illustrates Example compounds defined by the general Formula (I). In table 1, the HPLC System is one of the methods as defined in the Materials and methods section.

**Table 1: Illustrative Examples of the Invention**

| **Cpd Number** | **IUPAC name** | **¹H NMR** | **HPLC retention time (Method)** | **Synthesis method** |
|---|---|---|---|---|
| A-1 | (2*S*)-2-{4-bromo-2-[2-(methoxymethyl)cyclopr | (500 MHz, CDCl3) δ 7.30 (dt, *J* = 2.6, 0.8 Hz, 0.5H), | 3.910 and 4.027 | A |
| | opyl]phenoxy}propanoic acid (1:1 mixture of diastereoisomers) | 7.29 - 7.27 (m, 0.5H), 7.21 (dd, *J* = 2.5, 0.9 Hz, 0.5H), 7.13 (dd, *J* = 2.5, 0.8 Hz, 0.5H), 6.74 (dd, *J* = 8.7, 0.6 Hz, 0.5H), 6.69 (d, *J* = 8.7 Hz, 0.5H), 5.02 - 4.96 (m, 0.5H), 4.94 - 4.87 (m, 0.5H), 3.87 (dd, *J* = 9.7, 4.5 Hz, 0.5H), 3.81 (dd, *J* = 10.0, 5.0 Hz, 0.5H), 3.44 (s, 1.5H), 3.43 (s, 1.5H), 3.07 (t, *J* = 9.7 Hz, 0.5H), 3.00 (t, *J* = 9.7 Hz, 0.5H), 2.00 (dt, *J* = 8.5, 5.3 Hz, 0.5H), 1.92 (dt, *J* = 9.0, 5.2 Hz, 0.5H), 1.65 (app dd, *J* = 6.7, 6.2 Hz, 3H), 1.26 - 1.15 (m, 1H), 1.13 - 1.02 (m, 1H), 0.83 (ddt, *J* = 8.4, 6.6, 5.2 Hz, 1H). | (2) | |
| A-2 | (2*S*)-2-(4-bromo-2-cyclopropylphenoxy)pro panoic acid | (400 MHz, DMSO-d6) δ 13.09 (s, 1H), 7.23 (dd, *J* = 8.7, 2.5 Hz, 1H), 6.94 (d, *J* = 2.5 Hz, 1H), 6.74 (d, *J* = 8.8 Hz, 1H), 4.83 (q, *J* = 6.7 Hz, 1H), 2.17 (tt, *J* = 8.5, 5.3 Hz, 1H); 1.53 (d, *J* = 6.8 Hz, 3H); 0.93 (dq, *J* = 8.5, 2.2 Hz, 2H); 0.74 (ddt, *J* = 9.4, 4.1, 2.1 Hz, 1H); 0.69-0.58 (m, 1H). | 4.298 (2) | A |
| A-3 | (2*S*)-2-(2-cyclopropyl-4-fluorophenoxy)propanoi c acid | (400 MHz,DMSO-d6) δ 13.06 (s, 1H); 6.87 (td, *J* = 8.6, 3.1 Hz, 1H); 6.78 (dd, *J* = 9.0, 4.8 Hz, 1H); 6.64 (dd, *J* = 10.0, 3.1 Hz, 1H); 4.77 (q, *J* = 6.7 Hz, 1H); 2.27-2.14 (m, 1H); 1.52 (d, *J* = 6.7 Hz, 3H); 0.99-0.87 (m, 2H); 0.81-0.69 (m, 1H); 0.68-0.59 (m, 1H). | 1.366 (3) | A |
| A-4 | (2*S*)-2-(4-chloro-2-cyclopropylphenoxy)pro panoic acid | (400 MHz, DMSO-d⁶) δ 6.98 (dd, J = 8.8, 2.7 Hz, 1H), 6.74 - 6.63 (m, 2H), 4.20 (q, J = 6.7 Hz, 1H), 2.20 (tt, J = 8.6, 5.3 Hz, 1H), 1.38 (d, J = 6.6 Hz, 3H), 0.97 - 0.83 (m, 2H), 0.77 - 0.66 (m, 1H), 0.66 - 0.56 (m, 1H). | 4.181 (2) | A |
| A-5 | (2*S*)-2-{4-bromo-2-[(1*S*,2*S*)-2-(hydroxymethyl)cyclopro pyl]phenoxy}propanoic acid | (400 MHz, Chloroform-d) δ 7.39 (br.s, 2H), 7.20 (dd, J = 8.7, 2.5 Hz, 1H), 6.92 (d, J = 2.4 Hz, 1H), 6.71 (d, J = 8.7 Hz, 1H), 4.89 (q, J = 6.7 Hz, 1H), 4.10 (dd, J = 11.4, 3.0 Hz, 1H), 3.20 - 3.03 (m, 1H), 2.22 (dt, J = 8.5, 5.2 Hz, 1H), 1.65 (d, J = 6.7 Hz, 3H), 1.12 (d, J = 6.0 Hz, 2H), 0.83 (d, J = 8.6 Hz, 1H). | 1.238 (3) | A |
| A-6 | (2*S*)-2-{4-bromo-2-[(1*R*,2*R*)-2-(hydroxymethyl)cyclopro pyl]phenoxy}propanoic acid | (400 MHz, CDCl3) δ 7.31 - 7.22 (m, 1H), 7.17 (d, J = 2.4 Hz, 1H), 6.67 (d, J = 8.7 Hz, 1H), 6.29 (br.s, 2H), 4.90 (q, J = 6.7 Hz, 1H), 4.06 (dd, J = 11.2, 4.8 Hz, 1H), 3.24 (dd, J = 11.2, 9.4 Hz, 1H), 2.00 - 1.86 (m, 1H), 1.67 (d, J = 6.7 Hz, 3H), 1.25 - 1.15 (m, 1H), 1.14 - 1.07 (m, 1H), 0.92 - 0.73 (m, 1H). | 1.176 (3) | A |
| A-7 | (2*S*)-2-(4-chloro-2-cyclopropyl-6-fluorophenoxy)propanoi c acid | (400 MHz,DMSO-d6) δ 12.99 (s, 1H); 7.23 (dd, *J* = 11.3, 2.5 Hz, 1H); 6.70 (dd, *J* = 2.5, 1.6 Hz, 1H); 4.70 (qd, *J* = 6.8, 1.1 Hz, 1H); 2.30 (tt, *J* = 8.5, 5.2 Hz, 1H); 1.49 (dd, J= 6.8, 0.8 Hz, 3H); 1.09-0.89 (m, 2H); 0.83-0.73 (m, 1H); 0.71-0.61 (m, 1H). | 4.165 (2) | A |
| A-8 | (2*S*)-2-[4-bromo-2-(2,2-dichlorocyclopropyl)phe noxy] propanoic acid (1:1 mixture of diastereoisomers) | (500 MHz, Chloroform-*d*) δ 7.44 - 7.36 (m, 1H), 7.19 (dd, *J* = 2.4, 0.9 Hz, 0.5H), 7.15 (dd, *J* = 2.5, 0.9 Hz, 0.5H), 6.75 (d, *J* = 8.7 Hz, 0.5H), 6.72 (d, *J* = 8.7 Hz, 0.5H), 4.92 - 4.83 (m, 1H), 3.05 - 2.98 (m, 0.5H), 2.96 - 2.88 (m, 0.5H), 2.05 - 1.98 (m, 1H), 1.88 - 1.78 (m, 2.5H), 1.76 (d *J* = 6.8 Hz, 1.5H). | 4.787 (2) | A |
| A-9 | (2*S*)-2-(2-cyclopropyl-4,6-difluorophenoxy)propan oic acid | (400 MHz, DMSO-d6) δ 12.96 (s, 1H); 7.05 (ddd, *J* = 11.6, 8.7, 3.0 Hz, 1H); 6.53 (ddd, *J* = 10.0, 3.0, 1.8 Hz, 1H); 4.62 (qd, *J* = 6.8, 0.9 Hz, 1H); 2.34 (ttd, *J* = 8.4, 5.2, 1.5 Hz, 1H); 1.48 (dd, *J* = 6.7, 0.8 Hz, 3H); | 3.569 (2) | A |
| | | 1.08-0.90 (m, 2H); 0.81-0.72 (m, 1H); 0.71-0.62 (m, 1H). | | |
| A-10 | (2*R*)-2-(4-chloro-2-cyclopropyl-6-fluorophenoxy)-3-fluoropropanoic acid | (400 MHz, DMSO-d6) δ 13.36 (s, 1H), 7.25 (dd, J = 11.4, 2.5 Hz, 1H), 6.72 (dd, J = 2.5, 1.6 Hz, 1H), 5.06 - 4.93 (m, 1H), 4.93 - 4.68 (m, 2H), 2.33 (tt, J = 8.5, 5.3 Hz, 1H), 1.04 - 0.92 (m, 2H), 0.85 - 0.75 (m, 1H), 0.75 - 0.66 (m, 1H). | 4.068 (2) | C |
| A-11 | (2*R*)-2-(4-bromo-2-cyclobutylphenoxy)-3-fluoropropanoic acid | (400 MHz, DMSO-d6) δ 13.49 (s, 1H), 7.36 - 7.21 (m, 2H), 6.79 (d, J = 9.4 Hz, 1H), 5.12 (d, J = 28.1 Hz, 1H), 4.94 - 4.71 (m, 2H), 3.70 (p, J = 8.6 Hz, 1H), 2.26 (dtd, J = 9.4, 6.9, 6.1, 2.4 Hz, 2H), 2.19 - 1.89 (m, 3H), 1.82 - 1.70 (m, 1H). | 4.873 (2) | C |
| A-12 | (2*S*)-2-{4-bromo-2-[(1s,3s)-3-methoxycyclobutyl]phen oxy} propanoic acid (3:1 cis/trans mixture) | (500 MHz, CDCl3) δ 7.37 (dd, *J* = 2.5, 0.9 Hz, 0.25H)7.32 (dd, *J* = 2.5, 0.9 Hz, 0.75H), 7.28 - 7.24 (m, 1H), 6.60 (d, *J* = 8.7 Hz, 1H), 4.82 - 4.75 (m, 1H), 4.06 - 4.01 (m, 0.25H), 3.92 (tt, *J* = 7.8, 6.6 Hz, 0.75H), 3.88 - 3.82 (m, 0.25H), 3.31 (s, 0.75H), 3.30 (s, 2.25H), 3.28 - 3.19 (m, 0.75H), 2.77 - 2.69 (m, 1.5H), 2.50 - 2.34 (m, 1H), 2.09 - 1.94 (m, 1.50H), 1.67 (d, *J* = 6.8 Hz, 3H). | 2.24 (4) | A |
| A-13 | (2*S*)-2-(2-cyclobutyl-4-fluorophenoxy)propanoi c acid | (400 MHz, Chloroform-d) δ 6.95 (ddd, J = 9.5, 3.1, 0.8 Hz, 1H), 6.82 - 6.76 (m, 1H), 6.65 (dd, J = 8.9, 4.5 Hz, 1H), 4.71 (q, J = 6.8 Hz, 1H), 3.82 - 3.69 (m, 1H), 2.41 - 2.29 (m, 2H), 2.18 - 1.97 (m, 3H), 1.89 - 1.79 (m, 1H), 1.63 (d, J = 6.8 Hz, 3H). | 2.33 (4) | A |
| A-14 | (2*S*)-2-(4-bromo-2-cyclobutylphenoxy)prop anoic acid (sodium salt) | (400 MHz, DMSO-d6) δ 7.16 (d, J = 8.2 Hz, 2H), 6.63 (d, J = 8.4 Hz, 1H), 4.16 (q, J = 6.6 Hz, 1H), 3.66 (p, J = 8.8 Hz, 1H), | 5.007 (2) | A |
| | | 2.31 - 2.09 (m, 3H), 2.04 - 1.87 (m, 2H), 1.82 - 1.70 (m, 1H), 1.35 (d, J = 6.6 Hz, 3H). | | |
| A-15 | (2*S*)-2-(4-chloro-2-cyclobutyl phen oxy)prop anoic acid | (400 MHz, DMSO-d6) δ 13.00 (s, 1H), 7.20 - 7.13 (m, 2H), 6.79 - 6.73 (m, 1H), 4.81 (q, J = 6.7 Hz, 1H), 3.67 (p, J = 8.7 Hz, 1H), 2.27 (dddq, J = 9.3, 7.9, 4.8, 1.5 Hz, 2H), 2.20 - 1.88 (m, 3H), 1.84 - 1.72 (m, 1H), 1.50 (d, J = 6.7 Hz, 3H). | 4.873 (2) | A |
| A-16 | (2*S*)-2-{4-bromo-2-[(E)-2-bromoethenyl]phenoxy} propanoic acid | (300 MHz, CDCl3) δ 10.88-10.20 (br, 1H); 7.43 (s, 1H); 7.37-7.23 (m, 2H); 6.96 (d, 1H); 6.64 (d, 1H); 4.81 (q, 1H); 1.72 (d, 3H). | 15.19 (1) | A |
| A-17 | (2*S*)-2-(4-bromo-2-ethenylphenoxy)propan oic acid | (300MHz, CDCl3): δ 10.17-9.48 (br, 1H), 7.62 (s, 1H), 6.66 (d, 1H), 5.77 (d, 1H), 5.34 (d, 1H), 4.77 (q, 1H). | 14.54 (1) | A |
| A-18 | sodium (2*S*)-2-(4-chloro-2-ethenylphenoxy)propan oate | (300MHz, CD3OD): δ 7.34 (s, 1H), 7.10-6.95 (m, 2H), 5.15 (d, 1H), 4.35 (q, 1H), 1.47 (d, 3H). | 13.67 (1) | A |
| A-19 | (2*S*)-2-(4-bromo-2-ethynylphenoxy)propano ic acid | (300MHz, CDCl3): δ 9.13-8.37 (br, 1H), 7.61 (s, 1H), 4.84 (q, 1H), 3.36 (s, 1H), 1.72 (d, 3H). | 13.52 (1) | A |
| A-20 | (2*S*)-2-(4-chloro-2-ethynylphenoxy)propano ic acid | (300 MHz, CDCl3) δ 9.80 (br s, 1H), 7.46 (d, 1H), 4.83 (q, 1H), 3.35 (s, 1H), 1.72 (d, 3H). | 14.23 (1) | A |
| A-21 | (2*S*)-2-{4-bromo-2-[(1E)-2-cyanoeth-1-en-1-yl]phenoxy}propanoic acid | (300 MHz, CDCl3) δ 6.70 (d, 1H), 6.08 (d, 1H), 4.87 (q, 1H), 1.74 (d, 3H); | 13.25 (1) | A |
| A-22 | sodium (2*S*)-2-(4-chloro-2-ethylphenoxy)propanoat e | (300MHz, CD3OD): δ 7.07 (s, 1H), 6.72 (d, 1H), 4.41 (q, 1H), 2.68-2.54 (m, 1H), 1.60- 1.55 (d, 3H), 1.20 (t, 3H). | 14.39 (1) | A |
| A-23 | sodium (2*S*)-2-(4-chloro-2-propylphenoxy)propanoi c acid | (400 MHz, CD3OD) δ 7.06 - 7.98 (m, 2H), 4.39 (q, 1H), 2.77 (m, 1H), 2.50 (m, 1H), 1.68 - 1.58 (m, 2H), 1.54 (d, 3H), 0.95 (m, 3H). | 14.98 (1) | A |
| A-24 | methyl (2*S*)-2-(4-chloro-2-ethylphenoxy)propanoat e | (300MHz, CDCl3): δ 7.15 (s, 1H), 6.60 (d, 1H), 4.74 (q, 1H), 3.75 (s, 3H),1.64 (d, 3H), 1.22 (t, 3H). | 11.48 (1) | A |
| A-25 | methyl (2*S*)-2-[4-chloro-2-(cyclopent-1-en-1-yl)phenoxy]propanoate | (300 MHz, CDCl3) δ 7.60 (t, 1H), 6.54 (m, 1H), 4.79 (q, 1H), 3.77 (s, 3H), 2.02 - 1.90 (m, 2H), 1.66 (d, 3H). | 16.72 (1) | A |
| A-26 | tert-butyl (2*S*)-2-(4-chloro-2-propanoylphenoxy)prop anoate | (300 MHz, CDCl3) δ 7.67 (d, 1H), 4.73 (q, 1H), 1.64 (d, 3H), 1.43 (s, 9H), 1.19 (t, 3H). | 19.07 (1) | A |
| A-27 | (2*S*)-2-{4-chloro-2-[(2,2-²H₂)propanoyl]phenoxy} propanoic acid | (300 MHz, CDCl3) δ 8.40 (br s, 1H), 7.41 (dd, 1H), 4.89 (q, 1H), 1.72 (d, 3H), 1.18 (s, 3H). | 7.705 (1) | A |
| A-28 | (2*S*)-2-(4-bromo-2-propanoylphenoxy)-3-methylbutanoic acid | (300 MHz, CD3OD) δ 7.66 (d, 1H), 4.70 (m, 1H), 3.11 (q, 2H), 2.36 (m, 1H), 1.18 - 1.08 (m, 9H). | 15.09 (1) | A |
| A-29 | methyl (2*S*)-2-(4-bromo-2-propanoylphenoxy)-3-methylbutanoate | (300 MHz, CDCl3) δ 7.76 (d, 1H), 7.46 (dd, 1H), 6.66(d, 1H), 6.54 (m, 1H), 4.56 (d, 1H), 3.77 (s, 3H), 2.42 - 2.31 (m, 1H), 1.20 (t, 3H). | 15.72 (1) | A |
| A-30 | (2*S*)-2-(4-chloro-2-propanoylphenoxy)prop anoic acid | (300 MHz, CD3OD) δ 7.56 (d, 1H), 4.89 (q, 1H), 3.16-3.02 (m, 2H), 1.64 (d, 3H), 1.13 (t, 3H). | 13.1 (1) | A |
| A-31 | (2*S*)-2-(4-bromo-2-propanoylphenoxy)prop anoic acid | (300 MHz, CDCl3) δ 7.86 (d, 1H), 4.93 (q, 1H), 1.76 (d, 3H), 1.25 (t, 3H). | 16.07 (1) | A |
| A-32 | (2*S*)-2-(4-fluoro-2-propanoylphenoxy)prop anoic acid | (300 MHz, CDCl3) δ 7.43 (dd, 1H), 4.88 (q, 1H), 3.16 - 2.89 (m, 2H), 1.73 (d, 3H), 1.25 (t, 3H). | 12.88 (1) | A |
| A-33 | (2*S*)-2-(2,4-difluoro-6-propanoylphenoxy)prop anoic acid | (300 MHz, CDCl3) δ 8.03 (br s, 1H), 7.15 (m, 1H), 4.94 (q, 1H), 3.15 - 2.93 (m, 2H), 1.66 (d, 3H), 1.19 (t, 3H). | 15.45 (1) | A |
| A-34 | (2*S*)-2-(2-acetyl-4-chlorophenoxy)propanoi c acid | (300 MHz, CDCl3) δ 7.45 (dd, 1H), 4.91 (q, 1H), 2.67 (s, 3H), 1.75 (d, 3H). | 12.91 (1) | A |
| A-35 | 2-(4-bromophenoxy)-2-(cyclopenten-1-yl)acetic acid | (300 MHz, CD3OD) δ 7.32 (m, 2H), 5.86 - 5.82 (m, 1H), 5.00 (m, 1H), 2.50 - 2.40 (m, 2H), 1.94 - 1.83 (m, 2H). | 14.93 (1) | A |
| A-36 | (2*S*)-2-[4-bromo-2-(2,2-difluoroethenyl)phenoxy] propanoic acid | (300 MHz, CDCl3) δ 10.65-9.49 (br, 1H); 7.61 (s, 1H); 7.31 (d, 1H); 6.65 (d, 1H); 5.68 (dd, 1H); 4.78 (q, 1H); 1.70 (d, 3H). | 15.09 (1) | A |
| A-37 | (2*S*)-2-{2-[2-(benzyloxy)cyclobutyl]- | (500 MHz, CDCl3) δ 7.38 - 7.29 (m, 5H), 7.18 (dd, J = | 4.79 and 4.95 | A |
| | 4-chlorophenoxy}propanoi c acid | 8.7, 2.6 Hz, 0.5H), 7.15 (ddd, J = 8.7, 2.6, 0.8 Hz, 0.5H), 7.12 (d, J = 2.6 Hz, 0.5H), 7.06 (dd, J = 2.6, 1.0 Hz, 0.5H), 6.74 (d, J = 8.7 Hz, 0.5H), 6.69 (d, J = 8.7 Hz, 0.5H), 5.00 - 4.94 (m, 0.5H), 4.81 (q, J = 6.7 Hz, 0.5H), 4.65 (d, J = 12.3 Hz, 0.5H), 4.60 (d, J = 12.5 Hz, 0.5H), 4.51 (app dd, J = 12.4, 5.5 Hz, 2H), 4.01 - 3.94 (m, 0.5H), 3.81 - 3.71 (m, 1H), 3.64 (q, J = 8.9 Hz, 0.5H), 2.25 - 2.17 (m, 1H), 2.15 - 2.09 (m, 0.5H), 2.06 - 1.73 (m, 2.5H), 1.59 (d, J = 6.7 Hz, 1.5H), 1.56 (d, J = 6.8 Hz, 1.5H). | (2) | |
| A-39 | (2*S*)-2-[4-bromo-2-(2-methoxyethyl)phenoxy]p ropanoic acid | (300 MHz, CD₃OD) δ 7.26 (s, 1H), 6.70 (d, 1H), 4.43 (q, 1H), 1.56 (d, 3H); | 11.135 (1) | A |
| A-40 | (2*S*)-2-[2,4-dibromo-6-(2-methoxyethyl)phenoxy]p ropanoic acid | (300 MHz, CD3OD) δ 7.58 (s, 1H), 7.42 (s, 1H), 4.61 (q, 1H), 1.50 (d, 3H); | 11.934 (1) | A |
| A-41 | (2*S*)-2-[4-bromo-2-(cyclopropylidenemethyl )phenoxy]propanoic acid | (300MHz, CDCl3) δ 7.87 (s, 1H), 7.24 (d, 1H), 7.11 (s, 1H), 6.61-6.10 (br, 1 H), 4.77 (q, 1 H), 1.43 (t, 2H), 1.19 (t, 2H). | 12.039 (1) | A |
| A-42 | (2S)-2-(4-bromo-2-ethenyl-5-fluorophenoxy)propanoi c acid | (300MHz, CDCl3): δ 10.77-10.14 (br, 1H), 7.64 (d, 1H), 6.96 (dd, 1H), 5.71 (d, 1H), 4.75 (q, 1H), 1.72 (d, 3H). | 13.34 (1) | A |
| A-43 | (2S)-2-(2-acetyl-4-bromo-5-fluorophenoxy)propanoi c acid | (500 MHz, DMSO-d6) δ 13.33 (s, 1H); 7.83 (d, 1H); 7.25 (d, 1H); 5.21 (q, 1H); 2.59 (s, 3H); 1.59 (d, 3H) (free acid). | 3.351 (2) | A |
| A-44 | (2S)-2-(4-bromo-2-cyclopropyl-5-fluorophenoxy)propanoi c acid | (300MHz, CDCl3) δ 11.15-10.59 (br, 1H), 7.01 (d, 1H), 6.57 (d, 1H), 2.18-2.04 (m, 1H), 1.01-0.88 (m, 2H), 0.75-0.53 (m, 2H). | 12.81 (1) | A |
| A-45 | (2S)-2-[4-bromo-2-(2,2-difluoroethenyl)-5-fluorophenoxy]propanoic acid | (300MHz, CDCl3) δ 9.09-8.05 (br, 1H), 7.64 (d, 1H), 5.60 (dd, 1H), 4.74 (d, 1H) 1.69 (d, 3H). | 12.97 (1) | A |
| A-46 | (2S)-2-(4-bromo-2-ethynylphenoxy)-2-cyclobutylacetic acid | (300MHz, CDCl3): δ 10.37-9.49 (br, 1H), 7.60 (d, 1H), 7.38 (dd, 1H), 4.59 (d, 1H), 3.35 (s, 1H), 2.36-1.79 (m, | 13.32 (1) | A |
| | | 6H). | | |
| A-47 | (2S)-2-(4-bromo-2-ethynyl-5-fluorophenoxy)propanoi c acid | (300MHz, CDCl3) δ 10.25-9.38 (br, 1H), 7.66 (d, 1H), 4.83 (q, 1H), 3.31 (s, 1H), 1.74 (d, 3H). | 14.915 (1) | A |
| A-48 | (2S)-2-(4-chloro-2-ethynyl-5-fluorophenoxy)propanoi c acid | (300 MHz, CDCl3) δ 11.24 (s, 1H), 6.68 (d, 1H), 4.82 (q, 1H), 1.73 (d, 3H); | 11.668 (1) | A |
| A-49 | (2S)-2-(4-bromo-2-cyclopropylphenoxy)-4-fluorobutanoic acid | (300MHz, CDCl3) δ 10.50-9.46 (br, 1H), 7.20 (dd, 1H), 6.99 (d, 1H), 4.81 (dd, 1 H), 4.66 (dd, 1H), 2.59-2.25 (m, 2H), 2.24-2.10 (m, 1H), 0.81-0.55 (m, 2H). | 14.405 (1) | A |
| A-50 | (2S)-2-(4-bromo-2-ethynylphenoxy)-4-fluorobutanoic acid | (300MHz, CDCl3): δ 9.71-8.62 (br, 1H), 7.61 (d, 1H), 7.40 (dd, 1H), 4.91 (dd, 1H), 4.83 (t, 1H), 4.68 (t, 1H), 2.60-2.26 (m, 2H). | 11.89 (1) | A |
| A-51 | (2S)-2-(4-bromo-2-ethenylphenoxy)-4-fluorobutanoic acid | (300MHz, CD3OD): δ 7.54 (d, 1H), 7.22 (dd, 1H), 7.08 (dd, 1H), 5.69 (d, 1H), 5.23 (d, 1H), 4.58-4.40 (m, 1H), 2.43-2.08 (m, 2H). | 12.03 (1) | A |
| A-52 | (2S)-2-{4-bromo-2-[(1E)-2-fluoroethenyl]phenoxy}p ropanoic acid | (300MHz, CDCl3): δ 11.16-10.48 (br, 1H), 7.56 (d, 0.5 H), 7.36 (d, 1H), 7.28 (dd, 1.5 H),6.48 (dd, 1H), 4.83 (q, 1H), 1.71 (d, 3H). | 14.93 | A |
| A-53 | (2S)-2-{4-chloro-2-[(1E)-2-fluoroethenyl]phenoxy}p ropanoic acid | (300MHz, CDCl3): δ 10.85-10.15 (br, 1H), 7.57 (d, 0.5 H), 7.29 (d, 0.5H), 7.21 (d, 1H), 7.14 (dd, 1H), 4.83 (q, 1H), 1.71 (d, 3H). | 14.77 (1) | A |
| A-54 | (2S)-2-(4-bromo-2-ethynylphenoxy)butanoi c acid | (300MHz, CDCl3): δ 7.61 (d, 1H), 7.39 (dd, 1H), 6.71 (d, 1H), 4.70 (t, 1H), 2.1 (q, 2H), 1.14 (t, 3H). | 14.21 (1) | A |
| A-55 | 2-(4-bromo-2-cyclopropylphenoxy)ace tic acid | (400 MHz, DMSO-d6) δ 7.14 (dd, 2.5 Hz, 1H); 6.82 (d, 1H); 6.61 (d, 1H); 4.10 (s, 2H); 2.18 (tt, 1H); 1.00-0.83 (m, 2H); 0.73-0.49 (m, 2H). | 2.208 (2) | A |
| A-56 | 2-(4-bromo-2-ethynylphenoxy)acetic acid | (300MHz, CD3OD): δ 7.49 (s, 1H), 7.39 (d, 1H), 6.79 (d, 1H), 4.45 (s, 2H). | 9.59 (1) | A |
| A-57 | (2S)-2-[4-bromo-2-(2,2-difluorocyclobutyl)pheno xy]propanoic acid | (300MHz, CDCl3) δ 7.45-7.19 (m, 2H), 6.71-6.51 (m, 1H), 5.12-4.35-4.06 (m, 1 H), 2.80 (s, 0.5H), 2.25-1.94 (m, 0.5H), 1.64 (t, 3H). | 12.221 (1) | A |

### Specific examples of Syntheses

### Example 1: Synthesis of (S)-2-(4-bromo-2-ethynylphenoxy)propanoic acid; following the synthetic strategy of General Method A

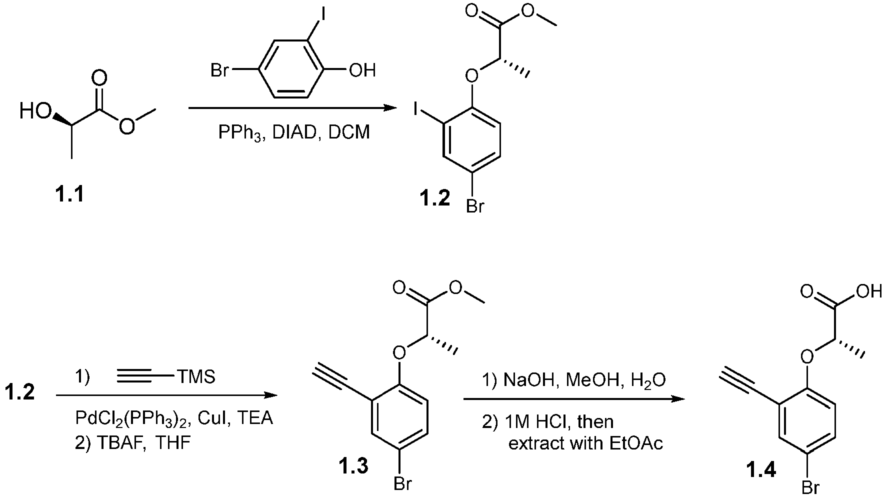

### (S)-Methyl 2-(4-bromo-2-iodophenoxv)propanoate (1.2)

To a solution of (*R*)-methyl 2-hydroxypropanoate (**1.1**) (1 mmol), 4-bromo-2-iodophenol (1 mmol) and triphenylphosphine (1.2 mmol) in THF (15 mL) at 0°C was added DIAD (1.2 mmol) dropwise over 20 min. The solution was stirred for a further 15 min at 0 °C. The bright yellow solution was allowed to warm to RT and stirred overnight. Volatiles were removed *in vacuo* to afford a dark orange oil. The crude product was purified by chromatography on silica gel (0-10% EtOAc/hexane) to afford (S)-methyl 2-(4-bromo-2-iodophenoxy)propanoate (**1.2**) (75% yield) as a colourless oil. ¹H NMR (300 MHz, CDCl3) δ 7.91 (d, 1H); 7.37 (dd, 1H); 6.58 (d, 1H); 4.73 (q, 1H); 3.77 (s, 3H); 1.70 (d, 3H); ES-MS: 386 [M+1].

### (S)-Methyl 2-(4-bromo-2-ethynylphenoxy) propanoate (1.3)

1) A mixture of (*S*)-methyl 2-(4-bromo-2-iodophenoxy)propanoate (**1.2**) (1 mmol), PdCl₂(PPh₃)₂ (4 mol%), copper Iodide (4 mol%) and ethynyltrimethylsilane (1.5 mmol) in triethylamine (10 mL) was evacuated and purged with argon 3 times and the mixture was heated at 80°C for 8 h then allowed to cool to room temperature. Volatiles were removed *in vacuo* and the crude product was purified by chromatography on silica gel (0-10% EtOAc/hexane) to afford (S)-methyl 2-(4-bromo-2-((trimethylsilyl)ethynyl)phenoxy) propanoate as a brown oil. The crude product was used with no further purification in step 2.
2) (*S*)-Methyl 2-(4-bromo-2-((trimethylsilyl)ethynyl)phenoxy)propanoate (1 mmol) was dissolved in dry THF (16 mL) and cooled to 0°C. TBAF (1.2 mmol) was added and the mixture was stirred at room temperature for 60 min. The reaction was quenched with water and extracted with EtOAc (3 × 500 mL). Organics were washed with brine (50 mL) dried over sodium sulphate, filtered and evaporated. The crude product was purified by chromatography on silica gel (0-10% EtOAc/hexane) to afford (S)-methyl 2-(4-bromo-2-ethynylphenoxy) propanoate (1.3) (82% yield) as a colourless oil. ¹H-NMR (300MHz, CDCl3): δ 7.58 (d, 1H), 7.36 (dd, 1H), 6.68 (d, 1H), 4.79 (q, 1H), 3.75 (s, 3H), 3.33 (s, 1H), 1.67 (d, 3H). ES-MS: 284 [M+1].

### (S)-2-(4-bromo-2-ethynylphenoxy)propanoic acid (1.4)

To a stirred solution of (S)-methyl 2-(4-bromo-2-ethynylphenoxy) propanoate (**1.3**) (1.0 mmol) in MeOH: H₂O (3:1) at room temperature solid NaOH (1.1 eq) was added and the resulting mixture was stirred at room temperature for 40 min. After completion of the reaction, volatiles were removed and the crude product was diluted with water and the aqueous layer was washed with DCM (2X50 mL) (to remove unreacted ester and other impurities). The aqueous phase was acidified with aq. HCl (1 M) and extracted with EtOAc. The combined organic phases were dried over MgSO₄. The solvents were removed and dried under vacuum to afford (*S*)-2-(4-bromo-2-ethynylphenoxy)propanoic acid (**1.4**) (95% yield) as an off-white solid. ¹H-NMR (300MHz, CDCl3): δ 9.13-8.37 (br, 1H), 7.61 (s, 1H), 7.40 (dd, 1H), 6.74 (d, 1H), 4.84 (q, 1H), 3.36 (s, 1H), 1.72 (d, 3H). ES-MS: 268 [M-1].

In conclusions, this example demonstrates that compound **1.4** can be prepared using the synthetic strategy of general method A.

### Example 2: Synthesis of (2S)-2-(4-bromo-2-ethenylphenoxy)propanoic acid; following the synthetic strategy of General Method A

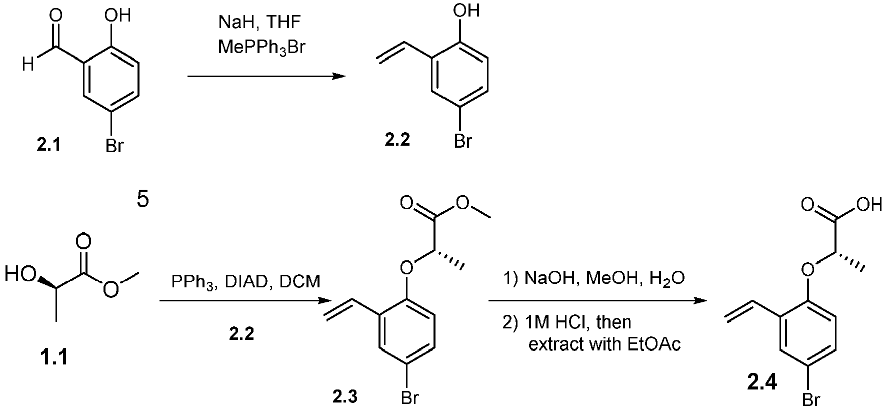

### 4-Bromo-2-vinylphenol (2.2)

To a solution of sodium hydride (6 mmol) in THF (15 mL) at 0°C, methyltriphenylphosphonium bromide (2 mmol) was added and the mixture stirred for 1h before 5-bromo-2-hydroxybenzaldehyde (**2.1**) (1mmol) in THF was introduced dropwise over 20 min. The solution was stirred for a further 15 min. at 0°C. The bright yellow solution was allowed to warm to RT and stirred overnight. The reaction was quenched with aqueous sat. ammonium chloride solution and the aqueous solution was extracted with EtOAc (2×100mL). The combined organics were dried over MgSO₄ and adsorbed onto silica. The crude product was purified by chromatography on silica gel (0-10% EtOAc/hexane) to afford 4-bromo-2-vinylphenol (**2.2**) (65% yield) as a colourless liquid. ¹H NMR (300 MHz, CDCl3) δ 7.58 (d, 1H); 7.34 (dd, 1H); 6.98 (dd, 1H); 6.75 (d, 1H); 5,75 (d, 1H); 5.32(d, 1H); 3.84 (s, 3H); ES-MS: 198 [M-1].

### (S)-Methyl 2-(4-bromo-2-vinylphenoxy) propanoate (2.3)

To a solution of (*R*)-methyl 2-hydroxypropanoate (7) (1 mmol), 4-bromo-2-vinylphenol (**6**) (1 mmol) and triphenylphosphine (1.2 mmol) in THF (15 mL) at 0 °C was added DIAD (1.2 mmol) dropwise over 20 min. The solution was stirred for a further 15 min at 0 °C. The bright yellow solution was allowed to warm to RT and stirred overnight. Volatiles were removed *in vacuo* to afford a dark orange oil. The crude product was purified by chromatography on silica gel (0-10% EtOAc/hexane) to afford (*S*)-methyl 2-(4-bromo-2-vinylphenoxy) propanoate (**2.3**) (78% yield) as a colourless oil. ¹H NMR (300 MHz, CDCl3) δ 7.61 (d, 1H); 7.27 (dd, 1H); 7.05 (dd, 1H); 6.62 (d, 1H); 5.76 (d, 1H); 5.33 (d, 1H); 4.74 (q, 1H); 3.76 (s, 3H); 1.65 (d, 3H); ES-MS: 286 [M+1].

### (S)-2-(4-Bromo-2-vinylphenoxy)propanoic acid (2.4)

To a stirred solution of (*S*)-methyl 2-(4-bromo-2-vinylphenoxy)propanoate (**2.3**) (1.0 mmol) in MeOH and H₂O (3:1) at room temperature solid NaOH (1.1 eq) was added and the resultant mixture was stirred at room temperature for 40 min. After completion of the reaction, volatiles were removed and water was added to the residue. The aqueous layer was washed with DCM (2 × 50 mL) (to remove unreacted ester and other impurities). The aqueous layer was acidified with aq. HCl (1 M) and extracted with EtOAc (2 × 50 mL). The combined organic extracts were dried over MgSO₄. The solvents were removed and dried under vacuum to afford (*S*)-2-(4-bromo-2-vinylphenoxy)propanoic acid (**2.4**) (93 % yield) as an off-white solid. ¹H-NMR (300MHz, CDCl3): δ 10.17-9.48 (br, 1H), 7.62 (s, 1H), 7.30 (d, 1H), 7.03 (dd, 1H), 6.66 (d, 1H), 5.77 (d, 1H), 5.34 (d, 1H), 4.77 (q, 1H), 1.69 (d, 3H). ES-MS: 270 [M-1].

In conclusions, this example demonstrates that compound 2.4 can be prepared using the synthetic strategy of general method A.

### Example 3: Synthesis of (2S)-2-(4-bromo-2-cyclobutylphenoxy)propanoic acid and sodium salt thereof, following the synthetic strategy of General Method A

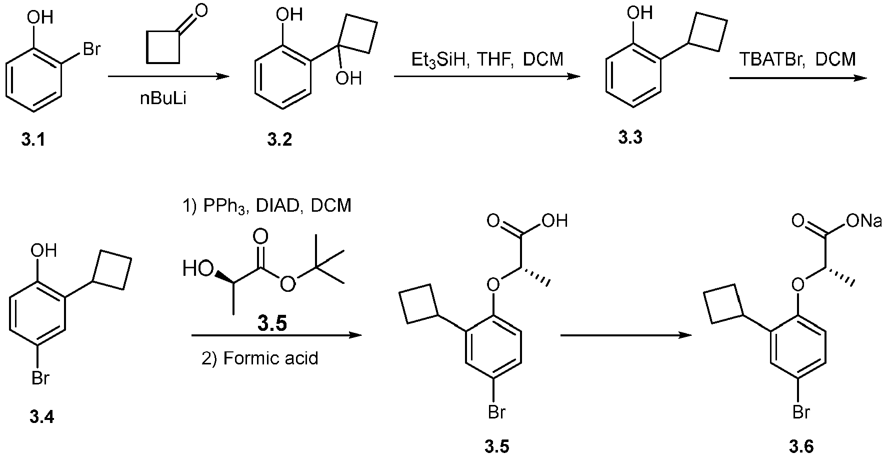

### 2-(1-Hydroxycyclobutyl)phenol (3.2)

2-Bromophenol (**3.1**) (8.04 mL, 69.4 mmol) was dissolved in dry diethyl ether (144 mL) and cooled to -78 °C under nitrogen. N-butyllithium (2.5 M in hexane) (61.0 mL, 153 mmol) was added dropwise maintaining the reaction temperature below - 70 °C. Once the addition was complete the cooling bath was removed and the reaction mixture allowed to warm to RT and stirred for 2.5 hours. The reaction mixture was cooled to - 78°C whereafter cyclobutanone (5.18 ml, 69.4 mmol) was added dropwise while maintaining the reaction temperature below -70°C. Once the addition was complete, the cooling bath was removed and the reaction mixture was allowed to warm to RT and left to stir overnight. The reaction mixture was quenched into ice cold ammonium chloride solution (500 mL) then extracted with EtOAc (3 × 500 mL). Organics were washed with brine (500 mL) dried over sodium sulphate, filtered and evaporated to give 2-(1-hydroxycyclobutyl)phenol (**3.2**) as a viscous orange oil (10.78 g, 57.1 mmol, 82 % yield). The product was analysed by LCMS (Agilent, X-Select, Waters X-Select C18, 2.5 µm, 4.6x30 mm, Acidic (0.1% Formic acid) 4 min method, 5-95% MeCN/ water): M/Z 163 (M+H)+ (ES+); at 1.54 min, 53% purity @ 254 nm.

### 2-Cyclobutylphenol (3.3)

2-(1-Hydroxycyclobutyl)phenol (3.2) (10.7 g, 56.7 mmol) was dissolved in dry DCM (160 mL) and cooled to 0°C. Triethylsilane (19.47 mL, 122 mmol) was added and the clear solution maintained at 0°C for 20 min. before TFA (17.47 mL, 227 mmol) was added dropwise. The stirring was continued for a further 10 min. whereafter the mixture was allowed to warm to RT and stirred overnight. The reaction mixture was quenched into ice-water (500 mL) and extracted with DCM (3 × 200 mL). The combined organics were dried using a phase separating cartridge and evaporated to give a deep orange oil (16.63 g, crude) The product was purified by liquid loading in DCM onto a 330g silica cartridge which was slow gradient eluted (100% isohexane to 20% EA) to give 2-cyclobutylphenol (3.3) (6.78 g, 43.9 mmol, 77 % yield). The product was analysed by LCMS (Agilent, X-Select, Waters X-Select C18, 2.5 µm, 4.6×30 mm, Acidic (0.1% Formic acid) 4 min method, 5-95% MeCN/water): 1916-91-2, M/Z 147 (M-H)- (ES-), at 2.152 min, 97% purity @ 254 nm.

### 4-Bromo-2-cyclobutylphenol (3.4)

Tetra-n-butylammonium tribromide (TBATBr) (22.06 g, 45.7 mmol) was added in a single portion to a stirred solution of 2-cyclobutylphenol (3.3) (6.78 g, 45.7 mmol) in dry DCM (136 ml, 2109 mmol) at room temperature. After 30 minutes, the reaction mixture was quenched by pouring it into a solution of sodium metabisulphite (500 mL) and extracted with DCM (500 mL). The aqueous phase was extracted with further DCM (2 × 100 mL). The combined organic extracts were dried using a phase separating cartridge and evaporated to give an opaque oil which was purified by liquid loading in DCM onto a 330g silica cartridge which was slow gradient eluted (100% isohexane to 20% EAisohexane) to give 4-bromo-2-cyclobutylphenol (**3.4**) (4.35g, 18.01 mmol, 39.4 % yield). The product was analysed by LCMS (Agilent, X-Select, Waters X-Select C18, 2.5 µm, 4.6x30 mm, Acidic (0.1% Formic acid) 4 min method, 5-95% MeCN/water): M/Z 225/227 (M-H)- (ES-), at 2.453 min, 97.7% purity @ 254 nm.

### (S)-2-(4-Bromo-2-cyclobutylphenoxy)propanoic acid (3.5)

DIAD (5.59 ml, 28.7 mmol) was added to a stirred solution of 4-bromo-2-cyclobutylphenol (**3.4**) (4.35 g, 19.15 mmol), (*R*)*-tert-butyl* 2-hydroxypropanoate (**3.5**) (3.08 g, 21.07 mmol) and triphenylphosphine (7.54 g, 28.7 mmol) in anhydrous THF (180 mL) at room temperature. After 16 hours, the solvent was removed under reduced pressure, the residue dissolved in formic acid (73.5 ml, 1915 mmol) and heated to 70 °C for 1 hour. The solution was evaporated to give a yellow oil which was co-evaporated with toluene (3 × 50mL). The residue was dissolved in ethyl acetate (100ml) and extracted with 2M sodium hydroxide solution (100mL). The aqueous phase was washed with ethyl acetate (2 × 100mL) then acidified to pH 2 with 1N HCl and extracted with EtOAc (3 × 100mL). The organic extracts were dried over magnesium sulphate, filtered and then evaporated to give a pale yellow oil which purified by was liquid loading in DCM onto a 80 g silica cartridge and gradient eluted (100% isohexane to 40% ethyl acetate-isohexane) to afford (S)-2-(4-bromo-2-cyclobutylphenoxy)propanoic acid (3.5) (1.33 g, 5.04 mmol, 26.3 % yield); ¹H NMR (400 MHz, DMSO-d6) δ 13.02 (s, 1H), 7.28 (d, J = 8.2 Hz, 2H), 6.70 (d, J = 8.3 Hz, 1H), 4.80 (q, J = 6.7 Hz, 1H), 3.66 (p, J = 8.8 Hz, 1H), 2.25 (dqt, J = 12.1, 6.2, 2.0 Hz, 2H), 2.17 - 1.89 (m, 3H), 1.80 - 1.72 (m, 1H), 1.49 (d, J = 6.7 Hz, 3H).

### Sodium (S)-2-(4-bromo-2-cyclobutylphenoxy)propanoate (3.6)

To a solution of (S)-2-(4-bromo-2-cyclobutylphenoxy)propanoic acid (**3.5**) (688 mg, 2.300 mmol) in a mixture of MeCN (15 mL) and H₂O (5 mL) was added 1M sodium hydroxide solution(2.185 mL, 2.185 mmol). The mixture was stirred at room temperature for 30 minutes whereafter solvent was removed under reduced pressure at 50 °C. The residue was co-evaporated with dry toluene (2 × 10 mL) then dried *in vacuo* at 50 °C overnight. The resulting solid was triturated with a mixture of isohexane-diethyl ether (10mL:1mL), the resulting solid was filtered and dried *in vacuo* at 50 °C for 3 hours to give pure sodium (S)-2-(4-bromo-2-cyclobutylphenoxy)propanoate (**3.6**) (732 mg, 2.234 mmol, 97 % yield), as a slightly hygroscopic light cream coloured solid. The product was analysed by LCMS (Waters Acquity UPLC, Acidic (0.1% Formic acid) 10 min method, 5-95% MeCN/water): (ES+); 297/299 (M-H)- (ES-), at 5.058 min, 98.9% purity @ 200-400 nm. ¹H NMR (400 MHz, DMSO-d⁶) δ 7.16 (d, J = 8.3 Hz, 2H), 6.63 (d, J = 8.3 Hz, 1H), 4.17 (q, J = 6.7 Hz, 1H), 3.66 (p, J = 8.8 Hz, 1H), 2.32 - 2.08 (m, 3H), 2.04 - 1.87 (m, 2H), 1.82 - 1.69 (m, 1H), 1.36 (d, J = 6.7 Hz, 3H).

In conclusions, this example demonstrates that compound **15** and the sodium salt thereof, can be prepared using the synthetic strategy of general method A.

### Example 4: Synthesis of (2S)-2-(4-chloro-2-cyclopropylphenoxy)propanoic acid and sodium salt thereof, following the synthetic strategy of General Method A

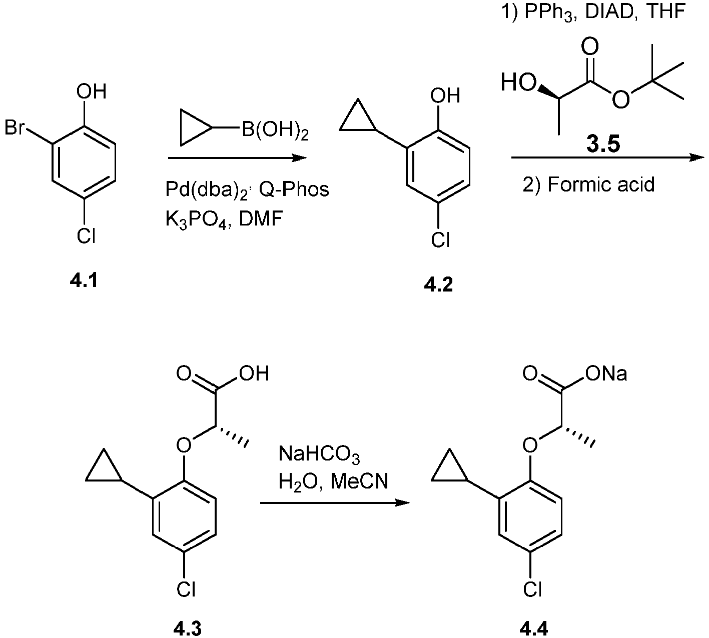

### 4-Chloro-2-cyclopropylphenol (4.2)

A mixture of 2-bromo-4-chlorophenol (**4.1**) (4.15 g, 20 mmol), cyclopropylboronic acid (3.44 g, 40.0 mmol) and potassium phosphate (5.44 g, 40.0 mmol) in DMF (28.6 ml) was evacuated and purged with nitrogen 3 times. Pd(dba)₂ (0.230 g, 0.400 mmol) and Q-phos (0.569 g, 0.800 mmol) were added and the mixture was evacuated and purged with nitrogen 3 further times. The mixture was heated at 120°C under microwave irradition for 8 h then allowed to cool to room temperature. The mixture was poured into water (50 mL) and acidified by addition of 1 M aq. HCl. The mixture was extracted with EtOAc (3 × 50 mL) and the combined organics were washed with water (150 mL), brine (150 mL), dried (MgSO4), filtered and concentrated under reduced pressure. The crude product was purified by chromatography on silica gel (80 g column, 0-10% EtOAc/isohexane), giving a mixture of orange solid and brown oil. The mixture was suspended in isohexane then filtered and the solvent removed under reduced pressure to afford 4-chloro-2-cyclopropylphenol (**4.2**) (1.9985 g, 11.45 mmol, 57.2% yield) as a clear brown oil. ¹H NMR in CDCl₃ was consistent with product structure at 97% purity.

### (S)-2-(4-Chloro-2-cyclopropylphenoxy)propanoic acid (4.3)

DIAD (3.12 ml, 16.03 mmol) was added to a stirred solution of (*R*)*-tert*-butyl 2-hydroxypropanoate (**14**) (1.841 g, 12.60 mmol), 4-chloro-2-cyclopropylphenol (**4.2**) (1.931 g, 11.45 mmol) and triphenylphosphine (4.20 g, 16.03 mmol) in anhydrous tetrahydrofuran (67.4 mL) at 0 °C and stirred for 30 minutes whereafter the reaction mixture was allowed to warm to room temperature. After 16 hours, the mixture was evaporated *in vacuo* to a syrup which was re-dissolved in formic acid (44ml) and heated to 70 °C for 1 hour. The resulting solution was evaporated *in vacuo* and the residue co-evaporated with toluene (2 × 30mL). The residue was dissolved in ethyl acetate (100mL) and extracted with 0.5M sodium hydroxide solution (100mL). The aqueous phase was washed with ethyl acetate (2 × 100mL) then acidified to pH 2-3 by dropwise addition of conc. hydrochloric acid. The resulting cloudy solution was extracted with ethyl acetate (3x 100mL). Organic extracts were filtered through a phase separating funnel. The residue was purified by column chromatography (40g Grace silica cartridge) with 0-30% ethyl acetate in isohexane gradient elution to give an oil which was dried *in vacuo* at 40 °C overnight to give: (S)-2-(4-chloro-2-cyclopropylphenoxy)propanoic acid (4.3) (0.792 g, 3.09 mmol, 27.0 % yield) as a colourless solid. (600 mg).

The product was analysed by LCMS (Waters Acquity UPLC, X-Select, Waters X-Select UPLC C18, 1.7 µm, 2.1x30mm, Acidic (0.1% Formic acid) 10 min method, 5-95% MeCN/water): M/Z 239.106 (M-H)- (ES-), at 4.184 min, 94% purity @ 210-400 nm. ¹H NMR in DMSO-d⁶ 1974-11-a1 was consistent with product structure at >95% purity. 1H NMR (400 MHz, DMSO-d6) δ 13.05 (s, 1H), 7.11 (dd, J = 8.7, 2.6 Hz, 1H), 6.82 (d, J = 2.7 Hz, 1H), 6.79 (d, J = 8.8 Hz, 1H), 4.83 (q, J = 6.7 Hz, 1H), 2.23 - 2.13 (m, 1H), 1.53 (d, J = 6.7 Hz, 3H), 0.99 - 0.87 (m, 2H), 0.79 - 0.71 (m, 1H), 0.71 - 0.60 (m, 1H).

### Sodium (S)-2-(4-chloro-2-cyclopropylphenoxy)propionate (4.4)

To (*S*)-2-(4-chloro-2-cyclopropylphenoxy)propanoic acid (**4.3**) (0.604 g, 2.510 mmol) in MeCN (25 mL) was added NaHCO₃ (0.211 g, 2.510 mmol) in H₂O (8 mL) and the mixture was stirred at room temperature for 30 min. The solvent was removed under reduced pressure to give a white solid which was dissolved in H₂O (30 mL) and washed with DCM (3 × 30 mL). The water was removed under reduced pressure and the resultant solid was dried at 45°C *in vacuo* for 24 hours to afford pure sodium (*S*)-2-(4-chloro-2-cyclopropylphenoxy)propanoate (4.4) (0.652 g, 2.383 mmol, 95 % yield) as a white solid. The product was analysed by LCMS (Waters Acquity UPLC, X-Select, Waters X-Select UPLC C18, 1.7 µm, 2.1×30mm, Acidic (0.1% Formic acid) 10 min method, 5-95% MeCN/water): M/Z 239.106 (M-H)- (ES-), at 4.181 min, 96% purity @ 210-400 nm. ¹H NMR (400 MHz, DMSO-d⁶) δ 6.98 (dd, J = 8.8, 2.7 Hz, 1H), 6.74 - 6.63 (m, 2H), 4.20 (q, J = 6.7 Hz, 1H), 2.20 (tt, J = 8.6, 5.3 Hz, 1H), 1.38 (d, J = 6.6 Hz, 3H), 0.97 - 0.83 (m, 2H), 0.77 - 0.66 (m, 1H), 0.66 - 0.56 (m, 1H).

In conclusions, this example demonstrates that compound 4.4 can be prepared using the synthetic strategy of general method A.

### Description of Pharmacological Methods and Drawings

### Example 5: Electrophysiological measurement of compound inhibition of CIC-1 in rat muscle

The investigatory goal of these experiments was to evaluate whether compounds inhibit CIC-1 channels in native tissue of rat skeletal muscle fibres. Apparent CIC-1 affinity was reported by the concentration of compound at which 50% of the compound's full inhibition of CIC-1 was observed (EC₅₀).

CIC-1 Cl⁻ ion channels generate around 80% of the total membrane conductance (Gₘ) in resting skeletal muscle fibres of most animals including rat and human (Bretag, A H. Muscle chloride channels. Physiological Reviews, 1987, 67, 618-724). Other ion channels that contribute to Gₘ can therefore be considered negligible, and it is possible to evaluate whether a compound inhibits CIC-1 in rat muscle by comparing Gₘ measurements before and after exposure to a compound. CIC-1 inhibition would in such recordings be reflected by a reduction of Gₘ.

Experimentally, Gₘ was measured in individual fibres of whole rat soleus muscles using a three micro-electrodes technique described in this example and in full detail elsewhere (Riisager et al., Determination of cable parameters in skeletal muscle fibres during repetitive firing of action potentials. Journal of Physiology, 2014, 592, 4417-4429). Briefly, intact rat soleus muscles were dissected out from 12-14 week old Wistar rats and placed in an experimental chamber that was perfused with a standard Krebs Ringer solution containing 122 mM NaCl, 25 mM NaHCO₃, 2.8 mM KCI, 1.2 mM KH₂PO₄, 1.2 mM MgSO₄, 1.3 mM CaCl₂, 5.0 mM D-glucose. During experiments, the solution was kept at approx. 30°C and continuously equilibrated with a mixture of 95% O₂ and 5% CO₂, pH ~7.4. The experimental chamber was placed in Nikon upright microscope that was used to visualize individual muscle fibres and the three electrodes (glass pipettes filled with 2 M potassium citrate). For Gₘ measurements, the electrodes were inserted into the same fibre with known inter-electrode distances of 0.35 - 0.5 mm (V1-V2, X1) and 1.1-1.5 mm (V1-V3, X3) (Figure 1A). The membrane potential of the impaled muscle fibre was recorded by all electrodes. Two of the electrodes were furthermore used to inject 50 ms current pulses of -30 nA. Given the positions of the electrodes, three different inter-electrode distances could be identified (X1-X2, X1-X3, X2-X3) and hence the membrane potential responses to the current injections could be obtained at three distances from the point of current injection. The steady state voltage deflection at each distance was divided by the magnitude of current injected (-30 nA) and the resulting transfer resistances were plotted against inter-electrode distance and the data was fitted to a mono-exponential function from which Gₘ could be calculated using linear cable theory (Figure 1B).

To establish a dose response relationship, Gₘ was first determined in 10 muscle fibres in the absence of compound and then at four increasing compound concentrations with Gₘ determinations in 5-10 fibres at each concentration. The average Gₘ values at each concentration were plotted against compound concentration and the data was fitted to sigmoidal function to obtain an EC₅₀ value (Figure 1C). Table 2 shows the EC₅₀ values for a range of compounds with n values referring to number of experiments that each reflect recordings from around 50 fibres.

**Table 2: Inhibition of CIC-1 ion channel using compounds of the invention**

| **Compound investigated** | **EC₅₀ (µM)** |
|---|---|
| Compound A-14 | 4.3 ± 2.3 (n=4) |
| Compound A-17 | 4.2 ± 0.7 (n=3) |
| Compound A-19 | 3.5 ± 0.6 (n=4) |
| Compound A-27 | 8.3 (n=1) |
| Compound A-36 | 3.7 (n=1) |
| Compound A-46 | 6.1 (n=1) |
| Compound A-51 | 4.0 ± 3.0 (n=2) |
| Compound A-52 | 3.0 (n=1) |
| Compound A-54 | 7.2 ± 3.5 (n=2) |
| Compound A-55 | 6.4 (n=1) |

In conclusion, this example demonstrates that the compounds of the present invention have an EC₅₀ value in the range of 3-10 µM.

### Example 6: Measurement of force in an in vitro model

The current invention relates to compounds that inhibit CIC-1 ion channels and increase muscle excitability and thereby improve muscle function in clinical conditions where muscle activation is failing. Such conditions result in loss of contractile function of skeletal muscle, weakness and excessive fatigue. In this series of experiments the compounds were tested for their ability to restore contractile function of isolated rat muscle when the neuromuscular transmission had been compromised akin to neuromuscular disorders.

Experimentally, soleus muscles from 4-5 week old rats were isolated with the motor nerve remaining attached. The nerve-muscle preparations were mounted in experimental setups that enabled electrical stimulation of the motor nerve. Stimulation of the motor nerve led to activation of the muscle fibres and ensuing force production that was recorded. The nerve-muscle preparations were also in these experiments incubated in the standard Krebs Ringer (see example 5) and the solution was heated to 30°C and continuously equilibrated with a mixture of 95% O₂ and 5% CO₂, pH ~7.4.

After mounting the nerve-muscle preparation in the experimental setup, the contractile function of the muscle was initially assessed under the control conditions (Figure 2A). Sub-maximal concentration of tubocurarine (115 nM), an acetylcholine receptors antagonist, was then added to the experimental bath to impose partial inhibition of the ability of the motor nerve to activate the muscle fibres. The experimental condition mimics the failing neuromuscular transmission in a range of neuromuscular disorders. After addition of tubocurarine the contractile force declined over the next 90 mins to 10-50 % of the control force. 50 µM of the test compound was then added and the contractile force recovered despite the continued presence of tubocurarine. To quantify the ability of the compound to restore force the percentage of the initial force that was restored was determined after 40 mins of compound exposure (Figure 2B) and the point increase is reported in Table 3.

**Table 3: Percentage increase of initial force that was restored**

| **Compound investigated** | **Point increase (%)** |
|---|---|
| Compound A-14 | 17 |
| Compound A-17 | 21 |
| Compound A-19 | 31 |
| Compound A-27 | 36 |
| Compound A-36 | 16 |
| Compound A-44 | 12 |
| Compound A-47 | 15 |
| Compound A-48 | 14 |
| Compound A-51 | 27 |
| Compound A-54 | 18 |
| Compound A-55 | 25 |
| Compound A-56 | 14 |

In conclusion, this example demonstrates that the compounds of the present invention are able to increase muscle excitability and thereby improve muscle function in clinical conditions. The muscle contractility was recovered by 15-40 % points, which meant almost complete restoration of the force.

### Example 7: Screening of compounds on the human isoform of CIC-1 expressed in CHO cells using automated patch-clamp

The investigatory goal of these experiments was to evaluate how compounds affect the open probability and current amplitude of human CIC-1 channels expressed in CHO cells. Experiments were performed using an automated patch clamp system that allowed high throughput testing of whole cell patches together with both intracellular and extracellular addition of compound.

### Automated voltage clamp measurements

Automated whole cell patch clamp experiments were performed with the Qpatch 16 system (Sophion Bioscience, Ballerup, Denmark) at room temperature. Data acquisition and analysis were performed in the Qassay software (ver. 5.6, Odense).

### Voltage protocol and analysis of whole cell CIC-1 currents

To evoke CIC-1 currents in whole cell patches, the membrane potential was initially stepped from a holding potential of -30 mV to +60 mV for 100 ms and then to various test voltages (sweeps) ranging from +120 mV to -140 mV in steps of 20 mV for 300 ms. To obtain tail currents, the membrane potential was stepped to -100 mV after each test voltage for 300 ms and then relaxed to -30 mV for 2 sec between sweeps (Figure 3). I/V relationships for whole cell instant and steady state current amplitudes were obtained by plotting average current densities at the beginning and at the end of the 300 ms step against the membrane potential (Figure 4).

In order to determine the relative overall open probability (P₀), the instantaneous tail currents were normalized to the maximal tail current obtained following the most positive voltage step and plotted against the test voltage. Plots of normalized tail currents from each whole cell patch were then fitted to a Boltzmann function allowing determination of half activation voltages (V_{1/2}, Figure 5).

### Solutions

For automated patch clamp experiments extracellular solutions contained: 2 mM CaCl₂, 1 mM MgCl₂, 10 mM HEPES, 4 mM KCI, 145 mM NaCl, 10 mM Glucose, pH adjusted to 7.4 with NaOH (2 M). Osmolality adjusted to ~320 using sucrose.

Intracellular solutions contained: 80 mM CsF, 60 mM CsCI, 5/1 mM KOH/EGTA, 10 mM HEPES, 10 mM NaCl, pH adjusted to 7.2 with NaOH (2 M). Osmolality adjusted to ~320 mOsm using sucrose.

### Cell line information:

Cells used in patch clamp experiments were Chinese hamster ovary cells (CHO) constitutively expressing human CIC-1 channels. The amino acid sequence encoded by the cDNA used to create this cell line was identical to the translated sequence for GenBank accession number NM_000083.2. Cells were produced by Charles River (Catalogue CT6175, Cleveland OH, USA) in a cryopreserved format. Experiments were performed on the cells directly after thawing (3 × 10⁶ cells used in each experiment).

### Test protocol

To evaluate the compound effect on CIC-1, when applied directly to the intracellular side of the cell membrane, the half activation voltage, V_{1/2}, was determined from whole cell patches with compound added to the intracellular solution and then compared to V_{1/2} determined from control cell patches with only vehicle added to the intracellular solution. Additionally, the effect of extracellular added compound was evaluated by determine V_{1/2} and steady state current amplitudes before and after exchanging the extracellular solution to contain compound.

The difference in half activation voltage of CIC-1 channels, ΔV_{1/2}, was determined as the difference between the cell patches treated intracellularly with compound and control cells patches and is reported in Table 4 below. A positive shift in ΔV_{1/2} is reflecting CIC-1 channel inhibition by the tested compound. P-values of <0.05 is considered significant.

**Table 4: Percentage increase of initial force that was restored**

| **Compound investigated** | **ΔV1/2 (mV)** | **P-value** |
|---|---|---|
| Compound A-2 | 9.2 | <0.01 |
| Compound A-4 | 8.8 | <0.01 |
| Compound A-17 | 10.3 | <0.01 |
| Compound A-19 | 7.7 | <0.01 |
| Compound A-20 | 26.4 | <0.01 |
| Compound A-30 | 6.2 | <0.01 |
| Compound A-36 | 21.5 | <0.01 |

### Example 8: Measurement of In Situ Muscle Contractile Characteristics

Isometric hindlimb force was measured in 12-week old female Lewis rats in the presence and absence of compound.

Rats were placed under anesthesia with isoflurane (2-4%), intubated and subsequently connected to a micro ventilator (Microvent 1, Hallowell EMC, US). Two stimulation electrodes were inserted through the skin to stimulate the sciatic nerve. A small incision was made proximal to the ankle, to expose the Achilles tendon, which was tied by cotton string, and connected to a force transducer (Fort250, World Precision Instruments) with adjustable position (Vernier control). The Achilles tendon was then cut distal to the attached cotton string. The rat was placed on a heated pad, and to prevent movement artefacts from contraction of the ankle dorsiflexors, the foot was fixated by tape on a footplate.

Muscle contractile properties were assessed by applying an electrical current (under supramaximal voltage conditions) to the nerve and recording the force generated by the muscle. The muscle was stretched until maximal force was obtained, when assessed by 2 Hz stimulation. Isometric force was measured every 30 seconds at 12 Hz (Twitch), 10 pulses, and at every 5 minutes at 80 Hz (Tetanic) for 1 second (80 pulses). This stimulation pattern was employed throughout the experiment, expect in few cases where 80 Hz stimulation was replaced by 12 Hz (10 pulses). Neuromuscular transmission was partially inhibited by constant infusion of Cisatracurium (Nimbex, GlaxoSmithKline) at a concentration of 0.1 mg/kg at an adjustable infusion speed, adjusted individually for each animal to obtain a level of inhibition of ca. 50% of the forced generated at 12 Hz stimulation on the 4^{th} pulse. When the level of neuromuscular inhibition was stable, the test article was injected i.v. at the chosen concentration. The effect of test article was assessed on its ability to increase force generated from the stimulation pattern applied. The effect was assessed in the ability to increase force per se (tetanic, 80 Hz, stimulation), and the ratio between individual twitch peaks (12 Hz stimulation). The effect was monitored for at least 1 hour after injection of test article. In addition, the time from injection of test article to maximal effect on force (both twitch and tetanic) was noted and the time for the effect to disappear (return to baseline), if possible. When appropriate the infusion of neuromuscular blocking agent was ceased, with the stimulation pattern continued, and the return of force to control levels was monitored. Animals were sacrificed by cervical dislocation while still fully sedated.

Compound A-2 was dosed 21.5 mg/kg i.v. The average increase in tetanic force was 28.5% but there was no significant change in twitch peaks (3 experiments). Compound A-19 was dosed 40 mg/kg i.v. The average increase in tetanic force was 44.5% and the average increase in twitch peaks was 9.6% (3 experiments).

This demonstrates that compounds of the invention, such as Compounds A-2 and A-19 can restore force to muscles *in vivo* which have been partially inhibited by a neuromuscular blocker.

## Claims

1. A compound of Formula (I): 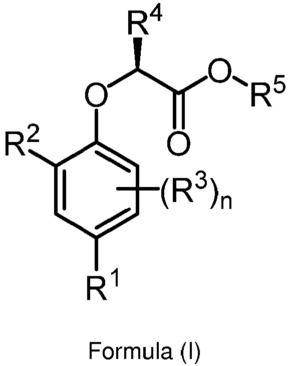 wherein:
- R¹ is selected from the group consisting of F, Cl, Br, I, -CN, -CF3, -CCl₃, - CHF₂, -CHCl₂, -CH₂F, -CH₂Cl, -OCF₃ and -OCCl₃;
- R² is selected from the group consisting of C₂₋₆ alkyl, C₂₋₆ alkenyl, C₂₋₆ alkynyl, C₃₋₆ cycloalkyl, C₅₋₆ cycloalkenyl, -C(=O)-C₁₋₅ alkyl, -C(=O)-C₁₋₅ alkenyl, -C(=O)-C₁₋₅ alkynyl, -C(=O)-C₃₋₅ cycloalkyl and -C(=O)-C₅ cycloalkenyl, each of which may be optionally substituted with one or more, identical or different, substituents R⁶;
- R³ is selected from the group consisting of hydrogen, deuterium, tritium, F, Cl, Br, I, -CN, -CF₃, -CCl₃, -CHF₂, -CHCl₂, -CH₂F, -CH₂Cl, -OCF₃, -OCCl₃ and isocyanide;
- R⁴ is selected from the group consisting of C₁₋₅ alkyl, C₁₋₅ alkenyl, C₁₋₅ alkynyl, C₃₋₅ cycloalkyl, C₅ cycloalkenyl, each of which may be optionally substituted with one or more, identical or different, substituents R⁷;
- R⁵ is selected from the group consisting of H, C₁₋₅ alkyl optionally substituted with one or more, identical or different, substituents R¹¹, C₂₋₅ alkenyl, C₂₋₅ alkynyl, C₃₋₆ cycloalkyl optionally substituted with one or more, identical or different, substituents R¹¹, phenyl optionally substituted with one or more, identical or different, substituents R¹² and benzyl optionally substituted with one or more, identical or different, substituents R¹²;
- R⁶ is independently selected from the group consisting of hydrogen, deuterium, tritium, F, Cl, Br, I, -CN, isocyanide, -O-C₁₋₃ alkyl, -S-C₁₋₃ alkyl, -CH₂-OH, -CH₂-O-C₁₋₃ alkyl, -O-CH₂-Ph, -CH₂-SH and -CH₂-S-C₁₋₃ alkyl;
- R⁷ is independently selected from the group consisting of deuterium, tritium, F, Cl, Br, I, -CN, isocyanide, -O-C₁₋₃ alkyl, -S-C₁₋₃ alkyl, -CH₂-O-C₁₋₃ alkyl and -CH₂-S-C₁₋₃ alkyl;
- R¹¹ is independently selected from the group consisting of deuterium and F;
- R¹² is independently selected from the group consisting of deuterium, methoxy, nitro, cyano, Cl, Br, I and F; and
- n is an integer 0, 1, 2 or 3;
or a pharmaceutically acceptable salt, hydrate, polymorph, tautomer, or solvate thereof,
for use in treating, ameliorating and/or preventing a neuromuscular disorder, and/or for use in reversing and/or ameliorating a neuromuscular blockade.

2. The compound for use according to claim 1, wherein:
- R¹ is selected from the group consisting of F, Cl, Br and I;
- R² is selected from the group consisting of ethyl, vinyl, ethynyl, cyclopropyl, cyclobutyl, -C(=O)-methyl and -C(=O)-ethyl, each of which may be optionally substituted with one or more, identical or different, substituents R⁶;
- R³ is selected from the group consisting of deuterium, tritium, F, Cl, Br and I;
- R⁴ is selected from the group consisting of C₁₋₅ alkyl, C₁₋₅ alkenyl, C₁₋₅ alkynyl, C₃₋₅ cycloalkyl, C₅ cycloalkenyl, each of which may be optionally substituted with one or more, identical or different, substituents R⁷;
- R⁵ is selected from the group consisting of H, C₁₋₅ alkyl optionally substituted with one or more, identical or different, substituents R¹¹, C₂₋₅ alkenyl, C₂₋₅ alkynyl, C₃₋₆ cycloalkyl optionally substituted with one or more, identical or different, substituents R¹¹, phenyl optionally substituted with one or more, identical or different, substituents R¹² and benzyl optionally substituted with one or more, identical or different, substituents R¹²;
- R⁶ is independently selected from the group consisting of deuterium, tritium, F, Cl, Br, I, -CN, isocyanide, -O-C₁₋₃ alkyl, -S-C₁₋₃ alkyl, -CH₂-OH, -CH2-O-C₁₋₃ alkyl, -O-CH₂-Ph, CH2-SH and -CH₂-S-C₁₋₃ alkyl;
- R⁷ is independently selected from the group consisting of deuterium, tritium, F, Cl, Br and I;
- R¹¹ is independently selected from the group consisting of deuterium and F;
- R¹² is independently selected from the group consisting of deuterium, methoxy, nitro, cyano, Cl, Br, I and F; and
- n is an integer 0 or 1.

3. The compound for use according to any one of claims 1 to 2, wherein the compound is of Formula (II): 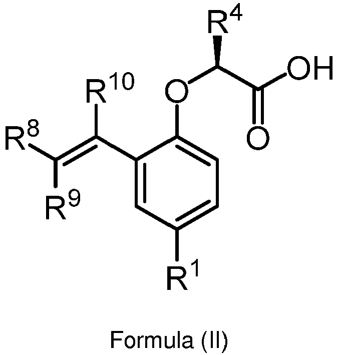 wherein:
R¹ is selected from the group consisting of F, Cl, Br, I, -CN, -CF₃, -CCl₃, - CHF₂, -CHCl₂, -CH₂F, -CH₂Cl, -OCF₃ and -OCCl₃;
R⁴ is methyl, ethyl, n-propyl, isopropyl or -CH₂F;
R⁸ and R⁹ are independently selected from the group consisting of hydrogen, deuterium, tritium, F, Cl, Br, I, -CN, isocyanide, -O-C₁₋₃ alkyl, -S-C₁₋₃ alkyl, -CH₂-OH, -CH₂-O-C₁₋₃ alkyl, -CH₂-SH, -CH₂-S-C₁₋₃ alkyl, C₁₋₄ alkyl and C₁₋₄ alkenyl and wherein the C₁₋₄ alkyl and C₁₋₄ alkenyl group may be optionally substituted with one or more, identical or different, substituents R⁶; and
R⁶ is independently selected from the group consisting of hydrogen, deuterium, tritium, F, Cl, Br, I, -CN, isocyanide, -O-C₁₋₃ alkyl, -S-C₁₋₃ alkyl, -CH₂-OH, -CH₂-O-C₁₋₃ alkyl, -O-CH₂-Ph, -CH₂-SH and -CH₂-S-C₁₋₃ alkyl.

4. The compound for use according to any one of claims 1 to 2, wherein the compound is of Formula (III): 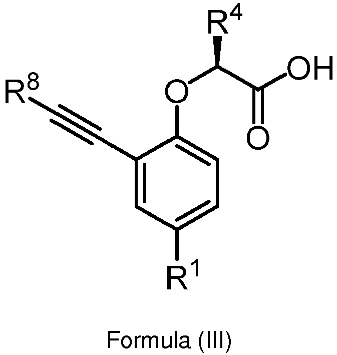 wherein:
R¹ is selected from the group consisting of F, Cl, Br, I, -CN, -CF3, -CCl₃, - CHF₂, -CHCl₂, -CH₂F, -CH₂Cl, -OCF₃ and -OCCl₃;
R⁴ is methyl, ethyl, n-propyl, isopropyl or -CH₂F;
R⁸ is independently selected from the group consisting of hydrogen, deuterium, tritium, F, Cl, Br, I, -CN, isocyanide, -O-C₁₋₃ alkyl, -S-C₁₋₃ alkyl, -CH₂-OH, -CH₂-O-C₁₋₃ alkyl, -CH₂-SH, -CH₂-S-C₁₋₃ alkyl, C₁₋₄ alkyl and C₁₋₄ alkenyl and wherein the C₁₋₄ alkyl and C₁₋₄ alkenyl group may be optionally substituted with one or more, identical or different, substituents R⁶; and
R⁶ is independently selected from the group consisting of hydrogen, deuterium, tritium, F, Cl, Br, I, -CN, isocyanide, -O-C₁₋₃ alkyl, -S-C₁₋₃ alkyl, -CH₂-OH, -CH₂-O-C₁₋₃ alkyl, -O-CH₂-Ph, -CH₂-SH and -CH₂-S-C₁₋₃ alkyl.

5. The compound for use according to any one of claims 1 to 2, wherein the compound is of Formula (IV): 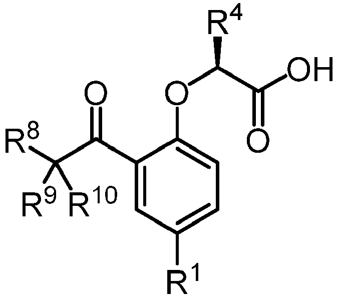 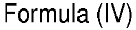 wherein:
R¹ is selected from the group consisting of F, Cl, Br, I, -CN, -CF₃, -CCl₃, - CHF₂, -CHCl₂, -CH₂F, -CH₂Cl, -OCF₃ and -OCCl₃;
R⁴ is methyl, ethyl, n-propyl, isopropyl or -CH₂F;
R⁶ is independently selected from the group consisting of hydrogen, deuterium, tritium, F, Cl, Br, I, -CN, isocyanide, -O-C₁₋₃ alkyl, -S-C₁₋₃ alkyl, -CH₂-OH, -CH₂-O-C₁₋₃ alkyl, -O-CH₂-Ph, -CH₂-SH and -CH₂-S-C₁₋₃ alkyl; and
R⁸, R⁹ and R¹⁰ are independently selected from the group consisting of hydrogen, deuterium, tritium, F, Cl, Br, I, -CN, isocyanide, -O-C₁₋₃ alkyl, - S-C₁₋₃ alkyl, -CH₂-OH, -CH₂-O-C₁₋₃ alkyl, -CH₂-SH, -CH₂-S-C₁₋₃ alkyl, C₁₋₄ alkyl and C₁₋₄ alkenyl and wherein the C₁₋₄ alkyl and C₁₋₄ alkenyl group may be optionally substituted with one or more, identical or different, substituents R⁶.

6. The compound for use according to any one of claims 1 to 2, wherein the compound is of Formula (V): 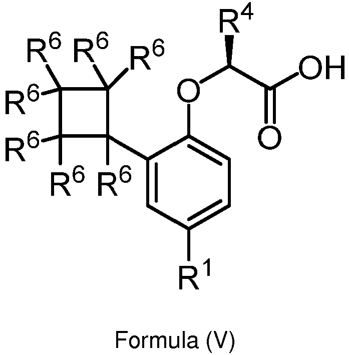 wherein:
R¹ is selected from the group consisting of F, Cl, Br, I, -CN, -CF₃, -CCl₃, - CHF₂, -CHCl₂, -CH₂F, -CH₂Cl, -OCF₃ and -OCCl₃;
R⁴ is methyl, ethyl, n-propyl, isopropyl or -CH₂F; and
R⁶ is independently selected from the group consisting of hydrogen, deuterium, tritium, F, Cl, Br, I, -CN, isocyanide, -O-C₁₋₃ alkyl, -S-C₁₋₃ alkyl, -CH₂-OH, -CH₂-O-C₁₋₃ alkyl, -O-CH₂-Ph, -CH₂-SH and -CH₂-S-C₁₋₃ alkyl.

7. The compound for use according to any one of claims 1 to 2, wherein the compound is of Formula (VI): 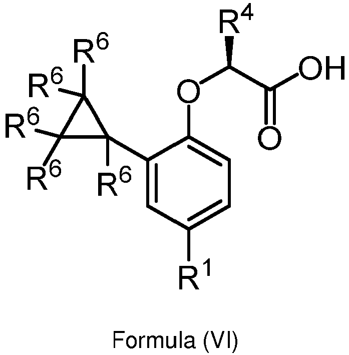 wherein:
R¹ is selected from the group consisting of F, Cl, Br, I, -CN, -CF₃, -CCl₃, - CHF₂, -CHCl₂, -CH₂F, -CH₂Cl, -OCF₃ and -OCCl₃;
R⁴ is methyl, ethyl, n-propyl, isopropyl or -CH₂F; and
R⁶ is independently selected from the group consisting of hydrogen, deuterium, tritium, F, Cl, Br, I, -CN, isocyanide, -O-C₁₋₃ alkyl, -S-C₁₋₃ alkyl, -CH₂-OH, -CH₂-O-C₁₋₃ alkyl, -O-CH₂-Ph, -CH₂-SH and -CH₂-S-C₁₋₃ alkyl.

8. The compound for use according to any one of claims 1 to 2, wherein the compound is of Formula (VII): 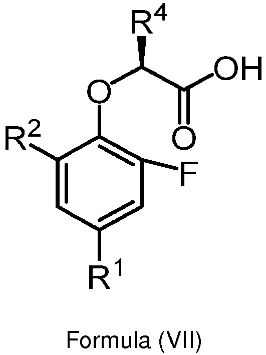 wherein:
R¹ is selected from the group consisting of F, Cl, Br, I, -CN, -CF₃, -CCl₃, - CHF₂, -CHCl₂, -CH₂F, -CH₂Cl, -OCF₃ and -OCCl₃;
R² is selected from the group consisting of C₂₋₆ alkyl, C₂₋₆ alkenyl, C₂₋₆ alkynyl, C₃₋₆ cycloalkyl, C₅₋₆ cycloalkenyl, -C(=O)-C₁₋₅ alkyl, -C(=O)-C₁₋₅ alkenyl, - C(=O)-C₁₋₅ alkynyl, -C(=O)-C₃₋₅ cycloalkyl and -C(=O)-C₅ cycloalkenyl, each of which may be optionally substituted with one or more, identical or different, substituents R⁶;
R⁴ is selected from the group consisting of C₁₋₅ alkyl, C₁₋₅ alkenyl, C₁₋₅ alkynyl, C₃₋₅ cycloalkyl, C₅ cycloalkenyl, each of which may be optionally substituted with one or more, identical or different, substituents R⁷;
R⁶ is independently selected from the group consisting of hydrogen, deuterium, tritium, F, Cl, Br, I, -CN, isocyanide, -O-C₁₋₃ alkyl, -S-C₁₋₃ alkyl, -CH₂-OH, -CH₂-O-C₁₋₃ alkyl, -O-CH₂-Ph, -CH₂-SH and -CH₂-S-C₁₋₃ alkyl; and
R⁷ is independently selected from the group consisting of deuterium, tritium, F, Cl, Br, I, -CN, isocyanide, -O-C₁₋₃ alkyl, -S-C₁₋₃ alkyl, -CH₂-O-C₁₋₃ alkyl and -CH₂-S-C₁₋₃ alkyl.

9. The compound for use according to any one of claims 1 to 2, wherein the compound is of Formula (VIII): 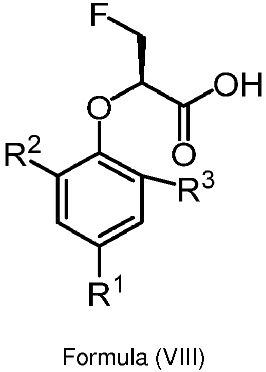 wherein:
R¹ is selected from the group consisting of F, Cl, Br, I, -CN, -CF₃, -CCl₃, - CHF₂, -CHCl₂, -CH₂F, -CH₂Cl, -OCF₃ and -OCCl₃;
R² is selected from the group consisting of C₂₋₆ alkyl, C₂₋₆ alkenyl, C₂₋₆ alkynyl, C₃₋₆ cycloalkyl, C₅₋₆ cycloalkenyl, -C(=O)-C₁₋₅ alkyl, -C(=O)-C₁₋₅ alkenyl, - C(=O)-C₁₋₅ alkynyl, -C(=O)-C₃₋₅ cycloalkyl and -C(=O)-C₅ cycloalkenyl, each of which may be optionally substituted with one or more, identical or different, substituents R⁶;
R³ is selected from the group consisting of hydrogen, deuterium, tritium, F, Cl, Br, I, -CN, -CF₃, -CCl₃, -CHF₂, -CHCl₂, -CH₂F, -CH₂Cl, -OCF₃, -OCCl₃ and isocyanide; and
R⁶ is independently selected from the group consisting of hydrogen, deuterium, tritium, F, Cl, Br, I, -CN, isocyanide, -O-C₁₋₃ alkyl, -S-C₁₋₃ alkyl, -CH₂-OH, -CH₂-O-C₁₋₃ alkyl, -O-CH₂-Ph, -CH₂-SH and -CH₂-S-C₁₋₃ alkyl.

10. The compound for use according to any one of claims 1 to 9, wherein the compound is an inhibitor of the CIC-1 ion channel.

11. The compound for use according to any one of claims 1 to 10, wherein the compound is for use in the treatment of symptoms of an indication selected from the group consisting of myasthenia gravis (such as autoimmune and congenital myasthenia gravis), Lambert-Eaton Syndrome, critical illness myopathy, amyotrophic lateral sclerosis (ALS), spinal muscular atrophy (SMA), critical illness myopathy (CIM), reversal diabetic polyneuropathy, Guillain-Barré syndrome, poliomyelitis, post-polio syndrome, chronic fatigue syndrome, and critical illness polyneuropathy.

12. The compound for use according to any one of claims 1 to 10, wherein the neuromuscular disorder has been induced by a neuromuscular blocking agent.

13. A compound of Formula (I): 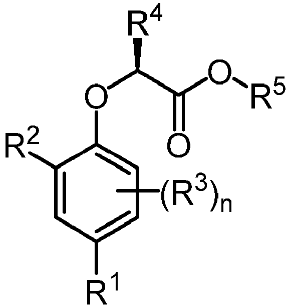 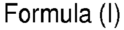 wherein:
- R¹ is selected from the group consisting of F, Cl, Br and I;
- R² is selected from the group consisting of C₂₋₆ alkyl, C₂₋₆ alkenyl, C₂₋₆ alkynyl, C₃₋₅ cycloalkyl, C₅ cycloalkenyl, -C(=O)-C₁₋₅ alkyl, -C(=O)-C₁₋₅ alkenyl, - C(=O)-C₁₋₅ alkynyl, -C(=O)-C₃₋₅ cycloalkyl and -C(=O)-C₅ cycloalkenyl, each of which may be optionally substituted with one or more, identical or different, substituents R⁶;
- R³ is selected from the group consisting of hydrogen, deuterium, tritium, F, Cl, Br, I, -CN, -CF₃, -CCl₃, -CHF₂, -CHCl₂, -CH₂F, -CH₂Cl, -OCF₃, -OCCl₃ and isocyanide;
- R⁴ is selected from the group consisting of C₁₋₅ alkyl, C₁₋₅ alkenyl, C₁₋₅ alkynyl, C₃₋₅cycloalkyl, C₅ cycloalkenyl, each of which may be optionally substituted with one or more, identical or different, substituents R⁷;
- R⁵ is selected from the group consisting of H, C₁₋₅ alkyl optionally substituted with one or more, identical or different, substituents R¹¹, C₂₋₅ alkenyl, C₂₋₅ alkynyl, C₃₋₆ cycloalkyl optionally substituted with one or more, identical or different, substituents R¹¹, phenyl optionally substituted with one or more, identical or different, substituents R¹² and benzyl optionally substituted with one or more, identical or different, substituents R¹²;
- R⁶ is independently selected from the group consisting of hydrogen, deuterium, tritium, F, Cl, Br, I, -CN, isocyanide, -O-C₁₋₃ alkyl, -S-C₁₋₃ alkyl,
- CH₂-OH, -CH₂-O-C₁₋₃ alkyl, -O-CH₂-Ph, -CH₂-SH and -CH₂-S-C₁₋₃ alkyl;
- R⁷ is independently selected from the group consisting of deuterium, tritium, F, Cl, Br, I, -CN, isocyanide, -O-C₁₋₃ alkyl, -S-C₁₋₃ alkyl, -CH₂-O-C₁₋₃ alkyl and -CH₂-S-C₁₋₃ alkyl;
- R¹¹ is independently selected from the group consisting of deuterium and F;
- R¹² is independently selected from the group consisting of deuterium, methoxy, nitro, cyano, Cl, Br, I and F; and
- n is an integer 0, 1, 2 or 3;
or a pharmaceutically acceptable salt, hydrate, polymorph, tautomer, or solvate thereof, with the proviso that when R² is C(=O)-CH₃, R¹ is Br and R⁵ is H or Me then R⁴ is not Me or CH₂CHMe₂; and with the proviso that when R² is CHMe₂, R¹ is Br and R⁵ is H or Me then R⁴ is not Me.

14. The compound according to claim 13, wherein:
- R¹ is selected from the group consisting of F, Cl, Br and I;
- R² is selected from the group consisting of ethyl, vinyl, ethynyl, cyclopropyl, cyclobutyl, -C(=O)-methyl and -C(=O)-ethyl, each of which may be optionally substituted with one or more, identical or different, substituents R⁶;
- R³ is selected from the group consisting of deuterium, tritium, F, Cl, Br and I;
- R⁴ is selected from the group consisting of C₁₋₅ alkyl, C₁₋₅ alkenyl, C₁₋₅ alkynyl, C₃₋₅ cycloalkyl, C₅ cycloalkenyl, each of which may be optionally substituted with one or more, identical or different, substituents R⁷;
- R⁵ is selected from the group consisting of H, C₁₋₅ alkyl optionally substituted with one or more, identical or different, substituents R¹¹, C₂₋₅ alkenyl, C₂₋₅ alkynyl, C₃₋₆ cycloalkyl optionally substituted with one or more, identical or different, substituents R¹¹, phenyl optionally substituted with one or more, identical or different, substituents R¹² and benzyl optionally substituted with one or more, identical or different, substituents R¹²;
- R⁶ is independently selected from the group consisting of deuterium, tritium, F, Cl, Br, I, -CN, isocyanide, -O-C₁₋₃ alkyl, -S-C₁₋₃ alkyl, -CH₂-OH, -CH₂-O-C₁₋₃ alkyl, -O-CH₂-Ph, -CH₂-SH and -CH₂-S-C₁₋₃ alkyl;
- R⁷ is independently selected from the group consisting of deuterium, tritium, F, Cl, Br and I;
- R¹¹ is independently selected from the group consisting of deuterium and F;
- R¹² is independently selected from the group consisting of deuterium, methoxy, nitro, cyano, Cl, Br, I and F; and
- n is an integer 0 or 1.

15. The compound according to any one of claims 13 to 14, wherein the compound is selected from the group consisting of:
(2*S*)-2-{4-bromo-2-[2-(methoxymethyl)cyclopropyl]phenoxy}propanoic acid;
(2*S*)-2-[4-bromo-2-(2,2-dichlorocyclopropyl)phenoxy]propanoic acid;
(2*S*)-2-{4-bromo-2-[(1s,3s)-3-methoxycyclobutyl]phenoxy}propanoic acid;
(2S)-2-{4-bromo-2-[(E)-2-bromoethenyl]phenoxy}propanoic acid;
(2*R*)-2-(4-bromo-2-cyclobutylphenoxy)-3-fluoropropanoic acid;
(2*S*)-2-{4-bromo-2-[(1*S*,2*S*)-2-(hydroxymethyl)cyclopropyl]phenoxy}propanoic acid;
(2*S*)-2-{4-bromo-2-[(1*R*,2*R*)-2-(hydroxymethyL)cyc!LpropyL]phenoxy}propanoic acid;
(2S)-2-(4-bromo-2-ethynylphenoxy)propanoic acid;
(2*S*)-2-{4-bromo-2-[(1*E*)-2-cyanoeth-1-en-1-yl]phenoxy}propanoic acid;
(2S)-2-(4-bromo-2-cyclopropylphenoxy)propanoic acid;
(2S)-2-(4-bromo-2-ethenylphenoxy)propanoic acid;
(2S)-2-(2-cyclopropyl-4-fluorophenoxy)propanoic acid;
(2S)-2-(2-cyclobutyl-4-fluorophenoxy)propanoic acid;
(2S)-2-(4-bromo-2-cyclobutylphenoxy)propanoic acid;
(2*S*)-2-(4-chloro-2-cyclobutylphenoxy)propanoic acid;
tert-butyl (2S)-2-(4-chloro-2-propanoylphenoxy)propanoate;
(2*S*)-2-{4-chloro-2-[(2,2-²H₂)propanoyl]phenoxy}propanoic acid;
(2S)-2-(4-bromo-2-propanoylphenoxy)-3-methylbutanoic acid;
methyl (2*S*)-2-[4-chloro-2-(cyclopent-1-en-1-yl)phenoxy]propanoate;
methyl (2S)-2-(4-bromo-2-propanoylphenoxy)-3-methylbutanoate;
(2S)-2-(4-chloro-2-ethynylphenoxy)propanoic acid;
(2S)-2-(4-chloro-2-propanoylphenoxy)propanoic acid;
sodium (2S)-2-(4-chloro-2-ethenylphenoxy)propanoate;
(2S)-2-(4-chloro-2-cyclopropylphenoxy)propanoic acid;
sodium (2S)-2-(4-chloro-2-propylphenoxy)propanoic acid;
sodium (2S)-2-(4-chloro-2-ethylphenoxy)propanoate;
methyl (2S)-2-(4-chloro-2-ethylphenoxy)propanoate;
(2*R*)-2-(4-chloro-2-cyclopropyl-6-fluorophenoxy)-3-fluoropropanoic acid;
(2*S*)-2-(2-cyclopropyl-4,6-difluorophenoxy)propanoic acid;
(2S)-2-(4-bromo-2-propanoylphenoxy)propanoic acid;
(2*S*)-2-(4-chloro-2-cyclopropyl-6-fluorophenoxy)propanoic acid;
(2S)-2-(2,4-difluoro-6-propanoylphenoxy)propanoic acid;
(2S)-2-(2-acetyl-4-chlorophenoxy)propanoic acid;
(2S)-2-(4-fluoro-2-propanoylphenoxy)propanoic acid;
(2*S*)-2-[4-bromo-2-(cyclopent-1-en-1-yl)phenoxy]propanoic acid;
(2S)-2-[4-bromo-2-(2,2-difluoroethenyl)phenoxy]propanoic acid;
(2*S*)-2-{2-[2-(benzyloxy)cyclobutyl]-4-chlorophenoxy}propanoic acid;
(2*S*)-2-[4-bromo-2-(cyclopent-1-en-1-yl)phenoxy]propanoic acid;
(2S)-2-[4-bromo-2-(2-methoxyethyl)phenoxy]propanoic acid;
(2S)-2-[2,4-dibromo-6-(2-methoxyethyl)phenoxy]propanoic acid;
(2*S*)-2-[4-bromo-2-(cyclopropylidenemethyl)phenoxy]propanoic acid;
(2*S*)-2-(4-bromo-2-ethenyl-5-fluorophenoxy)propanoic acid;
(2*S*)-2-(2-acetyl-4-bromo-5-fluorophenoxy)propanoic acid;
(2*S*)-2-(4-bromo-2-cyclopropyl-5-fluorophenoxy)propanoic acid;
(2*S*)-2-[4-bromo-2-(2,2-difluoroethenyl)-5-fluorophenoxy]propanoic acid;
(2*S*)-2-(4-bromo-2-ethynylphenoxy)-2-cyclobutylacetic acid;
(2*S*)-2-(4-bromo-2-ethynyl-5-fluorophenoxy)propanoic acid;
(2*S*)-2-(4-chloro-2-ethynyl-5-fluorophenoxy)propanoic acid;
(2*S*)-2-(4-bromo-2-cyclopropylphenoxy)-4-fluorobutanoic acid;
(2*S*)-2-(4-bromo-2-ethynylphenoxy)-4-fluorobutanoic acid;
(2*S*)-2-(4-bromo-2-ethenylphenoxy)-4-fluorobutanoic acid;
(2*S*)-2-{4-bromo-2-[(1*E*)-2-fluoroethenyl]phenoxy}propanoic acid;
(2*S*)-2-{4-chloro-2-[(1*E*)2-fluoroethenyl]phenoxy}propanoic acid;
(2*S*)-2-(4-bromo-2-ethynylphenoxy)butanoic acid; and
(2*S*)-2-[4-bromo-2-(2,2-difluorocyclobutyl)phenoxy]propanoic acid.

## Patentansprüche

1. Verbindung der Formel (I): 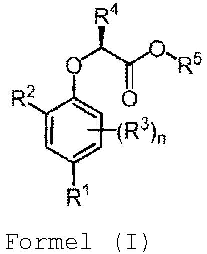 wobei:
- R¹ aus der Gruppe bestehend aus F, Cl, Br, I, -CN, -CF₃, - CCl₃, -CHF₂, -CHCl₂, -CH₂F, -CH₂Cl, -OCF₃ und -OCCl₃ ausgewählt ist;
- R² aus der Gruppe bestehend aus C₂₋₆-Alkyl, C₂₋₆-Alkenyl, C₂₋₆-Alkinyl, C₃₋₆-Cycloalkyl, C₅₋₆-Cycloalkenyl, -C (=O)-C₁₋₅-Alkyl,
- C (=O)-C₁₋₅-Alkenyl, -C (=O)-C₁₋₅-Alkinyl, -C (=O)-C₃₋₅-Cycloalkyl und -C(=O)-C₅-Cycloalkenyl ausgewählt ist, die jeweils gegebenenfalls mit einem oder mehreren gleichen oder verschiedenen Substituenten R⁶ substituiert sein können;
- R³ aus der Gruppe bestehend aus Wasserstoff, Deuterium, Tritium, F, Cl, Br, I, -CN, -CF₃, -CCl₃, -CHF₂, -CHCl₂, -CH₂F, - CH₂Cl, -OCF₃, -OCCl₃ und Isocyanid ausgewählt ist;
- R⁴ aus der Gruppe bestehend aus C₁₋₅-Alkyl, C₁₋₅-Alkenyl, C₁₋₅-Alkinyl, C₃₋₅-Cycloalkyl, C₅-Cycloalkenyl ausgewählt ist, die jeweils gegebenenfalls mit einem oder mehreren gleichen oder verschiedenen Substituenten R⁷ substituiert sein können;
- R⁵ aus der Gruppe bestehend aus H, C₁₋₅-Alkyl, gegebenenfalls substituiert mit einem oder mehreren gleichen oder verschiedenen Substituenten R¹¹, C₂₋₅-Alkenyl, C₂₋₅-Alkinyl, C₃₋₆-Cycloalkyl, gegebenenfalls substituiert mit einem oder mehreren gleichen oder verschiedenen Substituenten R¹¹, Phenyl, gegebenenfalls substituiert mit einem oder mehreren gleichen oder verschiedenen Substituenten R¹², und Benzyl, gegebenenfalls substituiert mit einem oder mehreren gleichen oder verschiedenen Substituenten R¹², ausgewählt ist;
- R⁶ unabhängig aus der Gruppe bestehend aus Wasserstoff, Deuterium, Tritium, F, Cl, Br, I, -CN, Isocyanid, -O-C₁₋₃-Alkyl,
- S-C₁₋₃-Alkyl, -CH₂-OH, -CH₂-O-C₁₋₃-Alkyl, -O-CH₂-Ph, -CH₂-SH und
- CH₂-S-C₁₋₃-Alkyl ausgewählt ist;
- R⁷ unabhängig aus der Gruppe bestehend aus Deuterium, Tritium, F, Cl, Br, I, -CN, Isocyanid, -O-C₁₋₃-Alkyl,-S-C₁₋₃-Alkyl, -CH₂-O-C₁₋₃-Alkyl und -CH₂-S-C₁₋₃-Alkyl ausgewählt ist;
- R¹¹ unabhängig aus der Gruppe bestehend aus Deuterium und F ausgewählt ist;
- R¹² unabhängig aus der Gruppe bestehend aus Deuterium, Methoxy, Nitro, Cyano, Cl, Br, I und F ausgewählt ist; und
- n eine ganze Zahl 0, 1, 2 oder 3 ist;
oder ein pharmazeutisch verträgliches Salz, Hydrat, Polymorph, Tautomer oder Solvat davon,
zur Verwendung bei der Behandlung, Linderung und/oder Vorbeugung einer neuromuskulären Erkrankung und/oder zur Verwendung bei der Umkehrung und/oder Linderung einer neuromuskulären Blockade.

2. Verbindung zur Verwendung nach Anspruch 1, wobei:
- R¹ aus der Gruppe bestehend aus F, Cl, Br und I ausgewählt ist;
- R² aus der Gruppe bestehend aus Ethyl, Vinyl, Ethinyl, Cyclopropyl, Cyclobutyl, -C(=O)-Methyl und -C(=O)-Ethyl ausgewählt ist, die jeweils gegebenenfalls mit einem oder mehreren gleichen oder verschiedenen Substituenten R⁶ substituiert sein können;
- R³ aus der Gruppe bestehend aus Deuterium, Tritium, F, Cl, Br und I ausgewählt ist;
- R⁴ aus der Gruppe bestehend aus C₁₋₅-Alkyl, C₁₋₅-Alkenyl, C₁₋₅-Alkinyl, C₃₋₅-Cycloalkyl, C₅-Cycloalkenyl ausgewählt ist, die jeweils gegebenenfalls mit einem oder mehreren gleichen oder verschiedenen Substituenten R⁷ substituiert sein können;
- R⁵ aus der Gruppe bestehend aus H, C₁₋₅-Alkyl, gegebenenfalls substituiert mit einem oder mehreren gleichen oder verschiedenen Substituenten R¹¹, C₂₋₅-Alkenyl, C₂₋₅-Alkinyl, C₃₋₆-Cycloalkyl, gegebenenfalls substituiert mit einem oder mehreren gleichen oder verschiedenen Substituenten R¹¹, Phenyl, gegebenenfalls substituiert mit einem oder mehreren gleichen oder verschiedenen Substituenten R¹², und Benzyl, gegebenenfalls substituiert mit einem oder mehreren gleichen oder verschiedenen Substituenten R¹², ausgewählt ist;
- R⁶ unabhängig aus der Gruppe bestehend aus Deuterium, Tritium, F, Cl, Br, I, -CN, Isocyanid, -O-C₁₋₃-Alkyl,-S-C₁₋₃-Alkyl, -CH₂-OH, -CH₂-O-C₁₋₃-Alkyl, -O-CH₂-Ph, -CH₂-SH und -CH₂-S-C₁₋₃-Alkyl ausgewählt ist;
- R⁷ unabhängig aus der Gruppe bestehend aus Deuterium, Tritium, F, Cl, Br und I ausgewählt ist;
- R¹¹ unabhängig aus der Gruppe bestehend aus Deuterium und F ausgewählt ist;
- R¹² unabhängig aus der Gruppe bestehend aus Deuterium, Methoxy, Nitro, Cyano, Cl, Br, I und F ausgewählt ist; und
- n eine ganze Zahl 0 oder 1 ist.

3. Verbindung zur Verwendung nach einem der Ansprüche 1 bis 2, wobei die Verbindung die Formel (II) hat: 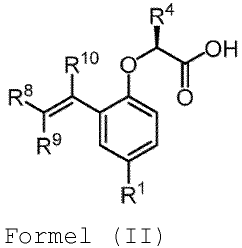 wobei:
R¹ aus der Gruppe bestehend aus F, Cl, Br, I, -CN, -CF₃, - CCL₃, -CHF₂, -CHCl₂, -CH₂F, -CH₂Cl, -OCF₃ und -OCCl₃ ausgewählt ist;
R⁴ Methyl, Ethyl, n-Propyl, Isopropyl oder -CH₂F ist;
R⁸ und R⁹ unabhängig aus der Gruppe bestehend aus Wasserstoff, Deuterium, Tritium, F, Cl, Br, I, -CN, Isocyanid, -O-C₁₋₃-Alkyl, -S-C₁₋₃-Alkyl, -CH₂-OH, -CH₂-O-C₁₋₃-Alkyl, -CH₂-SH, -CH₂-S-C₁₋₃-Alkyl, C₁₋₄-Alkyl und C₁₋₄-Alkenyl ausgewählt sind und wobei die C₁₋₄-Alkyl- und die C₁₋₄-Alkenylgruppe gegebenenfalls mit einem oder mehreren gleichen oder verschiedenen Substituenten R⁶ substituiert sein können; und
R⁶ unabhängig aus der Gruppe bestehend aus Wasserstoff, Deuterium, Tritium, F, Cl, Br, I, -CN, Isocyanid, -O-C₁₋₃-Alkyl, -S-C₁₋₃-Alkyl, -CH₂-OH, -CH₂-O-C₁₋₃-Alkyl, -O-CH₂-Ph, -CH₂-SH und -CH₂-S-C₁₋₃-Alkyl ausgewählt ist.

4. Verbindung zur Verwendung nach einem der Ansprüche 1 bis 2, wobei die Verbindung die Formel (III) hat: 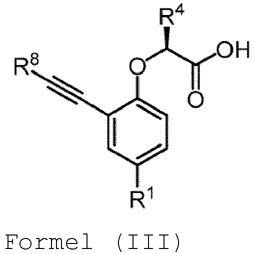 wobei:
R¹ aus der Gruppe bestehend aus F, Cl, Br, I, -CN, -CF₃, - CCL₃, -CHF₂, -CHCl₂, -CH₂F, -CH₂Cl, -OCF₃ und -OCCl₃ ausgewählt ist;
R⁴ Methyl, Ethyl, n-Propyl, Isopropyl oder -CH₂F ist;
R⁸ unabhängig aus der Gruppe bestehend aus Wasserstoff, Deuterium, Tritium, F, Cl, Br, I, -CN, Isocyanid, -O-C₁₋₃-Alkyl, -S-C₁₋₃-Alkyl, -CH₂-OH, -CH₂-O-C₁₋₃-Alkyl, -CH₂-SH, -CH₂-S-C₁₋₃-Alkyl, C₁₋₄-Alkyl und C₁₋₄-Alkenyl ausgewählt ist und wobei die C₁₋₄-Alkyl- und die C₁₋₄-Alkenylgruppe gegebenenfalls mit einem oder mehreren gleichen oder verschiedenen Substituenten R⁶ substituiert sein können; und
R⁶ unabhängig aus der Gruppe bestehend aus Wasserstoff, Deuterium, Tritium, F, Cl, Br, I, -CN, Isocyanid, -O-C₁₋₃-Alkyl, -S-C₁₋₃-Alkyl, -CH₂-OH, -CH₂-O-C₁₋₃-Alkyl, -O-CH₂-Ph, -CH₂-SH und -CH₂-S-C₁₋₃-Alkyl ausgewählt ist.

5. Verbindung zur Verwendung nach einem der Ansprüche 1 bis 2, wobei die Verbindung die Formel (IV) hat: 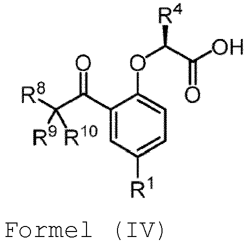 wobei:
R¹ aus der Gruppe bestehend aus F, Cl, Br, I, -CN, -CF₃, - CCL₃, -CHF₂, -CHCl₂, -CH₂F, -CH₂Cl, -OCF₃ und -OCCl₃ ausgewählt ist;
R⁴ Methyl, Ethyl, n-Propyl, Isopropyl oder -CH₂F ist;
R⁶ unabhängig aus der Gruppe bestehend aus Wasserstoff, Deuterium, Tritium, F, Cl, Br, I, -CN, Isocyanid, -O-C₁₋₃-Alkyl, -S-C₁₋₃-Alkyl, -CH₂-OH, -CH₂-O-C₁₋₃-Alkyl, -O-CH₂-Ph, -CH₂-SH und -CH₂-S-C₁₋₃-Alkyl ausgewählt ist; und
R⁸, R⁹ und R¹⁰ unabhängig aus der Gruppe bestehend aus Wasserstoff, Deuterium, Tritium, F, Cl, Br, I, -CN, Isocyanid, -O-C₁₋₃-Alkyl, -S-C₁₋₃-Alkyl, -CH₂-OH, -CH₂-O-C₁₋₃-Alkyl, -CH₂-SH, -CH₂-S-C₁₋₃-Alkyl, C₁₋₄-Alkyl und C₁₋₄-Alkenyl ausgewählt sind und wobei die C₁₋₄-Alkyl- und die C₁₋₄-Alkenylgruppe gegebenenfalls mit einem oder mehreren gleichen oder verschiedenen Substituenten R⁶ substituiert sein können.

6. Verbindung zur Verwendung nach einem der Ansprüche 1 bis 2, wobei die Verbindung die Formel (V) hat: 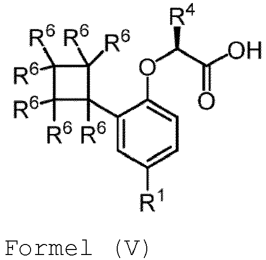 wobei:
R¹ aus der Gruppe bestehend aus F, Cl, Br, I, -CN, -CF₃, - CCL₃, -CHF₂, -CHCl₂, -CH₂F, -CH₂Cl, -OCF₃ und -OCCl₃ ausgewählt ist;
R⁴ Methyl, Ethyl, n-Propyl, Isopropyl oder -CH₂F ist; und
R⁶ unabhängig aus der Gruppe bestehend aus Wasserstoff, Deuterium, Tritium, F, Cl, Br, I, -CN, Isocyanid, -O-C₁₋₃-Alkyl, -S-C₁₋₃-Alkyl, -CH₂-OH, -CH₂-O-C₁₋₃-Alkyl, -O-CH₂-Ph, -CH₂-SH und -CH₂-S-C₁₋₃-Alkyl ausgewählt ist.

7. Verbindung zur Verwendung nach einem der Ansprüche 1 bis 2, wobei die Verbindung die Formel (VI) hat: 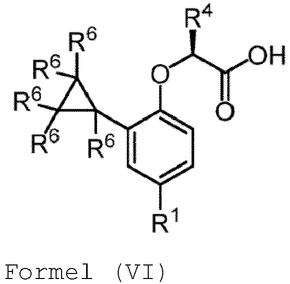 wobei:
R¹ aus der Gruppe bestehend aus F, Cl, Br, I, -CN, -CF₃, - CCL₃, -CHF₂, -CHCl₂, -CH₂F, -CH₂Cl, -OCF₃ und -OCCl₃ ausgewählt ist;
R⁴ Methyl, Ethyl, n-Propyl, Isopropyl oder -CH₂F ist; und
R⁶ unabhängig aus der Gruppe bestehend aus Wasserstoff, Deuterium, Tritium, F, Cl, Br, I, -CN, Isocyanid, -O-C₁₋₃-Alkyl, -S-C₁₋₃-Alkyl, -CH₂-OH, -CH₂-O-C₁₋₃-Alkyl, -O-CH₂-Ph, -CH₂-SH und -CH₂-S-C₁₋₃-Alkyl ausgewählt ist.

8. Verbindung zur Verwendung nach einem der Ansprüche 1 bis 2, wobei die Verbindung die Formel (VII) hat: 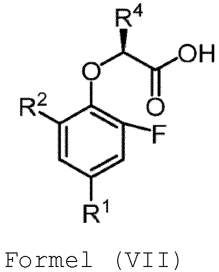 wobei:
R¹ aus der Gruppe bestehend aus F, Cl, Br, I, -CN, -CF₃, - CCL₃, -CHF₂, -CHCl₂, -CH₂F, -CH₂Cl, -OCF₃ und -OCCl₃ ausgewählt ist;
R² aus der Gruppe bestehend aus C₂₋₆-Alkyl, C₂₋₆-Alkenyl, C₂₋₆-Alkinyl, C₃₋₆-Cycloalkyl, C₅₋₆-Cycloalkenyl, -C(=O)-C₁₋₅-Alkyl, -C(=O)-C₁₋₅-Alkenyl, -C(=O)-C₁₋₅-Alkinyl, -C(=O)-C₃₋₅-Cycloalkyl und -C(=O)-C₅-Cycloalkenyl ausgewählt ist, die jeweils gegebenenfalls mit einem oder mehreren gleichen oder verschiedenen Substituenten R⁶ substituiert sein können;
R⁴ aus der Gruppe bestehend aus C₁₋₅-Alkyl, C₁₋₅-Alkenyl, C₁₋₅-Alkinyl, C₃₋₅-Cycloalkyl, C₅-Cycloalkenyl ausgewählt ist, die jeweils gegebenenfalls mit einem oder mehreren gleichen oder verschiedenen Substituenten R⁷ substituiert sein können;
R⁶ unabhängig aus der Gruppe bestehend aus Wasserstoff, Deuterium, Tritium, F, Cl, Br, I, -CN, Isocyanid, -O-C₁₋₃-Alkyl, -S-C₁₋₃-Alkyl, -CH₂-OH, -CH₂-O-C₁₋₃-Alkyl, -O-CH₂-Ph, -CH₂-SH und -CH₂-S-C₁₋₃-Alkyl ausgewählt ist; und
R⁷ unabhängig aus der Gruppe bestehend aus Deuterium, Tritium, F, Cl, Br, I, -CN, Isocyanid, -O-C₁₋₃-Alkyl,-S-C₁₋₃-Alkyl, -CH₂-O-C₁₋₃-Alkyl und -CH₂-S-C₁₋₃-Alkyl ausgewählt ist.

9. Verbindung zur Verwendung nach einem der Ansprüche 1 bis 2, wobei die Verbindung die Formel (VIII) hat: 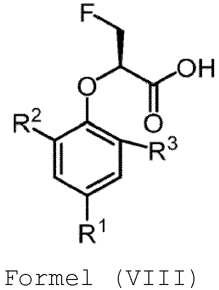 wobei:
R¹ aus der Gruppe bestehend aus F, Cl, Br, I, -CN, -CF₃, - CCL₃, -CHF₂, -CHCl₂, -CH₂F, -CH₂Cl, -OCF₃ und -OCCl₃ ausgewählt ist;
R² aus der Gruppe bestehend aus C₂₋₆-Alkyl, C₂₋₆-Alkenyl, C₂₋₆-Alkinyl, C₃₋₆-Cycloalkyl, C₅₋₆-Cycloalkenyl, -C (=O)-C₁₋₅-Alkyl, -C (=O)-C₁₋₅-Alkenyl, -C (=O)-C₁₋₅-Alkinyl, -C (=O)-C₃₋₅-Cycloalkyl und -C(=O)-C₅-Cycloalkenyl ausgewählt ist, die jeweils gegebenenfalls mit einem oder mehreren gleichen oder verschiedenen Substituenten R⁶ substituiert sein können;
R³ aus der Gruppe bestehend aus Wasserstoff, Deuterium, Tritium, F, Cl, Br, I, -CN, -CF₃, -CCl₃, -CHF₂, -CHCl₂, -CH₂F, - CH₂Cl, -OCF₃, -OCCl₃ und Isocyanid ausgewählt ist; und
R⁶ unabhängig aus der Gruppe bestehend aus Wasserstoff, Deuterium, Tritium, F, Cl, Br, I, -CN, Isocyanid, -O-C₁₋₃-Alkyl, -S-C₁₋₃-Alkyl, -CH₂-OH, -CH₂-O-C₁₋₃-Alkyl, -O-CH₂-Ph, -CH₂-SH und -CH₂-S-C₁₋₃-Alkyl ausgewählt ist.

10. Verbindung zur Verwendung nach einem der Ansprüche 1 bis 9, wobei die Verbindung ein Inhibitor des ClC-1-Ionenkanals ist.

11. Verbindung zur Verwendung nach einem der Ansprüche 1 bis 10, wobei die Verbindung zur Verwendung bei der Behandlung von Symptomen einer Indikation vorgesehen ist, die aus der Gruppe bestehend aus Myasthenia gravis (wie autoimmune und kongenitale Myasthenia gravis), Lambert-Eaton-Syndrom, Critical-Illness-Myopathie, amyotropher Lateralsklerose (ALS), spinaler Muskelatrophie (SMA), Critical-Illness-Myopathie (CIM), reversibler diabetischer Polyneuropathie, Guillain-Barré-Syndrom, Poliomyelitis, Post-Polio-Syndrom, chronischem Erschöpfungssyndrom und Critical-Illness-Polyneuropathie ausgewählt ist.

12. Verbindung zur Verwendung nach einem der Ansprüche 1 bis 10, wobei die neuromuskuläre Erkrankung durch einen neuromuskulären Blocker induziert wurde.

13. Verbindung der Formel (I): 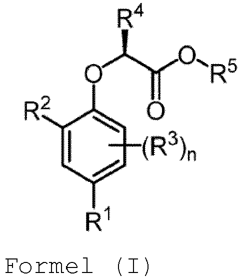 wobei:
- R¹ aus der Gruppe bestehend aus F, Cl, Br und I ausgewählt ist;
- R² aus der Gruppe bestehend aus C₂₋₆-Alkyl, C₂₋₆-Alkenyl, C₂-₆-Alkinyl, C₃₋₅-Cycloalkyl, C₅-Cycloalkenyl, -C(=O)-C₁₋₅-Alkyl, - C(=O)-C₁₋₅-Alkenyl, -C(=O)-C₁₋₅-Alkinyl, -C(=O)-C₃₋₅-Cycloalkyl und -C(=O)-C₅-Cycloalkenyl ausgewählt ist, die jeweils gegebenenfalls mit einem oder mehreren gleichen oder verschiedenen Substituenten R⁶ substituiert sein können;
- R³ aus der Gruppe bestehend aus Wasserstoff, Deuterium, Tritium, F, Cl, Br, I, -CN, -CF₃, -CCl₃, -CHF₂, -CHCl₂, -CH₂F, - CH₂Cl, -OCF₃, -OCCl₃ und Isocyanid ausgewählt ist;
- R⁴ aus der Gruppe bestehend aus C₁₋₅-Alkyl, C₁₋₅-Alkenyl, C₁₋₅-Alkinyl, C₃₋₅-Cycloalkyl, C₅-Cycloalkenyl ausgewählt ist, die jeweils gegebenenfalls mit einem oder mehreren gleichen oder verschiedenen Substituenten R⁷ substituiert sein können;
- R⁵ aus der Gruppe bestehend aus H, C₁₋₅-Alkyl, gegebenenfalls substituiert mit einem oder mehreren gleichen oder verschiedenen Substituenten R¹¹, C₂₋₅-Alkenyl, C₂₋₅-Alkinyl, C₃₋₆-Cycloalkyl, gegebenenfalls substituiert mit einem oder mehreren gleichen oder verschiedenen Substituenten R¹¹, Phenyl, gegebenenfalls substituiert mit einem oder mehreren gleichen oder verschiedenen Substituenten R¹², und Benzyl, gegebenenfalls substituiert mit einem oder mehreren gleichen oder verschiedenen Substituenten R¹², ausgewählt ist;
- R⁶ unabhängig aus der Gruppe bestehend aus Wasserstoff, Deuterium, Tritium, F, Cl, Br, I, -CN, Isocyanid, -O-C₁₋₃-Alkyl,
- S-C₁₋₃-Alkyl, -CH₂-OH, -CH₂-O-C₁₋₃-Alkyl, -O-CH₂-Ph, -CH₂-SH und
- CH₂-S-C₁₋₃-Alkyl ausgewählt ist;
- R⁷ unabhängig aus der Gruppe bestehend aus Deuterium, Tritium, F, Cl, Br, I, -CN, Isocyanid, -O-C₁₋₃-Alkyl,-S-C₁₋₃-Alkyl, -CH₂-O-C₁₋₃-Alkyl und -CH₂-S-C₁₋₃-Alkyl ausgewählt ist;
- R¹¹ unabhängig aus der Gruppe bestehend aus Deuterium und F ausgewählt ist;
- R¹² unabhängig aus der Gruppe bestehend aus Deuterium, Methoxy, Nitro, Cyano, Cl, Br, I und F ausgewählt ist; und
- n eine ganze Zahl 0, 1, 2 oder 3 ist;
oder ein pharmazeutisch verträgliches Salz, Hydrat, Polymorph, Tautomer oder Solvat davon, mit der Maßgabe, dass, wenn R² C(=O)-CH₃ ist, R¹ Br ist und R⁵ H oder Me ist, dann R⁴ nicht Me oder CH₂CHMe₂ ist; und mit der Maßgabe, dass, wenn R² CHMe₂ ist, R¹ Br ist und R⁵ H oder Me ist, dann R⁴ nicht Me ist.

14. Verbindung nach Anspruch 13, wobei:
- R¹ aus der Gruppe bestehend aus F, Cl, Br und I ausgewählt ist;
- R² aus der Gruppe bestehend aus Ethyl, Vinyl, Ethinyl, Cyclopropyl, Cyclobutyl, -C(=O)-Methyl und -C(=O)-Ethyl ausgewählt ist, die jeweils gegebenenfalls mit einem oder mehreren gleichen oder verschiedenen Substituenten R⁶ substituiert sein können;
- R³ aus der Gruppe bestehend aus Deuterium, Tritium, F, Cl, Br und I ausgewählt ist;
- R⁴ aus der Gruppe bestehend aus C₁₋₅-Alkyl, C₁₋₅-Alkenyl, C₁₋₅-Alkinyl, C₃₋₅-Cycloalkyl, C₅-Cycloalkenyl ausgewählt ist, die jeweils gegebenenfalls mit einem oder mehreren gleichen oder verschiedenen Substituenten R⁷ substituiert sein können;
- R⁵ aus der Gruppe bestehend aus H, C₁₋₅-Alkyl, gegebenenfalls substituiert mit einem oder mehreren gleichen oder verschiedenen Substituenten R¹¹, C₂₋₅-Alkenyl, C₂₋₅-Alkinyl, C₃₋₆-Cycloalkyl, gegebenenfalls substituiert mit einem oder mehreren gleichen oder verschiedenen Substituenten R¹¹, Phenyl, gegebenenfalls substituiert mit einem oder mehreren gleichen oder verschiedenen Substituenten R¹², und Benzyl, gegebenenfalls substituiert mit einem oder mehreren gleichen oder verschiedenen Substituenten R¹², ausgewählt ist;
- R⁶ unabhängig aus der Gruppe bestehend aus Deuterium, Tritium, F, Cl, Br, I, -CN, Isocyanid, -O-C₁₋₃-Alkyl,-S-C₁₋₃-Alkyl, -CH₂-OH, -CH₂-O-C₁₋₃-Alkyl, -O-CH₂-Ph, -CH₂-SH und -CH₂-S-C₁₋₃-Alkyl ausgewählt ist;
- R⁷ unabhängig aus der Gruppe bestehend aus Deuterium, Tritium, F, Cl, Br und I ausgewählt ist;
- R¹¹ unabhängig aus der Gruppe bestehend aus Deuterium und F ausgewählt ist;
- R¹² unabhängig aus der Gruppe bestehend aus Deuterium, Methoxy, Nitro, Cyano, Cl, Br, I und F ausgewählt ist; und
- n eine ganze Zahl 0 oder 1 ist.

15. Verbindung nach einem der Ansprüche 13 bis 14, wobei die Verbindung aus der Gruppe bestehend aus Folgendem ausgewählt ist:
[2*S*)-2-{4-Brom-2-[2-(methoxymethyl)cyclopropyl]phenoxy}propansäure;
(2*S*)-2-[4-Brom-2-(2,2-dichlorcyclopropyl)phenoxy]propansäure;
(2*S*)-2-{4-Brom-2-[(1s,3s)-3-methoxycyclobutyl]phenoxy}propansäure;
(2S*)*-2-{4-Brom-2-[(E)-2-bromethenyl]phenoxy}propansäure;
(2R)-2-(4-Brom-2-cyclobutylphenoxy)-3-fluorpropansäure;
(2*S*)*-*2*-*{4-Brom-2-[(1*S,*2*S*)*-*2-(hydroxymethyl)cyclopropyl]phenoxy}propansäure;
(2*S*)-2-{4-Brom-2-[(1*R*,2*R*)-2-(hydroxymethyl)cyclopropyl]phenoxy}propansäure;
(2S)-2-(4-Brom-2-ethinylphenoxy)propansäure;
(2S*)*-2-{4-Brom-2-[(1*E*)-2-cyanoeth-1-en-1-yl]phenoxy}propansäure;
(2S)-2-(4-Brom-2-cyclopropylphenoxy)propansäure;
(2S)-2-(4-Brom-2-ethenylphenoxy)propansäure;
(2S)-2-(2-Cyclopropyl-4-fluorphenoxy)propansäure;
(2S)-2-(2-Cyclobutyl-4-fluorphenoxy)propansäure;
(2S)-2-(4-Brom-2-cyclobutylphenoxy)propansäure;
(2S)-2-(4-Chlor-2-cyclobutylphenoxy)propansäure; tert-Butyl-(2S)-2-(4-chlor-2-propanoylphenoxy)propanoat;
(2*S*)-2-{4-Chlor-2-[(2,2-²H₂)propanoyl]phenoxy}propansäure;
(2S)-2-(4-Brom-2-propanoylphenoxy)-3-methylbutansäure; Methyl-(2*S*)-2-[4-chlor-2-(cyclopent-1-en-1-yl)phenoxy]propanoat; Methyl-(2S)-2-(4-brom-2-propanoylphenoxy)-3-methylbutanoat;
(2S)-2-(4-Chlor-2-ethinylphenoxy)propansäure;
(2S)-2-(4-Chlor-2-propanoylphenoxy)propansäure; Natrium-(2S)-2-(4-chlor-2-ethenylphenoxy)propanoat;
(2S)-2-(4-Chlor-2-cyclopropylphenoxy)propansäure; Natrium-(2S)-2-(4-chlor-2-propylphenoxy)propansäure; Natrium-(2S)-2-(4-chlor-2-ethylphenoxy)propanoat;
Methyl-(2S)-2-(4-chlor-2-ethylphenoxy)propanoat;
(2R)-2-(4-Chlor-2-cyclopropyl-6-fluorphenoxy)-3-fluorpropansäure;
(2S)-2-(2-Cyclopropyl-4,6-difluorphenoxy)propansäure;
(2S)-2-(4-Brom-2-propanoylphenoxy)propansäure;
(2S)-2-(4-Chlor-2-cyclopropyl-6-fluorphenoxy)propansäure;
(2S)-2-(2,4-Difluor-6-propanoylphenoxy)propansäure;
(2S)-2-(2-Acetyl-4-chlorphenoxy)propansäure;
(2*S*)-2-(4-Fluor-2-propanoylphenoxy)propansäure;
(2*S*)-2-[4-Brom-2-(cyclopent-1-en-1-yl)phenoxy]propansäure;
(2S)-2-[4-Brom-2-(2,2-difluorethenyl)phenoxy]propansäure;
(2*S*)-2-{2-[2-(Benzyloxy)cyclobutyl]-4-chlorphenoxy}propansäure;
(2*S*)-2-[4-Brom-2-(cyclopent-1-en-1-yl)phenoxy]propansäure;
(2*S*)-2-[4-Brom-2-(2-methoxyethyl)phenoxy]propansäure;
(2S)-2-[2,4-Dibrom-6-(2-methoxyethyl)phenoxy]propansäure;
(2*S*)-2-[4-Brom-2-(cyclopropylidenmethyl)phenoxy]propansäure;
(2S)-2-(4-Brom-2-ethenyl-5-fluorphenoxy)propansäure;
(2S)-2-(2-Acetyl-4-brom-5-fluorphenoxy)propansäure;
(2S)-2-(4-Brom-2-cyclopropyl-5-fluorphenoxy)propansäure;
(2S)-2-[4-Brom-2-(2,2-difluorethenyl)-5-fluorphenoxy]propansäure;
(2S)-2-(4-Brom-2-ethinylphenoxy)-2-cyclobutylessigsäure;
(2S)-2-(4-Brom-2-ethinyl-5-fluorphenoxy)propansäure;
(2S)-2-(4-Chlor-2-ethinyl-5-fluorphenoxy)propansäure;
(2S)-2-(4-Brom-2-cyclopropylphenoxy)-4-fluorbutansäure;
(2S)-2-(4-Brom-2-ethinylphenoxy)-4-fluorbutansäure;
(2S)-2-(4-Brom-2-ethenylphenoxy)-4-fluorbutansäure;
(2*S*)-2-{4-Brom-2-[(1*E*)-2-fluorethenyl]phenoxy}propansäure;
(2*S*)-2-{4-Chlor-2-[(1*E*)-2-fluorethenyl]phenoxy}propansäure;
(2S)-2-(4-Brom-2-ethinylphenoxy)butansäure; und
(2S)-2-[4-Brom-2-(2,2-Difluorcyclobutyl)phenoxy]propansäure.

## Revendications

1. Composé de formule (I) : 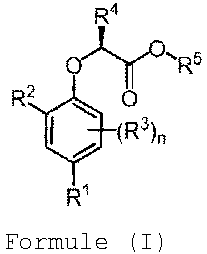 dans lequel :
- R¹ est choisi dans le groupe constitué de : F, Cl, Br, I,
- CN, -CF₃, -CCl₃, -CHF₂, -CHCl₂, -CH₂F, -CH₂Cl, -OCF₃ et -OCCl₃ ;
- R² est choisi dans le groupe constitué de : alkyle en C₂₋₆, alcényle en C₂₋₆, alcynyle en C₂₋₆, cycloalkyle en C₃₋₆, cycloalcényle en C₅₋₆, -C(=O)-alkyle en-C₁₋₅, -C(=O)-alcényle en C₁₋₅, -C(=O)-alcynyle en C₁₋₅, -C(=O)-cyclcoalkyle en C₃₋₅ et - C(=O)-cycloalcényle en C₅, chacun pouvant être éventuellement substitué par un ou plusieurs substituants R⁶ identiques ou différents ;
- R³ est choisi dans le groupe constitué de : hydrogène, deutérium, tritium, F, Cl, Br, I, -CN, -CF₃, -CCl₃, -CHF₂, - CHCl₂, -CH₂F, -CH₂Cl, -OCF₃, -OCCl₃ et isocyanure ;
- R⁴ est choisi dans le groupe constitué de : alkyle en C₁₋₅, alcényle en C₁₋₅, alcynyle en C₁₋₅, cycloalkyle en C₃₋₅, cycloalcényle en C₅, chacun pouvant être éventuellement substitué par un ou plusieurs substituants R⁷ identiques ou différents ;
- R⁵ est choisi dans le groupe constitué de : H, alkyle en C₁₋₅ éventuellement substitué par un ou plusieurs substituants R¹¹ identiques ou différents, alcényle en C₂₋₅, alcynyle en C₂₋₅, cycloalkyle en C₃₋₆ éventuellement substitué par un ou plusieurs substituants R¹¹ identiques ou différents, phényle éventuellement substitué par un ou plusieurs substituants R¹² identiques ou différents et benzyle éventuellement substitué par un ou plusieurs substituants R¹² identiques ou différents ;
- R⁶ est indépendamment choisi dans le groupe constitué de : hydrogène, deutérium, tritium, F, Cl, Br, I, -CN, isocyanure, - O-alkyle en C₁₋₃, -S-alkyle en C₁₋₃, -CH₂-OH, -CH₂-O-alkyle en C₁₋₃, -O-CH₂-Ph, -CH₂-SH et -CH₂-S-alkyle en C₁₋₃ ;
- R⁷ est indépendamment choisi dans le groupe constitué de : deutérium, tritium, F, Cl, Br, I, -CN, isocyanure, -O-alkyle en C₁₋₃, -S-alkyle en C₁₋₃, -CH₂-O-alkyle en C₁₋₃ et -CH₂-S-alkyle en C₁₋₃ ;
- R¹¹ est indépendamment choisi dans le groupe constitué de : deutérium et F ;
- R¹² est indépendamment choisi dans le groupe constitué de : deutérium, méthoxy, nitro, cyano, Cl, Br, I et F ; et
- n représente un nombre entier 0, 1, 2 ou 3 ;
ou un sel, un hydrate, un polymorphe, un tautomère, ou un solvate pharmaceutiquement acceptable de celui-ci,
pour une utilisation dans le traitement, l'amélioration et/ou la prévention d'un trouble neuromusculaire et/ou pour une utilisation dans l'inversion et/ou l'amélioration d'un blocage neuromusculaire.

2. Composé pour une utilisation selon la revendication 1, dans lequel :
- R¹ est choisi dans le groupe constitué de : F, Cl, Br et I ;
- R² est choisi dans le groupe constitué d'éthyle, vinyle, éthynyle, cyclopropyle, cyclobutyle, -C(=O-méthyle et -C(=O)-éthyle, chacun pouvant être éventuellement substitué par un ou plusieurs substituants R⁶ identiques ou différents ;
- R³ est choisi dans le groupe constitué de : deutérium, tritium, F, Cl, Br et I ;
- R⁴ est choisi dans le groupe constitué de : alkyle en C₁₋₅, alcényle en C₁₋₅, alcynyle en C₁₋₅, cycloalkyle en C₃₋₅, cycloalcényle en C₅, chacun pouvant être éventuellement substitué par un ou plusieurs substituants R⁷ identiques ou différents ;
- R⁵ est choisi dans le groupe constitué de : H, alkyle en C₁₋₅ éventuellement substitué par un ou plusieurs substituants R¹¹ identiques ou différents, alcényle en C₂₋₅, alcynyle en C₂₋₅, cycloalkyle en C₃₋₆ éventuellement substitué par un ou plusieurs substituants R¹¹ identiques ou différents, phényle éventuellement substitué par un ou plusieurs substituants R¹² identiques ou différents et benzyle éventuellement substitué par un ou plusieurs substituants R¹² identiques ou différents ;
- R⁶ est indépendamment choisi dans le groupe constitué de : deutérium, tritium, F, Cl, Br, I, -CN, isocyanure, -O-alkyle en C₁₋₃, -S-alkyle en C₁₋₃, -CH₂-OH, -CH₂-O-alkyle en C₁₋₃, -O-CH₂-Ph, -CH₂-SH et -CH₂-S-alkyle en C₁₋₃ ;
- R⁷ est indépendamment choisi dans le groupe constitué de : deutérium, tritium, F, Cl, Br et I ;
- R¹¹ est indépendamment choisi dans le groupe constitué de : deutérium et F ;
- R¹² est indépendamment choisi dans le groupe constitué de : deutérium, méthoxy, nitro, cyano, Cl, Br, I et F ; et
- n représente un nombre entier 0 ou 1.

3. Composé pour une utilisation selon l'une quelconque des revendications 1 à 2, le composé répondant à la Formule (II) : 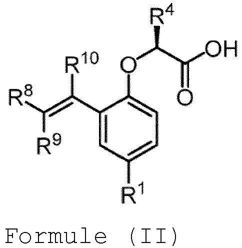 dans lequel :
R¹ est choisi dans le groupe constitué de : F, Cl, Br, I, -CN, -CF₃, -CCl₃, -CHF₂, -CHCl₂, -CH₂F, -CH₂Cl, -OCF₃ et -OCCl₃ ;
R⁴ représente un méthyle, un éthyle, un n-propyle, un isopropyle ou un -CH₂F ;
R⁸ et R⁹ sont indépendamment choisis dans le groupe constitué de : hydrogène, deutérium, tritium, F, Cl, Br, I, - CN, isocyanure, -O-alkyle en C₁₋₃, -S-alkyle en C₁₋₃, -CH₂-OH, - CH₂-O-alkyle en C₁₋₃, -CH₂-SH, -CH₂-S-alkyle en C₁₋₃, alkyle en C₁₋₄ et alcényle en C₁₋₄ et dans lequel le groupe alkyle en C₁₋₄ et alcényle en C₁₋₄ peut être éventuellement substitué par un ou plusieurs substituants R⁶ identiques ou différents ; et
R⁶ est indépendamment choisi dans le groupe constitué de : hydrogène, deutérium, tritium, F, Cl, Br, I, -CN, isocyanure, - O-alkyle en C₁₋₃, -S-alkyle en C₁₋₃, -CH₂-OH, -CH₂-O-alkyle en C₁₋₃, -O-CH₂-Ph, -CH₂-SH et -CH₂-S-alkyle en C₁₋₃.

4. Composé pour une utilisation selon l'une quelconque des revendications 1 à 2, le composé répondant à la Formule (III) : 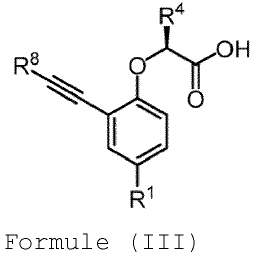 dans lequel :
R¹ est choisi dans le groupe constitué de : F, Cl, Br, I, -CN, -CF₃, -CCl₃, -CHF₂, -CHCl₂, -CH₂F, -CH₂Cl, -OCF₃ et -OCCl₃ ;
R⁴ représente un méthyle, un éthyle, un n-propyle, un isopropyle ou un -CH₂F ;
R⁸ est indépendamment choisi dans le groupe constitué de : hydrogène, deutérium, tritium, F, Cl, Br, I, -CN, isocyanure, - O-alkyle en C₁₋₃, -S-alkyle en C₁₋₃, -CH₂-OH, -CH₂-O-alkyle en C₁₋₃, -CH₂-SH, -CH₂-S-alkyle en C₁₋₃, alkyle en C₁₋₄ et alcényle en C₁₋₄ et dans lequel le groupe alkyle en C₁₋₄ et alcényle en C₁₋₄ peut être éventuellement substitué par un ou plusieurs substituants R⁶ identiques ou différents ; et
R⁶ est indépendamment choisi dans le groupe constitué de : hydrogène, deutérium, tritium, F, Cl, Br, I, -CN, isocyanure, -O-alkyle en C₁₋₃, -S-alkyle en C₁₋₃, -CH₂-OH, -CH₂-O-alkyle en C₁₋₃, -O-CH₂-Ph, -CH₂-SH et -CH₂-S-alkyle en C₁₋₃.

5. Composé pour une utilisation selon l'une quelconque des revendications 1 à 2, le composé répondant à la Formule (IV) : 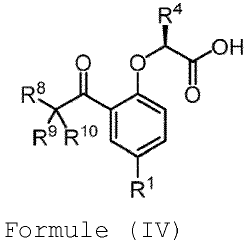 dans lequel :
R¹ est choisi dans le groupe constitué de : F, Cl, Br, I, -CN, -CF₃, -CCl₃, -CHF₂, -CHCl₂, -CH₂F, -CH₂Cl, -OCF₃ et -OCCl₃ ;
R⁴ représente un méthyle, un éthyle, un n-propyle, un isopropyle ou un -CH₂F ;
R⁶ est indépendamment choisi dans le groupe constitué de : hydrogène, deutérium, tritium, F, Cl, Br, I, -CN, isocyanure, - O-alkyle en C₁₋₃, -S-alkyle en C₁₋₃, -CH₂-OH, -CH₂-O-alkyle en C₁₋₃, -O-CH₂-Ph, -CH₂-SH et -CH₂-S-alkyle en C₁₋₃ ; et
R⁸, R⁹ et R¹⁰ sont indépendamment choisis dans le groupe constitué de : hydrogène, deutérium, tritium, F, Cl, Br, I, - CN, isocyanure, -O-alkyle en C₁₋₃, -S-alkyle en C₁₋₃, -CH₂-OH, - CH₂-O-alkyle en C₁₋₃, -CH₂-SH, -CH₂-S-alkyle en C₁₋₃, alkyle en C₁₋₄ et alcényle en C₁₋₄ et dans lequel le groupe alkyle en C₁₋₄ et alcényle en C₁₋₄ peut être éventuellement substitué par un ou plusieurs substituants R⁶ identiques ou différents.

6. Composé pour une utilisation selon l'une quelconque des revendications 1 à 2, le composé répondant à la Formule (V) : 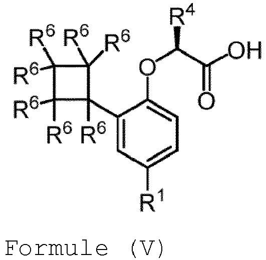 dans lequel :
R¹ est choisi dans le groupe constitué de : F, Cl, Br, I, -CN, -CF₃, -CCl₃, -CHF₂, -CHCl₂, -CH₂F, -CH₂Cl, -OCF₃ et -OCCl₃ ;
R⁴ représente un méthyle, un éthyle, un n-propyle, un isopropyle ou un -CH₂F ; et
R⁶ est indépendamment choisi dans le groupe constitué de : hydrogène, deutérium, tritium, F, Cl, Br, I, -CN, isocyanure, - O-alkyle en C₁₋₃, -S-alkyle en C₁₋₃, -CH₂-OH, -CH₂-O-alkyle en C₁₋₃, -O-CH₂-Ph, -CH₂-SH et -CH₂-S-alkyle en C₁₋₃.

7. Composé pour une utilisation selon l'une quelconque des revendications 1 à 2, le composé répondant à la Formule (VI) : 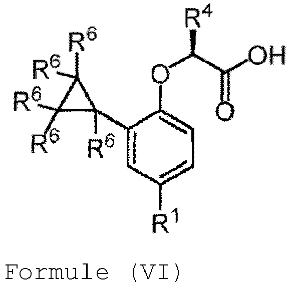 dans lequel :
R¹ est choisi dans le groupe constitué de : F, Cl, Br, I, -CN, -CF₃, -CCl₃, -CHF₂, -CHCl₂, -CH₂F, -CH₂Cl, -OCF₃ et -OCCl₃ ;
R⁴ représente un méthyle, un éthyle, un n-propyle, un isopropyle ou un -CH₂F ; et
R⁶ est indépendamment choisi dans le groupe constitué de : hydrogène, deutérium, tritium, F, Cl, Br, I, -CN, isocyanure, - O-alkyle en C₁₋₃, -S-alkyle en C₁₋₃, -CH₂-OH, -CH₂-O-alkyle en C₁₋₃, -O-CH₂-Ph, -CH₂-SH et -CH₂-S-alkyle en C₁₋₃.

8. Composé pour une utilisation selon l'une quelconque des revendications 1 à 2, le composé répondant à la Formule (VII) : 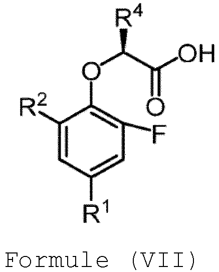 dans lequel :
R¹ est choisi dans le groupe constitué de : F, Cl, Br, I, -CN, -CF₃, -CCl₃, -CHF₂, -CHCl₂, -CH₂F, -CH₂Cl, -OCF₃ et -OCCl₃ ;
R² est choisi dans le groupe constitué de : alkyle en C₂₋₆, alcényle en C₂₋₆, alcynyle en C₂₋₆, cycloalkyle en C₃₋₆, cycloalcényle en C₅₋₆, -C(=O)-alkyle en-C₁₋₅, -C(=O)-alcényle en C₁₋₅, -C(=O)-alcynyle en C₁₋₅, -C(=O)-cyclcoalkyle en C₃₋₅ et -C(=O)-cycloalcényle en C₅, chacun pouvant être éventuellement substitué par un ou plusieurs substituants R⁶ identiques ou différents ;
R⁴ est choisi dans le groupe constitué de : alkyle en C₁₋₅, alcényle en C₁₋₅, alcynyle en C₁₋₅, cycloalkyle en C₃₋₅, et cycloalcényle en C₅, chacun pouvant être éventuellement substitué par un ou plusieurs substituants R⁷ identiques ou différents ;
R⁶ est indépendamment choisi dans le groupe constitué de : hydrogène, deutérium, tritium, F, Cl, Br, I, -CN, isocyanure, - O-alkyle en C₁₋₃, -S-alkyle en C₁₋₃, -CH₂-OH, -CH₂-O-alkyle en C₁₋₃, -O-CH₂-Ph, -CH₂-SH et -CH₂-S-alkyle en C₁₋₃ ; et
R⁷ est indépendamment choisi dans le groupe constitué de : deutérium, tritium, F, Cl, Br, I, -CN, isocyanure, -O-alkyle en C₁₋₃, -S-alkyle en C₁₋₃, -CH₂-O-alkyle en C₁₋₃ et -CH₂-S-alkyle en C₁₋₃.

9. Composé pour une utilisation selon l'une quelconque des revendications 1 à 2, le composé répondant à la Formule (VIII) : 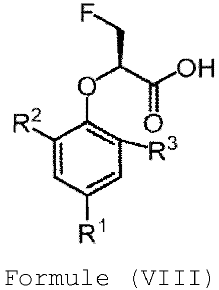 dans lequel :
R¹ est choisi dans le groupe constitué de : F, Cl, Br, I, -CN, -CF₃, -CCl₃, -CHF₂, -CHCl₂, -CH₂F, -CH₂Cl, -OCF₃ et -OCCl₃ ;
R² est choisi dans le groupe constitué de : alkyle en C₂₋₆, alcényle en C₂₋₆, alcynyle en C₂₋₆, cycloalkyle en C₃₋₆, cycloalcényle en C₅₋₆, -C(=O)-alkyle en-C₁₋₅, -C(=O)-alcényle en C₁₋₅, -C(=O)-alcynyle en C₁₋₅, -C(=O)-cyclcoalkyle en C₃₋₅ et - C(=O)-cycloalcényle en C₅, chacun pouvant être éventuellement substitué par un ou plusieurs substituants R⁶ identiques ou différents ;
R³ est choisi dans le groupe constitué de : hydrogène, deutérium, tritium, F, Cl, Br, I, -CN, -CF₃, -CCl₃, -CHF₂, - CHCl₂, -CH₂F, -CH₂Cl, -OCF₃, -OCCl₃ et isocyanure ; et
R⁶ est indépendamment choisi dans le groupe constitué de : hydrogène, deutérium, tritium, F, Cl, Br, I, -CN, isocyanure, - O-alkyle en C₁₋₃, -S-alkyle en C₁₋₃, -CH₂-OH, -CH₂-O-alkyle en C₁₋₃, -O-CH₂-Ph, -CH₂-SH et -CH₂-S-alkyle en C₁₋₃.

10. Composé pour une utilisation selon l'une quelconque des revendications 1 à 9, le composé étant un inhibiteur du canal ionique CIC-1.

11. Composé pour une utilisation selon l'une quelconque des revendications 1 à 10, le composé étant destiné pour une utilisation dans le traitement de symptômes d'une indication choisie dans le groupe constitué de la myasthénie grave (comme la myasthénie grave auto-immune et congénitale), le syndrome de Lambert-Eaton, la myopathie de réanimation, la sclérose latérale amyotrophique (SLA), l'amyotrophie spinale (SMA), la myopathie de réanimation (CIM), la polyneuropathie diabétique de réversion, le syndrome de Guillain-Barré, la poliomyélite, le syndrome de post-poliomyélite, le syndrome de fatigue chronique et la polyneuropathie de réanimation.

12. Composé destiné pour une utilisation selon l'une quelconque des revendications 1 à 10, dans lequel le trouble neuromusculaire a été induit par un bloqueur neuromusculaire.

13. Composé de formule (I) : 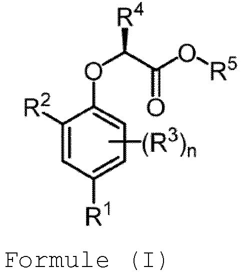 dans lequel :
- R¹ est choisi dans le groupe constitué de : F, Cl, Br et I ;
- R² est choisi dans le groupe constitué de : alkyle en C₂₋₆, alcényle en C₂₋₆, alcynyle en C₂₋₆, cycloalkyle en C₃₋₅, cycloalcényle en C₅, -C (=O)-alkyle en-C₁₋₅, -C (=O)-alcényle en C₁₋₅, -C (=O)-alcynyle en C₁₋₅, -C (=O)-cyclcoalkyle en C₃₋₅ et - C(=O)-cycloalcényle en C₅, chacun pouvant être éventuellement substitué par un ou plusieurs substituants R⁶ identiques ou différents ;
- R³ est choisi dans le groupe constitué de : hydrogène, deutérium, tritium, F, Cl, Br, I, d -CN, -CF₃, -CCl₃, -CHF₂, - CHCl₂, -CH₂F, -CH₂Cl, -OCF₃, -OCCl₃ et isocyanure ;
- R⁴ est choisi dans le groupe constitué de : alkyle en C₁₋₅, alcényle en C₁₋₅, alcynyle en C₁₋₅, cycloalkyle en C₃₋₅, cycloalcényle en C₅, chacun pouvant être éventuellement substitué par un ou plusieurs substituants R⁷ identiques ou différents ;
- R⁵ est choisi dans le groupe constitué de : H, alkyle en C₁₋₅ éventuellement substitué par un ou plusieurs substituants R¹¹ identiques ou différents, alcényle en C₂₋₅, alcynyle en C₂₋₅, cycloalkyle en C₃₋₆ éventuellement substitué par un ou plusieurs substituants R¹¹ identiques ou différents, phényle éventuellement substitué par un ou plusieurs substituants R¹² identiques ou différents et benzyle éventuellement substitué par un ou plusieurs substituants R¹² identiques ou différents ;
- R⁶ est indépendamment choisi dans le groupe constitué de : hydrogène, deutérium, tritium, F, Cl, Br, I, -CN, isocyanure, - O-alkyle en C₁₋₃, -S-alkyle en C₁₋₃, -CH₂-OH, -CH₂-O-alkyle en C₁₋₃, -O-CH₂-Ph, -CH₂-SH et -CH₂-S-alkyle en C₁₋₃ ;
- R⁷ est indépendamment choisi dans le groupe constitué de : deutérium, tritium, F, Cl, Br, I, -CN, isocyanure, -O-alkyle en C₁₋₃, -S-alkyle en C₁₋₃, -CH₂-O-alkyle en C₁₋₃ et -CH₂-S-alkyle en C₁₋₃ ;
- R¹¹ est indépendamment choisi dans le groupe constitué de : deutérium et F ;
- R¹² est indépendamment choisi dans le groupe constitué de : deutérium, méthoxy, nitro, cyano, Cl, Br, I et F ; et
- n représente un nombre entier 0, 1, 2 ou 3 ;
ou un sel, un hydrate, un polymorphe, un tautomère ou un solvate pharmaceutiquement acceptable de celui-ci, à condition que, lorsque R² représente C(=O)-CH₃, R¹ représente Br et R⁵ représente H ou Me, alors R⁴ ne représente pas Me ou CH₂CHMe₂ ; et à condition que, lorsque R² représente CHMe₂, R¹ représente Br et R⁵ représente H ou Me, alors R⁴ ne représente pas Me.

14. Composé selon la revendication 13, dans lequel :
- R¹ est choisi dans le groupe constitué de : F, Cl, Br et I ;
- R² est choisi dans le groupe constitué de : éthyle, vinyle, éthynyle, cyclopropyle, cyclobutyle, -C(=O-méthyle et -C(=O)-éthyle, chacun pouvant être éventuellement substitué par un ou plusieurs substituants R⁶ identiques ou différents ;
- R³ est choisi dans le groupe constitué de : deutérium, tritium, F, Cl, Br et I ;
- R⁴ est choisi dans le groupe constitué de : alkyle en C₁₋₅, alcényle en C₁₋₅, alcynyle en C₁₋₅, cycloalkyle en C₃₋₅, cycloalcényle en C₅, chacun pouvant être éventuellement substitué par un ou plusieurs substituants R⁷ identiques ou différents ;
- R⁵ est choisi dans le groupe constitué de : H, alkyle en C₁₋₅ éventuellement substitué par un ou plusieurs substituants R¹¹ identiques ou différents, alcényle en C₂₋₅, alcynyle en C₂₋₅, cycloalkyle en C₃₋₆ éventuellement substitué par un ou plusieurs substituants R¹¹ identiques ou différents, phényle éventuellement substitué par un ou plusieurs substituants R¹² identiques ou différents et benzyle éventuellement substitué par un ou plusieurs substituants R¹² identiques ou différents ;
- R⁶ est indépendamment choisi dans le groupe constitué de : deutérium, tritium, F, Cl, Br, I, -CN, isocyanure, -O-alkyle en C₁₋₃, -S-alkyle en C₁₋₃, -CH₂-OH, -CH₂-O-alkyle en C₁₋₃, -O-CH₂-Ph, -CH₂-SH et -CH₂-S-alkyle en C₁₋₃ ;
- R⁷ est indépendamment choisi dans le groupe constitué de : deutérium, tritium, F, Cl, Br et I ;
- R¹¹ est indépendamment choisi dans le groupe constitué de : deutérium et F ;
- R¹² est indépendamment choisi dans le groupe constitué de : deutérium, méthoxy, nitro, cyano, Cl, Br, I et F ; et
- n représente un nombre entier 0 ou 1.

15. Composé selon l'une quelconque des revendications 13 à 14, le composé étant choisi dans le groupe constitué de :
acide (2S)-2-{4-bromo-2-[2-(méthoxyméthyl)cyclopropyl]phénoxy}propanoïque ;
acide (2S)-2-[4-bromo-2-(2,2-dichlorocyclopropyl)phénoxy]propanoïque ;
acide (2*S*)-2-{4-bromo-2-[(1s,3s)-3-méthoxycyclobutyl]phénoxy}propanoïque ;
acide (2*S*)-2-{4-bromo-2-[(E)-2-bromoéthényl]phénoxy}propanoique ;
acide (2R)-2-(4-bromo-2-cyclobutylphénoxy)-3-fluoropropanoïque ;
acide (2*S*)-2-{4-bromo-2-[(1S,2S)-2-(hydroxyméthyl)cyclopropyl]phénoxy}propanoïque ;
acide (2*S*)-2-{4-bromo-2-[(1R,2R)-2-(hydroxyméthyl)cyclopropyl]phénoxy}propanoïque ;
acide (2S)-2-(4-bromo-2-éthynylphénoxy)propanoïque ;
acide (2*S*)-2-{4-bromo-2-[(1E)-2-cyanoéth-1-én-1-yl]phénoxy}propanoïque ;
acide (2S)-2-(4-bromo-2-cyclopropylphénoxy)propanoïque ;
acide (2S)-2-(4-bromo-2-éthénylphénoxy)propanoïque ;
acide (2S)-2-(2-cyclopropyl-4-fluorophénoxy)propanoïque ;
acide (2S)-2-(2-cyclobutyl-4-fluorophénoxy)propanoïque ;
acide (2S)-2-(4-bromo-2-cyclobutylphénoxy)propanoïque ;
acide (2S)-2-(4-chloro-2-cyclobutylphénoxy)propanoïque ;
(2*S*)-2-(4-chloro-2-propanoylphénoxy)propanoate de tert-butyle ;
acide (2*S*)-2-{4-chloro-2-[(2,2-²H₂)propanoyl]phénoxy}propanoïque ;
acide (2S)-2-(4-bromo-2-propanoylphénoxy)-3-méthylbutanoïque ;
(2*S*)-2-[4-chloro-2-(cyclopent-1-én-1-yl)phénoxy]propanoate de méthyle ;
(2*S*)-2-(4-bromo-2-propanoylphénoxy)-3-méthylbutanoate de méthyle ;
acide (2S)-2-(4-chloro-2-éthynylphénoxy)propanoïque ;
acide(2*S*)-2-(4-chloro-2-propanoylphénoxy)propanoïque ;
(2S)-2-(4-chloro-2-éthénylphénoxy)propanoate de sodium ;
acide (2S)-2-(4-chloro-2-cyclopropylphénoxy)propanoïque ;
acide (2S)-2-(4-chloro-2-propylphénoxy)propanoïque de sodium ;
(2S)-2-(4-chloro-2-éthylphénoxy)propanoate de sodium ;
(2S)-2-(4-chloro-2-éthylphénoxy)propanoate de méthyle ;
acide (2R)-2-(4-chloro-2-cyclopropyl-6-fluorophénoxy)-3-fluoropropanoïque ;
acide (2S)-2-(2-cyclopropyl-4,6-difluorophénoxy)propanoïque ;
acide (2S)-2-(4-bromo-2-propanoylphénoxy)propanoïque ;
acide (2S)-2-(4-chloro-2-cyclopropyl-6-fluorophénoxy)propanoïque ;
acide (2S)-2-(2,4-difluoro-6-propanoylphénoxy)propanoïque ;
acide (2S)-2-(2-acétyl-4-chlorophénoxy)propanoïque ;
acide (2S)-2-(4-fluoro-2-propanoylphénoxy)propanoïque ;
acide (2*S*)-2-[4-bromo-2-(cyclopent-1-én-1-yl)phénoxy]propanoïque ;
acide (2S)-2-[4-bromo-2-(2,2-difluoroéthényl)phénoxy]propanoïque ;
acide (2*S*)-2-{2-[2-(benzyloxy)cyclobutyl]-4-chlorophénoxy}propanoïque ;
acide (2*S*)-2-[4-bromo-2-(cyclopent-1-én-1-yl)phénoxy]propanoïque ;
acide (2S)-2-[4-bromo-2-(2-méthoxyéthyl)phénoxy]propanoïque ;
acide (2S)-2-[2,4-dibromo-6-(2-méthoxyéthyl)phénoxy]propanoïque ;
acide (2S)-2-[4-bromo-2-(cyclopropylidèneméthyl)phénoxy]propanoïque ;
acide (2S)-2-(4-bromo-2-éthényl-5-fluorophénoxy)propanoïque ;
acide (2S)-2-(2-acétyl-4-bromo-5-fluorophénoxy)propanoïque ;
acide (2S)-2-(4-bromo-2-cyclopropyl-5-fluorophénoxy)propanoïque ;
acide (2S)-2-[4-bromo-2-(2,2-difluoroéthényl)-5-fluorophénoxy]propanoïque ;
acide (2S)-2-(4-bromo-2-éthynylphénoxy)-2-cyclobutylacétique ;
acide (2S)-2-(4-bromo-2-éthynyl-5-fluorophénoxy)propanoïque ;
acide (2S)-2-(4-chloro-2-éthynyl-5-fluorophénoxy)propanoïque ;
acide (2S)-2-(4-bromo-2-cyclopropylphénoxy)-4-fluorobutanoïque ;
acide (2S)-2-(4-bromo-2-éthynylphénoxy)-4-fluorobutanoïque ;
acide (2S)-2-(4-bromo-2-éthénylphénoxy)-4-fluorobutanoïque ;
acide (2*S*)-2-{4-bromo-2-[(1*E*)-2-fluoroéthényl]phénoxy}propanoïque ;
acide (2*S*)-2-{4-chloro-2-[(1*E*)-2-fluoroéthényl]phénoxy}propanoïque ;
acide (2S)-2-(4-bromo-2-éthynylphénoxy)butanoïque ; et
acide (2S)-2-[4-bromo-2-(2,2-difluorocyclobutyl)phénoxy]propanoïque.
